# EUROPEAN PATENT APPLICATION

(11) **EP 0 812 916 A2**
(43) Date of publication of application: **17.12.1997**
(21) Application number: 97303395.4
(22) Date of filing: 19.05.1997
(51) Int. Cl.: C12N 15/57, C12N 9/64, C12N 5/10, C12Q 1/68, C12Q 1/37, A61K 38/48, A61K 48/00

(54) **Cathepsin k gene**

(30) Priority: 14.06.1996 US 19942; 17.06.1996 US 20273; 26.08.1996 US 26083
(71) Applicant: SMITHKLINE BEECHAM CORPORATION, Philadelphia Pennsylvania 19103 (US); HUMAN GENOME SCIENCES, INC., Rockville, MD 20850-3338 (US); THE INSTITUTE FOR GENOMIC RESEARCH, Rockville, Maryland 20850 (US)
(72) Inventor: Adams, Mark D., The Inst. for Genomic Res., Rockville, Maryland 20850 (US); Blake, Judith A., The Inst. for Genomic Res., Rockville, Maryland 20850 (US); Fitzgerald, Lisa M., The Inst. for Genomic Res., Rockville, Maryland 20850 (US); Fraser, Claire M., The Inst. for Genomic Res., Rockville, Maryland 20850 (US); Kirkness, Ewen F., The Inst. for Genomic Res., Rockville, Maryland 20850 (US); Lee, Norman H., The Inst. for Genomic Res., Rockville, Maryland 20850 (US); Debouck, Christine M., SmithKline Beecham Pharm., King of Prussia, Pennsylvania 19406 (US); Drake, Fred H., SmithKline Beecham Pharm., King of Prussia, Pennsylvania 19406 (US); Gowen, Maxine, SmithKline Beecham Pharm., King of Prussia, Pennsylvania 19406 (US); Rood, Julie, SmithKline Beecham Pharm., King of Prussia, Pennsylvania 19406 (US); Hastings, Gregg A., Human Genome Sciences, Inc., Rockville, Maryland 20850 (US)
(74) Representative: Thompson, Clive Beresford

(57) **Abstract**

The invention relates to cathepsin K polypeptides, polynucleotides encoding the polypeptides, methods for producing the polypeptides, in particular by expressing the polynucleotides, and agonists and antagonists of the polypeptides. The invention further relates to methods for utilizing such polynucleotides, polypeptides, agonists and antagonists for applications, which relate, in part, to research, diagnostic and clinical arts.

SEQUENCE LISTING

(1) GENERAL INFORMATION

(i) APPLICANT: Debouck, Christine
Drake, Fred
Gowen, Maxine
Rood, Julie
Hastings, Gregg
Adams, Mark
Fraser, Claire
Lee, Norman
Kirkness, Ewen
Blake, Judith
Fitzgerald, Lisa

(ii) TITLE OF THE INVENTION: CATHEPSIN K GENE

(iii) NUMBER OF SEQUENCES: 32

(iv) CORRESPONDENCE ADDRESS:
(A) ADDRESSEE: SmithKline Beecham Corporation
(B) STREET: 709 Swedeland Road
(C) CITY: King of Prussia
(D) STATE: PA
(E) COUNTRY: USA
(F) ZIP: 19406-2799

(v) COMPUTER READABLE FORM:
(A) MEDIUM TYPE: Diskette
(B) COMPUTER: IBM Compatible
(C) OPERATING SYSTEM: DOS
(D) SOFTWARE: FastSEQ Version 1.5

(vi) CURRENT APPLICATION DATA:
(A) APPLICATION NUMBER:
(B) FILING DATE: 07-MAY-1997
(C) CLASSIFICATION:

(vii) PRIOR APPLICATION DATA:
(A) APPLICATION NUMBER: 60/019,942
(B) FILING DATE: 14-JUNE-1996

PRIOR APPLICATION DATA:
(A) APPLICATION NUMBER: 60/020,273
(B) FILING DATE: 17-JUNE-1996

PRIOR APPLICATION DATA:
(A) APPLICATION NUMBER: 60/026,273
(B) FILING DATE: 26-AUG-1996

(viii) ATTORNEY/AGENT INFORMATION:
(A) NAME: Han, William T
(B) REGISTRATION NUMBER: 34,344
(C) REFERENCE/DOCKET NUMBER: ATG50006-2

(ix) TELECOMMUNICATION INFORMATION:
(A) TELEPHONE: 610-270-5219
(B) TELEFAX: 610-270-5090
(C) TELEX:

(2) INFORMATION FOR SEQ ID NO:1:

(i) SEQUENCE CHARACTERISTICS:
(A) LENGTH: 13674 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:1:

GCTTTGGCTC CCAAAGGCCT GGGATTACAG GCGTGAACCA CTGCGCCTAG CCTGTTAGCA 60
GCTCTTAAAA TCCAGAGGCA TAAGCCTGTA TTTTTGAGGG TTTATGCATG GAATCCAGCT 120
AGAAACTGAG TCTATTACAG ATCCCATTTA TTATCCTTTC TATTCCAAGA AGCCTTTTTT 180
TCTCCTTCCC CACATCTGTT TATGGAAGAA AATGAAGTTT GGGGTGTGGT TTGAGGAATC 240
AGCTAGATTC TTATGATCTG TCACATGCTT GGATGTTGGG GAAGCATTTG GAGAAGCTCA 300
TGTGACTTGT CCTAGATTGG GGATTTTAAT TGAGACAGAT GATGTTTATC GGGCATCCCA 360
CCACCTGAGA GTTTTAGCAA CAGAGTCACA TGTGAGTCCA TCAGAACTTA CGGCATTGAT 420
TCAAGTGCTG TCATAAATAA CCAGGACTGC TGTTTTTGGT TACTTTTAAA GACAGTTTCA 480
TCTGGACTTT CTGGGCATAT CCTCCTTCAG CAAAACCACA TTAGGCTGGG AAAACTATTC 540

TGCCTGGAAG TAATGACAAC TTGCAACCAA CAAGCTTATA AAAATACAAA GAATTCTGGA 600
GCCTATGGCT TCCATTACAT TATTCTTTTA TAGCCTTTTA TGTTCATTAC CGCATCCCAG 660
AGGTGAGAGT CAGACACAAA TATGAAAATA GGTTTCAATG TTGGAGAGGT AAATCCTAAC 720
AGGAAAGGGG TAGGAAAAGA TATAATCCCC CAATATTAAA ATAAAGATAT TGAAGAAGAA 780
GGATGGGAGA GACTAGGGCT GTGTCCTTCC TTTTACTCAC CAAAAGAGAA AGTAAGCTCC 840
TATTTGAGTC AATAGATATT GAGGTCTTGT TATTTGCCAC CAAAGACAGT CTTGTGAGAC 900
TAAATAGCTA GTAATTCCCT ACCCTGGCAC ACATGCTGCA TACACACAGA AACACTGCAA 960
ATCCACTGCC TCCTTCCCTC CTCCCTACCC TTCCTTCTCT CAGCATTTCT ATCCCCGCCT 1020
CCTCCTCTTA CCCAAATTTT CCAGCCGATC ACTGGAGCTG ACTTCCGCAA TCCCGATGGA 1080
ATAAATCTAG CACCCCTGAT GGTGTGCCCA CACTTTGCTG CCGAAACGAA GCCAGACAAC 1140
AGATTTCCAT CAGCAGGTAA CGTTTGCAAC TTCCTAGATC TTTTAGCTTT TCATTCCTGT 1200
CAATTCTCTG AGTATTAGGG ATGTAGTGAC TTGAGGATCA CAATAAACTT TTAGCCTCTG 1260
CAGATGAAAA CAGAGATGCA CTTCTTAGGT CATTCCCTGG CTAAATAAAA TCTGCCTGGA 1320
AATCTGTAGA ATTCCTTGTA TGATTTATAT ATATACATAC ATGATTGTTA GTAAAAGCAA 1380
AGTATATAGG GAATCATTTC CCCATCCTTC AAGAGTGGCC TTTCTGCAGT GTTTTCTACT 1440
TTGGCCAACA AGGATCAAAA CGGTTAACTC CTTAGTGAGG AGGAGGAGAG TGGTATGGGG 1500
AGGTAGTAGC TCAGTGCTTC CTGTTCACTG AGACATCTCA AAGCCCTTAA CACTCTAGTT 1560
TTTAAATGTC CTACTGGACA TTTTGCCAGT TTGCAAAATT ACATGTAAAT GGACTATAAG 1620
CAATTGTGTA AGCCATATGT CATGCTGCAG GCTGCAAATT GTTCTTAAAA TGGAGGATTT 1680
GTAATTAAGA AAGCCAATGC AAGAAATGAG TGAAGCTAAC TAGAGTAAAC TTATGAAAAG 1740
CTGTGAATTT CATCATCATA GAACATTGCT TTTCAGTCTG AACATTCTTC TAACAAACCT 1800
TGGATCTGAG GCTTCTTGTC CTTTGCGGCA GCCACAGTGG GTTTTTGTTG TTAGGGGAAA 1860
ATAAAAAACC TTGCCCGCAG CATCTGGTTA AGATTAGGGC AGTTTCCTGC CTAAGGAGGG 1920
AAGGGAGAGA AAAAGGAAGA AGAAATGCAT AAGGAGAATG AGGAGATATA CAATGTCTCA 1980
GAAAACAGGA AACATTGTCC TATTTTCCCT TGTCCTCTTC TGACAAGATC TGGGAAAGTA 2040
CCAGAATTTA GGCACGAAAG AGAAGAACGC CTCGAAGAAA TGATCAGGAA GCAAAACTTA 2100
GACGGAAATC TCTCCTTTGT GTATTCTGAA CCCCACTACC ACCTTGCTAT TTGTCTGTCT 2160
CCAAGCCTGC TAGGGACCCT GGAGGAAACG CACTGAGCCC ATTCTGATTG TCCAGTTTCT 2220
ATCCCCCATT TCTGGTTGTG TACGTGTGTG TGTGTGTGTG TGTGTGTGTG TGTGTGTGTG 2280
TGTGTGTGAG AGAGAGAGAG ACAGAGAGAG AAACAGAGAG AGTGTGTGTT GCCTAAATCT 2340
CCCGAGAGAG AGAGAGAGAG AGAGAGAGAG AGAGAGAGAG AGAAAAGAGA GAAATGGCTA 2400
AATCCCCCTA GATCAAAGTC CTTGGAACCA GATGTACCAG CATCCTATCT AAACACAGGC 2460
CCCTCCTGAC TATCATTGTT TTATCACCCT TTTTCCGTCT ACCTTTCTCT TCCTCATAAA 2520
GCCTAGTTTT CCTCTGTTTC CCTGCCAAAT GGAAGAGTTT TCCCTAACTA CATTCTTCTG 2580
CAGGATGTGG GGGCTCAAGG TTCTGCTGCT ACCTGTGGTG AGCTTTGCTC TGTACCCTGA 2640
GGAGATACTG GACACCCACT GGGAGCTATG GAAGAAGACC CACAGGAAGC AATATAACAA 2700
CAAGGTGCCT GGGGTCCTGG AGGGGGCATG GCAGGAAGGC TGAGACCTGA GCTCTCTCAT 2760
CTTAGCTTCC AGACTCCCTT CTTCAATCCA AATGCTTTAT TCCAAGCAAA TCAGTCCCTC 2820
TTCCCTAACT CATGTTAACA TACGGTTTTC ATTCCTATGC TTCAATCATC CTCTTGTCAA 2880
ACTTGTATTC CTTCCCTTTG GTTTTATAAG TGTGTAACAT TCCTCTTTTG GGAAGAGTCC 2940
CAAGATTAAT GCTGTTAATC CATAAGCAAT TTTTCTGTCT CTCCAGAGCT TGTGTGGTTG 3000
TTTACATATT ATCTCTCTTC TTGCAGGCTC TTAATTCCAT GGTTAGTTCC CCAACTAAAC 3060
TGTAAACTTT TATGATTGTG AGTTTCCTTT ATTCTCCTAA AACCCTTCAC AATATTACAT 3120
ATGAACTGTA GACAGTCTAT ACAAGTACTG ACTATGCTTT GTTTAGGTGG ATGAAATCTC 3180

TCGGCGTTTA ATTTGGGAAA AAAACCTGAA GTATATTTCC ATCCATAACC TTGAGGCTTC 3240
TCTTGGTGTC CATACATATG AACTGGCTAT GAACCACCTG GGGGACATGG CAAGTATAGC 3300
TTCAGCTCCT GTCCCACCTG CACCATTTGC TTTAGTTCCC TGCTGATGCC TGGCCTCTTT 3360
CTTCTTTGTC TTAGACCAGT GAAGAGGTGG TTCAGAAGAT GACTGGACTC AAAGTACCCC 3420
TGTCTCATTC CCGCAGTAAT GACACCCTTT ATATCCCAGA ATGGGAAGGT AGAGCCCCAG 3480
ACTCTGTCGA CTATCGAAAG AAAGGATATG TTACTCCTGT CAAAAATCAG GTACTCTCCT 3540
TTCTTCTGGG TGTGCATATG TAATCTGGCA TGACCTTTTC CTTTTTCTGC TGCTTTGTTC 3600
TTGAGGTGAA AGGGCACCAG GAAAAGAGGG CAAGGAATTA AGGTACATCT CCCCATTCCC 3660
ATTCTGTTAT TTAACCTCAT TTGTTTCTGT ACATTTGGGT TGTTTCTGGT TTTTCTTTTT 3720
CTTTTCCCTT TTTTTTTTTT TTTTTTTTTT GAGATAGAGT CTCACTCTGT CGCCCAGGAT 3780
GGAGTGCAGT GGTGCAATCT TGGCTCACTG CAACCTACAC CTCCCGGGTT CAAGCGATTC 3840
TCCTGCCTCA GCCTCCTGAG TAGCTGAGAT TACAGGCACG CGCCACTACG CCTGGCTAAT 3900
TTTTCTATTT TTATAGAGAT GCGTTTTCAC CATGTTGGCC AGGCTGGTCT TGAACTGACC 3960
TCAGGTGATC CACCTGCCTC AGCCTCCCAA AGTGCTGGGA TTAGAGTCAT GAGCCATCGC 4020
GGCCTGGTTT TTCTTTATTA CAAATAGTGT TGCAATAAGC ACCCTTGTGC ATATGTTTTT 4080
GTGCACATGT ACAAATATTT ATGCAAAATA AGTCCTAAAA TTGGAATTGT TAGGTCACAA 4140
ATAATCCTTT CCCCCCCCCC AAATTTTTTT TTTTTTTTTG AGACAGCGTC TCTGTCACCC 4200
AGGCTGGAGT CCAGTGGCGC AATCATGGCT CACTGCAGCC TCAACGTCTC AGGCTCAAGT 4260
GATTCTCCAA CCTCAGCCTC CCTAGTAGCT GGGAATTAGA AGCACATGCC ACCACACCCA 4320
GCTAATTTTA AAAAATTTTT TGTTAGAGAC AGGGTTTTGC CATGCTACCC AAGCTGGTCT 4380
CAAATTCCTG GGCTCAAGCA ATCTGCCCGC TTCGGCCTCC CAAAGTGCTA GGATTACAGA 4440
CATGAGCCAC CATGCCCAGC CCAAAAAAGT TTTTGCAATC TTACATTCTT ACTAGCATGA 4500
GAATGTCAGT TTTTTCACAA CCCAAACAAC ACAGGATTGT ATCAGCAAGA TAAACAATTG 4560
ATTTAACGTT CATTTAACAA ACACTTTTTG ACCCCCAGAA CCTACCAGAT GCAGTGTTAG 4620
GCAGCAGAGA CTCAAGATGA CTAAGACACA ACCTGTGTCC TCAGGAAATC TCAATCTAAA 4680
AAAATAGAAC AGGAAAGAAA GAAAAATCTA CAATCTAGCT GCACAAACAA TAATAGCTAA 4740
TACTTTTTGA GATTTTATTG TTTGTCAGGA ACTTCTTAAC TCTTTACATG AGTTTAAATA 4800
TTTAATCCCT TATAACAATA TTTTATGCAT AGAGAAACTG AGACACAGGC AAATTTAGTA 4860
ACTTACCCGG GGTCACATAG CTACTGGGTG GCAAAGTCAG GGTTAGCTCC CAGGACAAAT 4920
GCCTCCACAG CTGGTACTGT GCTCTGCTTT ACTGTAGCTA ATAGTAAAAA TGGTAGCAAA 4980
AATCAATAGC AGTAGAACAG TGCAACAGAT ATTAAGCGGA AGAGGAAGAC TCACAACAAT 5040
GACAACATTT GTGCTGAAAT TTTTAAGAAC ACATGGAATT TCCTTCAGCC GGGTAGAGAG 5100
AAGATATAGA AATGTAAACA CCAAAGATTC ATAGTTTCTC TGTATCCCTT TCAGGGTCAG 5160
TGTGGTTCCT GTTGGGCTTT TAGCTCTGTG GGTGCCCTGG AGGGCCAACT CAAGAAGAAA 5220
ACTGGCAAAC TCTTAAATCT GAGTCCCCAG AACCTAGTGG ATTGTGTGTC TGAGAATGAT 5280
GGCTGTGGAG GGGGCTACAT GACCAATGCC TTCCAATATG TGCAGAAGAA CCGGGGTATT 5340
GACTCTGAAG ATGCCTACCC ATATGTGGGA CAGGTGAGAT TGCTCCACAC AATTATACAG 5400
CTCTGTTGGC TCCTCCTTCC CCAGCATGAT GTTTTGTACT GGAAACAATT CCAGAAATAC 5460
TGTTTTCTGT TATCCTATCC TGCTTTCTTG ATGGAATAAT TTCCCACAGA AGGCCAAGAA 5520
GATTTCCACA ATCTGGGGGA ATTTAGGGAG CTTAAGCTAC TATAGCTCCT ATTTGCATCT 5580
CTGCCATGGA GAGAAAACAG AGGCTAGGCT ACCTACCCCA TAGACTTCCG AGCTGGGTTC 5640
TATAACCCTC TGCTCAATTC CTCACTCCCA CAACAAACCC ACAAACCCAC CATGCTATTT 5700
TCACAAATTG TGTGGCTTTA TTTTATATGA TCTCAGTGTG AGTTTTCAGA ACATTTCAGC 5760
AAATTATGTA AGTTTACATG CTAACATCTA TAAAATGAGA GAAAAAACAA GTTGCTTCAT 5820

ATAAGAGATA AGGGATTAAC TCAGTTCCTC CTGCATGATC CTCTAGTCAT AGGAAGGAAA 5880
TCATATCTGA AAGGGAGGCA ACCTGAGGGG TTTTTTATAC ACATAGGGCT GGGTCTGATA 5940
GACAATATAA TGTAGGGCCT TCACAACAGA AACCTCTGAA ACAGGGACAG CAAGTTTGAG 6000
AATAAAAATG ATGGCTACTG TGTTCTAAGC CGTGTCCTTA GTGCATTTTT TCTTTTTCTT 6060
TTTTTCATTT AATCTCATAA CAACTCTGTT AGGTAGACTT ATCTTGAATG TATAGGTGAG 6120
GAAATGGACA CTTAAGGAGA TAAGACAGTA TAATTCATAC CACTAGTATG TAACAATGTA 6180
AGATGTATCT ACCAGGGATG TTTATCTTCT GCAAACATTC CTAGGTATAT CTCCCATGCA 6240
CATGTGCAAG AATTTCTTAC TAGGATATAA TGCCTTGGAA CTGAATTGTC TGGGTCTTAG 6300
GGTATGTCTG TCTTCAACTT TACTACACAA TGTCAAATTG TTTGCCAAAA TATTTGGAAA 6360
AATTTATACC TGCAATGTGT AAGAAATCCC CTTCAATCAC CTTTTTATCA GTATGTTTAT 6420
CTGGCCATTT GCATTTCTTC TTCAGTGAAT TAACTGTTTT TATCTCTTGC TCATTTGTTT 6480
TTCTTTTTAT TTTTTTGAAA TAGGGTCTTA CTCTGTTGCC CAAGGCTGGA GTGTGGTGAT 6540
ACAGTCATAG CTCACTGCAG CCTCCACTTC CGGGCTCAAG CAATCCTCTC GCCTCAGCCT 6600
CCCAAATAGC TAGGATATAG GTGCATGCCA TCATGCCCAC CAATTTCAAA AAACCTTTGA 6660
AATTTTTTTT TTGTAAAAGC TAGGCATGGT GGCTCATGCC TGTAATCCCA GCACTTTGGG 6720
AGGCTGAGGT GGGGURCNTN UDAGGATCGC TTGAGCCCAG GAATTGGAGG TCGGCCTGAT 6780
ACAACATAGC AAGACCTCAT CTCTACAGAA AAAATTTTTA AAAGTAGCCA GGTATGATGG 6840
CGTGCATAGT TCTAGCTACT CCGGAAGCTG GTTGGGAGGA CAACTTGAGC CTGGGAGTTC 6900
AAGGCTGCTG TGAACTGTGA TCATGTCACT GCTCTCTAGC CTGGGTGACA GAGTGAGACC 6960
CTGTCCCCAA AAACAACAAC CGTTTTTTTT GGTAGAGACA TTGTCTCGCT ATGTTGCCAA 7020
GGCTAGTCTC AAACTCCTGG GCTCAAGCAA TCCTCCCACC TCCCAAAGTG CTGGGATTAT 7080
AGATGTAAGC CACCATGCCT GGCCTACCCT TTTTTTTTTT TTTTGAAATG GAAGTTTTGC 7140
TTTTGTCACC TAGGCTTGAG TGCAGTGGCG CGATCTTGGC TCACTGCAAC CTCCACCTCC 7200
TGGATTCAAG CAATTCTCCT GCCTCAGCCT CCTGAATAGC TGGGATTATA GGCACCCGCA 7260
ACCACGCCCG GCTAGTTTTT GTATTTTTAG TACAGACAGG GTTTCACCAT GTTGGCCAGC 7320
TGGTCTTGAA CCCCTGACCT CAGGTGGTCC GCCCGCCTCG GCCTCCCAAA ATGCTGGGAT 7380
TAAAAGTGTG AGCCACCATG CCCCACCCCT TACTCATTTT TAATTGGATT GTTTTTTCTC 7440
TTTCTTAGCG ATTCTTAAAA GTTTAAAGAG AATATTTGGA TACAATACTA TGTATTTAAA 7500
AGTTGAGGTC TGTCTTTCCA TTCTTCTCAC GATGTCTTTC AATCTAGAAA AGTTAATTTT 7560
AATAGGCCTG GCGCGGTGGC TCACGCTTGT AATCCCAGCA CTTTGGGAGG CTGAGATGGG 7620
TGGATCACAA GGTCAGGAGA TGAAGACCAT CCTGGCTAAC ATGGGTGAAA CCCTGTTTCT 7680
ACTAAAAATA CAAAAAAATT AGCTGGGCGT GGTGGCAGGT GCCTGTAGTT CCAGCTACTC 7740
GGGAGGCTGA GGCAGGAGAA TGGCGTGAAC CCGGGAGGTG GAGCTTGCAG TGAGCCGAGA 7800
TTGCACCACT GCACTCCAGC CTGGGCAACT GAGCAAGACT GCGTTTCAAA AAAAAAAAAA 7860
GTTAATTTTA ATATAGTAAA ATTAGTAAAA GGATTAATTT TCCCTTTGCA ATTTTTGTAA 7920
TGTGTTTTAT TCGTTTATGA ATGGAGAAAG GTAAGAAAAA ATAAAATTTA AAAAAGAAGA 7980
GATGTGGCCA GGTACGGTGG CTCACACCTA TAATCCCAGT AGTTTGGGAG GCTGAGGCAG 8040
GCAGATCACT TGAGGTCAGG AGTTTGAGAC CAGCTGGGAT AACATGGTGA AACCCCATCT 8100
CTACTAAAAA TACAAAAATT AGCCAGGTGT GATTGCGCAC GCTTGTAATC CCAGCAGGCT 8160
GAGGCAGGAG AATTGCTCGA ACTCAGGAGG CAGAGGTTGC AGTGAGCCAA GATCATGCCA 8220
TTGCACTCCA GCCTGGGTAA CAGAGACTCT GTTTCAAAAA AATAAAAAGA TAAAAAAGGA 8280
AGAGATCTGA TAGGGCGGCC AGATAAACAT TTTAAAGGGG ATGGTATTAT AAGTTTGTTC 8340
CCAGCATAAT GCCAGGTTAT TCTGACTTTA AAGTATCATC ACATAATATC TTTTTGAGTC 8400
AATTTCCAAG ATATTCTGTT TCACTTGTAA TTCTGTGTAA TTTTTGGCAC CAGGAGGCAT 8460

CAGGGATTTG GAGCACATGG CAGAAACAAA GGCATCTTGA AAAATATCAA GGCAGTAGAC 8520
CACTGTAATC TTAAAATGGC ATATCAAATG CTGCTATTGC TGTTAATATT TAGATAATGT 8580
TAGATAATGT ATTTTTTTAG AGGGTATCTC ACTATCTTGC ACAGGCTGGA GTAGAGTGGC 8640
TATTCACAGC ATGATCACAG TACACTAAAG GCTCAAACTC CTGGGCACAA ACAATCCTCC 8700
TGCCTCAGCC TGCTGAGTAG TAGATAATAA GTTCTTGTGG ATGCAACCTT AGGGTTCTGA 8760
AGGGGTAGTC TGTAGGAAAA TGAATTGCTG AAAAGAATAC ACCACCTTAA CATGGGCTAT 8820
TATTCGATTC CATAATTGTG GCTTGCCAAT GAAACATTGC TAACTACCTG TAAAATATAG 8880
TGTTGGAAGT CATAGGCTAA ATTGCTAAGT TCTTTAATCT ATTTTAGTGT CTTGTTATGT 8940
ACTTTTATAT TTTGTCTTTG ATGAGAGCAC AAGGATCACA CCAGTTCCCC TGATATAGGT 9000
GCAGAGGGCC CAGGTCTTCC CTCTAGCTAA GCCTTGGCCT TGGCCTCCTA CCCACACAGC 9060
AGCTGGTGCC TTCCTGCCCC CTGAGGCTAA TACATACTAT GTGGCCAGAA GATGGTTTAT 9120
GCTTTTTAAA AAAATCTTAT TTCAGAAATC TTTCCCTACT GTTTTCCTCC CACATTTATG 9180
TCTTAAAACA CCTGTAGGGG ATTTTTTTTT TTTTTTTTTT TTTGAGATGG AGTCTCGCTC 9240
TCGCCCAGGC TGGAGTGCAA TGGCGCGATC TTGGCTCACT GCAAGGTCTG CCTCCCAGGT 9300
TCACGCCATT CTCCTGCCTC AGCCTCCCCA GTAGCTGGGA CTACAGGCGC CCGCTACCAC 9360
GCCTGGCTAA TTTTTTTGCA TTTTTAGTAG AGACAGGGTT TCACTGTTAG CCAGGATGGT 9420
CTCGATCTCC TGACCTCGTG ATCCACCCTC CTCAGCCTCC AAAGTGCTGG GATTAACAGG 9480
CATGGAGCCC CACCGCACTG GCCTGTATTT GTGAGGAAGA ACAGACCCTC TTTAGAAGCC 9540
CTAGACTGCT GCCTCTGTTA GTTCACTGGC ATCACTCAAA ATATTGGTTG AGTTTCTTAC 9600
TCACTGAGTT GGTTTTTATG TGTGGTGGAA GGCGGGAATC CTCTTTTCAT ATTCGTTCTC 9660
ATTGCCTATT GCTTTGTCCT AGTCCTATTA CAATCTTGTT TCTTCCAGGA AGAGAGTTGT 9720
ATGTACAACC CAACAGGCAA GGCAGCTAAA TGCAGAGGGT ACAGAGAGAT CCCCGAGGGG 9780
AATGAGAAAG CCCTGAAGAG GGCAGTGGCC CGAGTGGGAC CTGTCTCTGT GGCCATTGAT 9840
GCAAGCCTGA CCTCCTTCCA GTTTTACAGC AAAGGTAAGA AGCTGCTGAT CCTATACAGC 9900
ACTGTCTTTT ATGATACAAA CTTGATGGTT TCTCGAAGGA CCTTGGGTAT TTTCAGTACT 9960
TAGTTTTTGT ATTCACATGG AGGTGGCCAG AGAGAAATTA ACAACTGCTG CAGTATGGAG 10020
CAGCATCTCT GTGGTAAACC CTCCTGACAC GGATGGAATT CTTCAAACAG TCTCCTAGAC 10080
TGGGAGATCC CACAGGGTGA CCCTTGGATT GCATAGAGCC TCACGCTGGT AGTTTGTATT 10140
CTAGGTGTGT ATTATGATGA AAGCTGCAAT AGCGATAATC TGAACCATGC GGTTTTGGCA 10200
GTGGGATATG GAATCCAGAA GGGAAACAAG CACTGGATAA TTAAAAACAG GTAATGATGG 10260
GAACACTACT TTTGTTATTC AGTCACCCTT TTAACACTCA ACCTCACCTC CAGCTTCCCG 10320
ATATTCCTTT CTCTGTCCCA AATCAAGAAA AAATTATCTC AGAGTTCTCA CTTCTATCTT 10380
CTCAGTCAGA GGCTCTTAAT TCTCAGTCTG ACACTTAATG GCCAGTGTGT TAGTCCATTT 10440
TGCATTGCCA CAAAAGAATA CCCGAGACTG GGTAGTTTAT AAAGAAACGA GGTTTGTTTG 10500
GCTATACAAA GCGTGGCACT AGTATCTGCT CAGCCTCTGA TGAGGCCTCA GAGCTTTTAC 10560
TCATGGCAGA AGGCAAAAGA GGGAGCAGGC ATGTCACATA GTGAGAGAGG GAGCAAGAGA 10620
GAGAGGGAGG TGCCGACTCT TTAAAGAACC AGCTCTTGCA TGAACTAATA GAGTGAGAAC 10680
TCACTCATCA CCAAGGCGAT GGCACCAAGC CATTCCATGA GGAATCCACT CTCATAACCC 10740
AAACACCTCC CACTATGCCC CACCTCCCAC ATTGGGGATC ACATTTCAGC ATGAGACTGG 10800
GAGGGGACAC ACATCCAAAC CATATCCGCC AGACAATAGT GCTCAATTAT GTGCTGGGCA 10860
GATGCTCCCT GTGTGCAAGG TGCTTAGTGA CATACATAAA CCAACGAGCA GATGACACCT 10920
TCAGTGAGCT CAGAGCCCAA TAAGACAGAC CTAACTAACC ATGAGATAAA GCAGTACAAA 10980
GAACCAGCAG GAGCTTTGGA ATTACGTATT TTTACTTTCT TTTGTCTCTA ATGTGATCAG 11040
TTTCTTAGAT GGTTTCCATT AGCAATCTGT CTTTAACAGT AGGGGAGCAG CGTTAAAGGT 11100

TTAATATTCC TTTTGAACAG TTTTTTTCCT TCAAAATACA CTTAAGATAC ACGTATATAA 11160
GAACTTGCCA AAGATTGTGA AGAGAAACAT TTTTTAGAAA TAAGATATAA ACAAAAAAAG 11220
TTAGTGTTAC TTTCCTATGT TGGGGAACAA AGAAAACTCC AGGGTACCTT GCTTCCCATT 11280
TCTCTTTAGC ACCTTGTGAC TTTTGGGGAG GGGCAGATTG ATAACAATTA TAGTTTTCCT 11340
TTCCTGGCTG ATCACCATTA ACCTGGCAGC AGCACTGGCT AAATCTCCTG TCCTTAGTGC 11400
CCTCCAAGGA GCAGGAGCCC TAGACTCTGG GTCGCTGACA GACTCACGCA GTGGTGTTGT 11460
TCAAACCTGA AGCAACTTTT TATATCACAG TTCCAACTCA AGGTGAACCT GAGCATCTTC 11520
CCAAGTCTCC CACAGCTTCT GTCCTGTGTT GTCCCTTCTC TTGACTCCCA GGTCCAAGCA 11580
CTTACCCTGT TCTTTCATGA TCAGGTACCA TGTGTGGAGA TAGCTTCCAA GAGAGCTGGG 11640
AGGAAGAAAG GACACACCCG GGCAGGATCA GGAACACTGG GGGCCCCTGG AGAAGGGGAG 11700
AGTGGGGGAG GGTACAGGTT TTAAATAAAA TGTGTTGGTA ATTAGAGAAT TGCTGGTTGG 11760
GGAAAGAGGT CTGAAAACAA TTCAGGAAGA TAAACAAGAC AATCTCTCCT CTCTCCTCTT 11820
TCTCACGTCG TCTCTCTTGT CTTCTAGTCT CGCTACTCAT TTCCTTAGTA ATCTCATCCA 11880
CTCTCATAGT TTCATCCATC TCTCCTATGG GGTTTACCCC CAAATCAAGA TCACCAGCTT 11940
CAGCCTCCTT CTTATGCTCT AAACTCACAT TTTCAAGATT AATATTCCCC AAATACAGCT 12000
CTGATCATAT CACTCTCCCA CTCAAAATCC CTCACTGGCT CCTCACGATG ATGGGTCACA 12060
GAGTAAAGGT GAAGCTTTTT AACCTTGCAG TAAAGGTAAT TCAACCTGAT CTCAATCTGC 12120
CTTTCCAGAC ATCTCTCCCA CTACACCCTG TTAGGCACAC TGCTTTTCAG CTACATGATC 12180
CTAACAGTGC CCCACACTTT CCTGCCTCTG TTGTTCATTT CACACCCTTC CACTGGCATC 12240
CCCTTCCCAC AGGTCGAAAT TCTACTTAGC CTTTTGGCTC AGCTCAAATG CCACCTCTTA 12300
CATCAAGCCT CTAAGATTCT CTTGATCAGA AGGAATCTTT CCCTCCTTTG ATACCTACAG 12360
TATTATGCCT TCTCCCTATT TCTTGACTTT AAACTCTTTA AAGTTAAAAA ACATCATATT 12420
CATTTTTGTG TACCATCAGT ACCTCGCACA ATACTCAGTA AATATTTTAA TGAATAAATA 12480
AACTGAGAGT ACTAAGTATT TTTCTTGATT GGTCTTACAG CTGGGGAGAA AACTGGGGAA 12540
ACAAAGGATA TATCCTCATG GCTCGAAATA AGAACAACGC CTGTGGCATT GCCAACCTGG 12600
CCAGCTTCCC CAAGATGTGA CTCCAGCCAG CCCAAATCCA TCCTGCTCTT CCATTTCCTT 12660
CCACGATGGT GCAGTGTAAC GATGCACTTT GGAAGGGAGT TGGTGTGCTA TTTTTGAAGC 12720
AGATGTGGTG ATACTGAGAT TGTCTGTTCA GTTTCCCCAT TTGTTTGTGC TTCAAATGAT 12780
CCTTCCTACT TTGCTTCTCT CCACCCATGA CCTTTTTCCA CTGTGGCCAT CAGGACTTTC 12840
CCCTGACAGC TGTGTACTCT TAGGCTAAGA GATGTGACTA CAGCCTGCCC CTGACTGTGT 12900
TGTCCCAGGG CTGATGCTGT ACAGGTACAG GCTGGAGATT TTCACATAGG TTAGATTCTC 12960
ATTCACGGGA CTAGTTAGCT TTAAGCACCC TAGAGGACTA GGGTAATCTG ACTTCTCACT 13020
TCCTAAGTTC CCTTCTATAT CCTCAAGGTA GAAATGTCTA TGTTTTCTAC TCCAATTCAT 13080
AAATCTATTC ATAAGTCTTT GGTACAAGTT TACATGATAA AAAGAAATGT GATTTGTCTT 13140
CCCTTCTTTG CACTTTTGAA ATAAAGTATT TATCTCCTGT CTACAGTTTA ATAAATAGCA 13200
TCTAGTACAC ATTCATTTTG TGTTGGATAC TGTGTTAGGT GCTGGAGGAA AAAAGATGAA 13260
TAGAACATCT TCTATGTACT TGATGCGCTC ACAGTCTGGT TGTAGAGACT GTCACATAAA 13320
CATTTCATCC CAATTCATTT ATTTGTTCAT TCCTTCAGCC AATATATATT GAGTTCTTAC 13380
TCTGTGCCAA GAACTGTACT ACATTTCTGG GATTAAGTGG ATATAAGGAG ATCTCAGTGT 13440
TTAATCTGCC TGAGGGGAGA CTAAATTAAG TGACATGGAA ACTTGGGTCT TGAAAAACAT 13500
TTTAAGGTTA TTTTTTCTTT TCTCTCTCTC TCGCTCTGTC TTTCTCTCTC TTTCGTCAGG 13560
GTCTCCCTCT GTTGCCCAGG CTGGAGTCAG TGGCACTCAT AGCTCACTGC AGCCTTGATC 13620
TCCTGGGCTC AAGAGTTCTT CCCACCTCAG TCTCCTAAGT AGCTTGGACT ACGG 13674

(2) INFORMATION FOR SEQ ID NO:2:

(i) SEQUENCE CHARACTERISTICS:
(A) LENGTH: 1108 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:2:

GCTTTGGCTC CCAAAGGCCT GGGATTACAG GCGTGAACCA CTGCGCCTAG CCTGTTAGCA 60
GCTCTTAAAA TCCAGAGGCA TAAGCCTGTA TTTTTGAGGG TTTATGCATG GAATCCAGCT 120
AGAAACTGAG TCTATTACAG ATCCCATTTA TTATCCTTTC TATTCCAAGA AGCCTTTTTT 180
TCTCCTTCCC CACATCTGTT TATGGAAGAA AATGAAGTTT GGGGTGTGGT TTGAGGAATC 240
AGCTAGATTC TTATGATCTG TCACATGCTT GGATGTTGGG GAAGCATTTG GAGAAGCTCA 300
TGTGACTTGT CCTAGATTGG GGATTTTAAT TGAGACAGAT GATGTTTATC GGGCATCCCA 360
CCACCTGAGA GTTTTAGCAA CAGAGTCACA TGTGAGTCCA TCAGAACTTA CGGCATTGAT 420
TCAAGTGCTG TCATAAATAA CCAGGACTGC TGTTTTTGGT TACTTTTAAA GACAGTTTCA 480
TCTGGACTTT CTGGGCATAT CCTCCTTCAG CAAAACCACA TTAGGCTGGG AAAACTATTC 540
TGCCTGGAAG TAATGACAAC TTGCAACCAA CAAGCTTATA AAAATACAAA GAATTCTGGA 600
GCCTATGGCT TCCATTACAT TATTCTTTTA TAGCCTTTTA TGTTCATTAC CGCATCCCAG 660
AGGTGAGAGT CAGACACAAA TATGAAAATA GGTTTCAATG TTGGAGAGGT AAATCCTAAC 720
AGGAAAGGGG TAGGAAAAGA TATAATCCCC CAATATTAAA ATAAAGATAT TGAAGAAGAA 780
GGATGGGAGA GACTAGGGCT GTGTCCTTCC TTTTACTCAC CAAAAGAGAA AGTAAGCTCC 840
TATTTGAGTC AATAGATATT GAGGTCTTGT TATTTGCCAC CAAAGACAGT CTTGTGAGAC 900
TAAATAGCTA GTAATTCCCT ACCCTGGCAC ACATGCTGCA TACACACAGA AACACTGCAA 960
ATCCACTGCC TCCTTCCCTC CTCCCTACCC TTCCTTCTCT CAGCATTTCT ATCCCCGCCT 1020
CCTCCTCTTA CCCAAATTTT CCAGCCGATC ACTGGAGCTG ACTTCCGCAA TCCCGATGGA 1080
ATAAATCTAG CACCCCTGAT GGTGTGCC 1108

(2) INFORMATION FOR SEQ ID NO:3:

(i) SEQUENCE CHARACTERISTICS:
(A) LENGTH: 48 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:3:

CACACTTTGC TGCCGAAACG AAGCCAGACA ACAGATTTCC ATCAGCAG 48

(2) INFORMATION FOR SEQ ID NO:4:

(i) SEQUENCE CHARACTERISTICS:
(A) LENGTH: 1427 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:4:

GTAACGTTTG CAACTTCCTA GATCTTTTAG CTTTTCATTC CTGTCAATTC TCTGAGTATT 60
AGGGATGTAG TGACTTGAGG ATCACAATAA ACTTTTAGCC TCTGCAGATG AAAACAGAGA 120
TGCACTTCTT AGGTCATTCC CTGGCTAAAT AAAATCTGCC TGGAAATCTG TAGAATTCCT 180
TGTATGATTT ATATATATAC ATACATGATT GTTAGTAAAA GCAAAGTATA TAGGGAATCA 240
TTTCCCCATC CTTCAAGAGT GGCCTTTCTG CAGTGTTTTC TACTTTGGCC AACAAGGATC 300
AAAACGGTTA ACTCCTTAGT GAGGAGGAGG AGAGTGGTAT GGGGAGGTAG TAGCTCAGTG 360
CTTCCTGTTC ACTGAGACAT CTCAAAGCCC TTAACACTCT AGTTTTTAAA TGTCCTACTG 420
GACATTTTGC CAGTTTGCAA AATTACATGT AAATGGACTA TAAGCAATTG TGTAAGCCAT 480
ATGTCATGCT GCAGGCTGCA AATTGTTCTT AAAATGGAGG ATTTGTAATT AAGAAAGCCA 540
ATGCAAGAAA TGAGTGAAGC TAACTAGAGT AAACTTATGA AAAGCTGTGA ATTTCATCAT 600
CATAGAACAT TGCTTTTCAG TCTGAACATT CTTCTAACAA ACCTTGGATC TGAGGCTTCT 660
TGTCCTTTGC GGCAGCCACA GTGGGTTTTT GTTGTTAGGG GAAAATAAAA AACCTTGCCC 720
GCAGCATCTG GTTAAGATTA GGGCAGTTTC CTGCCTAAGG AGGGAAGGGA GAGAAAAAGG 780
AAGAAGAAAT GCATAAGGAG AATGAGGAGA TATACAATGT CTCAGAAAAC AGGAAACATT 840
GTCCTATTTT CCCTTGTCCT CTTCTGACAA GATCTGGGAA AGTACCAGAA TTTAGGCACG 900
AAAGAGAAGA ACGCCTCGAA GAAATGATCA GGAAGCAAAA CTTAGACGGA AATCTCTCCT 960
TTGTGTATTC TGAACCCCAC TACCACCTTG CTATTTGTCT GTCTCCAAGC CTGCTAGGGA 1020
CCCTGGAGGA AACGCACTGA GCCCATTCTG ATTGTCCAGT TTCTATCCCC CATTTCTGGT 1080

TGTGTACGTG TGTGTGTGTG TGTGTGTGTG TGTGTGTGTG TGTGTGTGTG TGAGAGAGAG 1140
AGAGACAGAG AGAGAAACAG AGAGAGTGTG TGTTGCCTAA ATCTCCCGAG AGAGAGAGAG 1200
AGAGAGAGAG AGAGAGAGAG AGAGAGAAAA GAGAGAAATG GCTAAATCCC CCTAGATCAA 1260
AGTCCTTGGA ACCAGATGTA CCAGCATCCT ATCTAAACAC AGGCCCCTCC TGACTATCAT 1320
TGTTTTATCA CCCTTTTTCC GTCTACCTTT CTCTTCCTCA TAAAGCCTAG TTTTCCTCTG 1380
TTTCCCTGCC AAATGGAAGA GTTTTCCCTA ACTACATTCT TCTGCAG 1427
(2) INFORMATION FOR SEQ ID NO:5:

(i) SEQUENCE CHARACTERISTICS:
(A) LENGTH: 121 base pairs
(B) TYPE: nucleic acid
(C) STRANDEDNESS: single
(D) TOPOLOGY: linear
(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:5:

GATGTGGGGG CTCAAGGTTC TGCTGCTACC TGTGGTGAGC TTTGCTCTGT ACCCTGAGGA 60
GATACTGGAC ACCCACTGGG AGCTATGGAA GAAGACCCAC AGGAAGCAAT ATAACAACAA 120
G 121

(2) INFORMATION FOR SEQ ID NO:6:

(i) SEQUENCE CHARACTERISTICS:
(A) LENGTH: 462 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:6:

GTGCCTGGGG TCCTGGAGGG GGCATGGCAG GAAGGCTGAG ACCTGAGCTC TCTCATCTTA 60
GCTTCCAGAC TCCCTTCTTC AATCCAAATG CTTTATTCCA AGCAAATCAG TCCCTCTTCC 120
CTAACTCATG TTAACATACG GTTTTCATTC CTATGCTTCA ATCATCCTCT TGTCAAACTT 180

GTATTCCTTC CCTTTGGTTT TATAAGTGTG TAACATTCCT CTTTTGGGAA GAGTCCCAAG 240
ATTAATGCTG TTAATCCATA AGCAATTTTT CTGTCTCTCC AGAGCTTGTG TGGTTGTTTA 300
CATATTATCT CTCTTCTTGC AGGCTCTTAA TTCCATGGTT AGTTCCCCAA CTAAACTGTA 360
AACTTTTATG ATTGTGAGTT TCCTTTATTC TCCTAAAACC CTTCACAATA TTACATATGA 420
ACTGTAGACA GTCTATACAA GTACTGACTA TGCTTTGTTT AG 462

(2) INFORMATION FOR SEQ ID NO:7:

(i) SEQUENCE CHARACTERISTICS:
(A) LENGTH: 123 base pairs
(B) TYPE: nucleic acid
(C) STRANDEDNESS: single
(D) TOPOLOGY: linear
(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:7:

GTGGATGAAA TCTCTCGGCG TTTAATTTGG GAAAAAAACC TGAAGTATAT TTCCATCCAT 60
AACCTTGAGG CTTCTCTTGG TGTCCATACA TATGAACTGG CTATGAACCA CCTGGGGGAC 120
ATG 123

(2) INFORMATION FOR SEQ ID NO:8:

(i) SEQUENCE CHARACTERISTICS:
(A) LENGTH: 85 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:8:

GCAAGTATAG CTTCAGCTCC TGTCCCACCT GCACCATTTG CTTTAGTTCC CTGCTGATGC 60
CTGGCCTCTT TCTTCTTTGT CTTAG 85

(2) INFORMATION FOR SEQ ID NO:9:

(i) SEQUENCE CHARACTERISTICS:
(A) LENGTH: 156 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:
(xi) SEQUENCE DESCRIPTION: SEQ ID NO:9:

ACCAGTGAAG AGGTGGTTCA GAAGATGACT GGACTCAAAG TACCCCTGTC TCATTCCCGC 60
AGTAATGACA CCCTTTATAT CCCAGAATGG GAAGGTAGAG CCCCAGACTC TGTCGACTAT 120
CGAAAGAAAG GATATGTTAC TCCTGTCAAA AATCAG 156

(2) INFORMATION FOR SEQ ID NO:10:

(i) SEQUENCE CHARACTERISTICS:
(A) LENGTH: 1624 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:10:

GTACTCTCCT TTCTTCTGGG TGTGCATATG TAATCTGGCA TGACCTTTTC CTTTTTCTGC 60
TGCTTTGTTC TTGAGGTGAA AGGGCACCAG GAAAAGAGGG CAAGGAATTA AGGTACATCT 120
CCCCATTCCC ATTCTGTTAT TTAACCTCAT TTGTTTCTGT ACATTTGGGT TGTTTCTGGT 180
TTTTCTTTTT CTTTTCCCTT TTTTTTTTTT TTTTTTTTTT GAGATAGAGT CTCACTCTGT 240
CGCCCAGGAT GGAGTGCAGT GGTGCAATCT TGGCTCACTG CAACCTACAC CTCCCGGGTT 300
CAAGCGATTC TCCTGCCTCA GCCTCCTGAG TAGCTGAGAT TACAGGCACG CGCCACTACG 360
CCTGGCTAAT TTTTCTATTT TTATAGAGAT GCGTTTTCAC CATGTTGGCC AGGCTGGTCT 420
TGAACTGACC TCAGGTGATC CACCTGCCTC AGCCTCCCAA AGTGCTGGGA TTAGAGTCAT 480
GAGCCATCGC GGCCTGGTTT TTCTTTATTA CAAATAGTGT TGCAATAAGC ACCCTTGTGC 540

ATATGTTTTT GTGCACATGT ACAAATATTT ATGCAAAATA AGTCCTAAAA TTGGAATTGT 600
TAGGTCACAA ATAATCCTTT CCCCCCCCCC AAATTTTTTT TTTTTTTTTG AGACAGCGTC 660
TCTGTCACCC AGGCTGGAGT CCAGTGGCGC AATCATGGCT CACTGCAGCC TCAACGTCTC 720
AGGCTCAAGT GATTCTCCAA CCTCAGCCTC CCTAGTAGCT GGGAATTAGA AGCACATGCC 780
ACCACACCCA GCTAATTTTA AAAAATTTTT TGTTAGAGAC AGGGTTTTGC CATGCTACCC 840
AAGCTGGTCT CAAATTCCTG GGCTCAAGCA ATCTGCCCGC TTCGGCCTCC CAAAGTGCTA 900
GGATTACAGA CATGAGCCAC CATGCCCAGC CCAAAAAAGT TTTTGCAATC TTACATTCTT 960
ACTAGCATGA GAATGTCAGT TTTTTCACAA CCCAAACAAC ACAGGATTGT ATCAGCAAGA 1020
TAAACAATTG ATTTAACGTT CATTTAACAA ACACTTTTTG ACCCCCAGAA CCTACCAGAT 1080
GCAGTGTTAG GCAGCAGAGA CTCAAGATGA CTAAGACACA ACCTGTGTCC TCAGGAAATC 1140
TCAATCTAAA AAAATAGAAC AGGAAAGAAA GAAAAATCTA CAATCTAGCT GCACAAACAA 1200
TAATAGCTAA TACTTTTTGA GATTTTATTG TTTGTCAGGA ACTTCTTAAC TCTTTACATG 1260
AGTTTAAATA TTTAATCCCT TATAACAATA TTTTATGCAT AGAGAAACTG AGACACAGGC 1320
AAATTTAGTA ACTTACCCGG GGTCACATAG CTACTGGGTG GCAAAGTCAG GGTTAGCTCC 1380
CAGGACAAAT GCCTCCACAG CTGGTACTGT GCTCTGCTTT ACTGTAGCTA ATAGTAAAAA 1440
TGGTAGCAAA AATCAATAGC AGTAGAACAG TGCAACAGAT ATTAAGCGGA AGAGGAAGAC 1500
TCACAACAAT GACAACATTT GTGCTGAAAT TTTTAAGAAC ACATGGAATT TCCTTCAGCC 1560
GGGTAGAGAG AAGATATAGA AATGTAAACA CCAAAGATTC ATAGTTTCTC TGTATCCCTT 1620
TCAG 1624

(2) INFORMATION FOR SEQ ID NO:11:

(i) SEQUENCE CHARACTERISTICS:
(A) LENGTH: 219 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:11:

GGTCAGTGTG GTTCCTGTTG GGCTTTTAGC TCTGTGGGTG CCCTGGAGGG CCAACTCAAG 60
AAGAAAACTG GCAAACTCTT AAATCTGAGT CCCCAGAACC TAGTGGATTG TGTGTCTGAG 120
AATGATGGCT GTGGAGGGGG CTACATGACC AATGCCTTCC AATATGTGCA GAAGAACCGG 180
GGTATTGACT CTGAAGATGC CTACCCATAT GTGGGACAG 219

(2) INFORMATION FOR SEQ ID NO:12:

(i) SEQUENCE CHARACTERISTICS:

(A) LENGTH: 4326 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:12:

GTGAGATTGC TCCACACAAT TATACAGCTC TGTTGGCTCC TCCTTCCCCA GCATGATGTT 60
TTGTACTGGA AACAATTCCA GAAATACTGT TTTCTGTTAT CCTATCCTGC TTTCTTGATG 120
GAATAATTTC CCACAGAAGG CCAAGAAGAT TTCCACAATC TGGGGGAATT TAGGGAGCTT 180
AAGCTACTAT AGCTCCTATT TGCATCTCTG CCATGGAGAG AAAACAGAGG CTAGGCTACC 240
TACCCCATAG ACTTCCGAGC TGGGTTCTAT AACCCTCTGC TCAATTCCTC ACTCCCACAA 300
CAAACCCACA AACCCACCAT GCTATTTTCA CAAATTGTGT GGCTTTATTT TATATGATCT 360
CAGTGTGAGT TTTCAGAACA TTTCAGCAAA TTATGTAAGT TTACATGCTA ACATCTATAA 420
AATGAGAGAA AAAACAAGTT GCTTCATATA AGAGATAAGG GATTAACTCA GTTCCTCCTG 480
CATGATCCTC TAGTCATAGG AAGGAAATCA TATCTGAAAG GGAGGCAACC TGAGGGGTTT 540
TTTATACACA TAGGGCTGGG TCTGATAGAC AATATAATGT AGGGCCTTCA CAACAGAAAC 600
CTCTGAAACA GGGACAGCAA GTTTGAGAAT AAAAATGATG GCTACTGTGT TCTAAGCCGT 660
GTCCTTAGTG CATTTTTTCT TTTTCTTTTT TTCATTTAAT CTCATAACAA CTCTGTTAGG 720
TAGACTTATC TTGAATGTAT AGGTGAGGAA ATGGACACTT AAGGAGATAA GACAGTATAA 780
TTCATACCAC TAGTATGTAA CAATGTAAGA TGTATCTACC AGGGATGTTT ATCTTCTGCA 840
AACATTCCTA GGTATATCTC CCATGCACAT GTGCAAGAAT TTCTTACTAG GATATAATGC 900
CTTGGAACTG AATTGTCTGG GTCTTAGGGT ATGTCTGTCT TCAACTTTAC TACACAATGT 960
CAAATTGTTT GCCAAAATAT TTGGAAAAAT TTATACCTGC AATGTGTAAG AAATCCCCTT 1020
CAATCACCTT TTTATCAGTA TGTTTATCTG GCCATTTGCA TTTCTTCTTC AGTGAATTAA 1080
CTGTTTTTAT CTCTTGCTCA TTTGTTTTTC TTTTTATTTT TTTGAAATAG GGTCTTACTC 1140
TGTTGCCCAA GGCTGGAGTG TGGTGATACA GTCATAGCTC ACTGCAGCCT CCACTTCCGG 1200
GCTCAAGCAA TCCTCTCGCC TCAGCCTCCC AAATAGCTAG GATATAGGTG CATGCCATCA 1260
TGCCCACCAA TTTCAAAAAA CCTTTGAAAT TTTTTTTTTG TAAAAGCTAG GCATGGTGGC 1320
TCATGCCTGT AATCCCAGCA CTTTGGGAGG CTGAGGTGGG AGGATCGCTT GAGCCCAGGA 1380
ATTGGAGGTC GGCCTGATAC AACATAGCAA GACCTCATCT CTACAGAAAA AATTTTTAAA 1440
AGTAGCCAGG TATGATGGCG TGCATAGTTC TAGCTACTCC GGAAGCTGGT TGGGAGGACA 1500
ACTTGAGCCT GGGAGTTCAA GGCTGCTGTG AACTGTGATC ATGTCACTGC TCTCTAGCCT 1560
GGGTGACAGA GTGAGACCCT GTCCCCAAAA ACAACAACCG TTTTTTTTGG TAGAGACATT 1620
GTCTCGCTAT GTTGCCAAGG CTAGTCTCAA ACTCCTGGGC TCAAGCAATC CTCCCACCTC 1680
CCAAAGTGCT GGGATTATAG ATGTAAGCCA CCATGCCTGG CCTACCCTTT TTTTTTTTTT 1740
TTGAAATGGA AGTTTTGCTT TTGTCACCTA GGCTTGAGTG CAGTGGCGCG ATCTTGGCTC 1800
ACTGCAACCT CCACCTCCTG GATTCAAGCA ATTCTCCTGC CTCAGCCTCC TGAATAGCTG 1860

GGATTATAGG CACCCGCAAC CACGCCCGGC TAGTTTTTGT ATTTTTAGTA CAGACAGGGT 1920
TTCACCATGT TGGCCAGCTG GTCTTGAACC CCTGACCTCA GGTGGTCCGC CCGCCTCGGC 1980
CTCCCAAAAT GCTGGGATTA AAAGTGTGAG CCACCATGCC CCACCCCTTA CTCATTTTTA 2040
ATTGGATTGT TTTTTCTCTT TCTTAGCGAT TCTTAAAAGT TTAAAGAGAA TATTTGGATA 2100
CAATACTATG TATTTAAAAG TTGAGGTCTG TCTTTCCATT CTTCTCACGA TGTCTTTCAA 2160
TCTAGAAAAG TTAATTTTAA TAGGCCTGGC GCGGTGGCTC ACGCTTGTAA TCCCAGCACT 2220
TTGGGAGGCT GAGATGGGTG GATCACAAGG TCAGGAGATG AAGACCATCC TGGCTAACAT 2280
GGGTGAAACC CTGTTTCTAC TAAAAATACA AAAAAATTAG CTGGGCGTGG TGGCAGGTGC 2340
CTGTAGTTCC AGCTACTCGG GAGGCTGAGG CAGGAGAATG GCGTGAACCC GGGAGGTGGA 2400
GCTTGCAGTG AGCCGAGATT GCACCACTGC ACTCCAGCCT GGGCAACTGA GCAAGACTGC 2460
GTTTCAAAAA AAAAAAAAGT TAATTTTAAT ATAGTAAAAT TAGTAAAAGG ATTAATTTTC 2520
CCTTTGCAAT TTTTGTAATG TGTTTTATTC GTTTATGAAT GGAGAAAGGT AAGAAAAAAT 2580
AAAATTTAAA AAAGAAGAGA TGTGGCCAGG TACGGTGGCT CACACCTATA ATCCCAGTAG 2640
TTTGGGAGGC TGAGGCAGGC AGATCACTTG AGGTCAGGAG TTTGAGACCA GCTGGGATAA 2700
CATGGTGAAA CCCCATCTCT ACTAAAAATA CAAAAATTAG CCAGGTGTGA TTGCGCACGC 2760
TTGTAATCCC AGCAGGCTGA GGCAGGAGAA TTGCTCGAAC TCAGGAGGCA GAGGTTGCAG 2820
TGAGCCAAGA TCATGCCATT GCACTCCAGC CTGGGTAACA GAGACTCTGT TTCAAAAAAA 2880
TAAAAAGATA AAAAAGGAAG AGATCTGATA GGGCGGCCAG ATAAACATTT TAAAGGGGAT 2940
GGTATTATAA GTTTGTTCCC AGCATAATGC CAGGTTATTC TGACTTTAAA GTATCATCAC 3000
ATAATATCTT TTTGAGTCAA TTTCCAAGAT ATTCTGTTTC ACTTGTAATT CTGTGTAATT 3060
TTTGGCACCA GGAGGCATCA GGGATTTGGA GCACATGGCA GAAACAAAGG CATCTTGAAA 3120
AATATCAAGG CAGTAGACCA CTGTAATCTT AAAATGGCAT ATCAAATGCT GCTATTGCTG 3180
TTAATATTTA GATAATGTTA GATAATGTAT TTTTTTAGAG GGTATCTCAC TATCTTGCAC 3240
AGGCTGGAGT AGAGTGGCTA TTCACAGCAT GATCACAGTA CACTAAAGGC TCAAACTCCT 3300
GGGCACAAAC AATCCTCCTG CCTCAGCCTG CTGAGTAGTA GATAATAAGT TCTTGTGGAT 3360
GCAACCTTAG GGTTCTGAAG GGGTAGTCTG TAGGAAAATG AATTGCTGAA AAGAATACAC 3420
CACCTTAACA TGGGCTATTA TTCGATTCCA TAATTGTGGC TTGCCAATGA AACATTGCTA 3480
ACTACCTGTA AAATATAGTG TTGGAAGTCA TAGGCTAAAT TGCTAAGTTC TTTAATCTAT 3540
TTTAGTGTCT TGTTATGTAC TTTTATATTT TGTCTTTGAT GAGAGCACAA GGATCACACC 3600
AGTTCCCCTG ATATAGGTGC AGAGGGCCCA GGTCTTCCCT CTAGCTAAGC CTTGGCCTTG 3660
GCCTCCTACC CACACAGCAG CTGGTGCCTT CCTGCCCCCT GAGGCTAATA CATACTATGT 3720
GGCCAGAAGA TGGTTTATGC TTTTTAAAAA AATCTTATTT CAGAAATCTT TCCCTACTGT 3780
TTTCCTCCCA CATTTATGTC TTAAAACACC TGTAGGGGAT TTTTTTTTTT TTTTTTTTTT 3840
TGAGATGGAG TCTCGCTCTC GCCCAGGCTG GAGTGCAATG GCGCGATCTT GGCTCACTGC 3900
AAGGTCTGCC TCCCAGGTTC ACGCCATTCT CCTGCCTCAG CCTCCCCAGT AGCTGGGACT 3960
ACAGGCGCCC GCTACCACGC CTGGCTAATT TTTTTGCATT TTTAGTAGAG ACAGGGTTTC 4020
ACTGTTAGCC AGGATGGTCT CGATCTCCTG ACCTCGTGAT CCACCCTCCT CAGCCTCCAA 4080
AGTGCTGGGA TTAACAGGCA TGGAGCCCCA CCGCACTGGC CTGTATTTGT GAGGAAGAAC 4140
AGACCCTCTT TAGAAGCCCT AGACTGCTGC CTCTGTTAGT TCACTGGCAT CACTCAAAAT 4200
ATTGGTTGAG TTTCTTACTC ACTGAGTTGG TTTTTATGTG TGGTGGAAGG CGGGAATCCT 4260
CTTTTCATAT TCGTTCTCAT TGCCTATTGC TTTGTCCTAG TCCTATTACA ATCTTGTTTC 4320
TTCCAG 4326

(2) INFORMATION FOR SEQ ID NO:13:

(i) SEQUENCE CHARACTERISTICS:
(A) LENGTH: 166 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:13:

GAAGAGAGTT GTATGTACAA CCCAACAGGC AAGGCAGCTA AATGCAGAGG GTACAGAGAG 60
ATCCCCGAGG GGAATGAGAA AGCCCTGAAG AGGGCAGTGG CCCGAGTGGG ACCTGTCTCT 120
GTGGCCATTG ATGCAAGCCT GACCTCCTTC CAGTTTTACA GCAAAG 166

(2) INFORMATION FOR SEQ ID NO:14:

(i) SEQUENCE CHARACTERISTICS:
(A) LENGTH: 270 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:14:

GTAAGAAGCT GCTGATCCTA TACAGCACTG TCTTTTATGA TACAAACTTG ATGGTTTCTC 60
GAAGGACCTT GGGTATTTTC AGTACTTAGT TTTTGTATTC ACATGGAGGT GGCCAGAGAG 120
AAATTAACAA CTGCTGCAGT ATGGAGCAGC ATCTCTGTGG TAAACCCTCC TGACACGGAT 180
GGAATTCTTC AAACAGTCTC CTAGACTGGG AGATCCCACA GGGTGACCCT TGGATTGCAT 240
AGAGCCTCAC GCTGGTAGTT TGTATTCTAG 270

(2) INFORMATION FOR SEQ ID NO:15:

(i) SEQUENCE CHARACTERISTICS:
(A) LENGTH: 106 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:15:

GTGTGTATTA TGATGAAAGC TGCAATAGCG ATAATCTGAA CCATGCGGTT TTGGCAGTGG 60
GATATGGAAT CCAGAAGGGA AACAAGCACT GGATAATTAA AAACAG 106

(2) INFORMATION FOR SEQ ID NO:16:

(i) SEQUENCE CHARACTERISTICS:
(A) LENGTH: 2270 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:16:

GTAATGATGG GAACACTACT TTTGTTATTC AGTCACCCTT TTAACACTCA ACCTCACCTC 60
CAGCTTCCCG ATATTCCTTT CTCTGTCCCA AATCAAGAAA AAATTATCTC AGAGTTCTCA 120
CTTCTATCTT CTCAGTCAGA GGCTCTTAAT TCTCAGTCTG ACACTTAATG GCCAGTGTGT 180
TAGTCCATTT TGCATTGCCA CAAAAGAATA CCCGAGACTG GGTAGTTTAT AAAGAAACGA 240
GGTTTGTTTG GCTATACAAA GCGTGGCACT AGTATCTGCT CAGCCTCTGA TGAGGCCTCA 300
GAGCTTTTAC TCATGGCAGA AGGCAAAAGA GGGAGCAGGC ATGTCACATA GTGAGAGAGG 360
GAGCAAGAGA GAGAGGGAGG TGCCGACTCT TTAAAGAACC AGCTCTTGCA TGAACTAATA 420
GAGTGAGAAC TCACTCATCA CCAAGGCGAT GGCACCAAGC CATTCCATGA GGAATCCACT 480
CTCATAACCC AAACACCTCC CACTATGCCC CACCTCCCAC ATTGGGGATC ACATTTCAGC 540
ATGAGACTGG GAGGGGACAC ACATCCAAAC CATATCCGCC AGACAATAGT GCTCAATTAT 600
GTGCTGGGCA GATGCTCCCT GTGTGCAAGG TGCTTAGTGA CATACATAAA CCAACGAGCA 660

GATGACACCT TCAGTGAGCT CAGAGCCCAA TAAGACAGAC CTAACTAACC ATGAGATAAA 720
GCAGTACAAA GAACCAGCAG GAGCTTTGGA ATTACGTATT TTTACTTTCT TTTGTCTCTA 780
ATGTGATCAG TTTCTTAGAT GGTTTCCATT AGCAATCTGT CTTTAACAGT AGGGGAGCAG 840
CGTTAAAGGT TTAATATTCC TTTTGAACAG TTTTTTTCCT TCAAAATACA CTTAAGATAC 900
ACGTATATAA GAACTTGCCA AAGATTGTGA AGAGAAACAT TTTTTAGAAA TAAGATATAA 960
ACAAAAAAAG TTAGTGTTAC TTTCCTATGT TGGGGAACAA AGAAAACTCC AGGGTACCTT 1020
GCTTCCCATT TCTCTTTAGC ACCTTGTGAC TTTTGGGGAG GGGCAGATTG ATAACAATTA 1080
TAGTTTTCCT TTCCTGGCTG ATCACCATTA ACCTGGCAGC AGCACTGGCT AAATCTCCTG 1140
TCCTTAGTGC CCTCCAAGGA GCAGGAGCCC TAGACTCTGG GTCGCTGACA GACTCACGCA 1200
GTGGTGTTGT TCAAACCTGA AGCAACTTTT TATATCACAG TTCCAACTCA AGGTGAACCT 1260
GAGCATCTTC CCAAGTCTCC CACAGCTTCT GTCCTGTGTT GTCCCTTCTC TTGACTCCCA 1320
GGTCCAAGCA CTTACCCTGT TCTTTCATGA TCAGGTACCA TGTGTGGAGA TAGCTTCCAA 1380
GAGAGCTGGG AGGAAGAAAG GACACACCCG GGCAGGATCA GGAACACTGG GGGCCCCTGG 1440
AGAAGGGGAG AGTGGGGGAG GGTACAGGTT TTAAATAAAA TGTGTTGGTA ATTAGAGAAT 1500
TGCTGGTTGG GGAAAGAGGT CTGAAAACAA TTCAGGAAGA TAAACAAGAC AATCTCTCCT 1560
CTCTCCTCTT TCTCACGTCG TCTCTCTTGT CTTCTAGTCT CGCTACTCAT TTCCTTAGTA 1620
ATCTCATCCA CTCTCATAGT TTCATCCATC TCTCCTATGG GGTTTACCCC CAAATCAAGA 1680
TCACCAGCTT CAGCCTCCTT CTTATGCTCT AAACTCACAT TTTCAAGATT AATATTCCCC 1740
AAATACAGCT CTGATCATAT CACTCTCCCA CTCAAAATCC CTCACTGGCT CCTCACGATG 1800
ATGGGTCACA GAGTAAAGGT GAAGCTTTTT AACCTTGCAG TAAAGGTAAT TCAACCTGAT 1860
CTCAATCTGC CTTTCCAGAC ATCTCTCCCA CTACACCCTG TTAGGCACAC TGCTTTTCAG 1920
CTACATGATC CTAACAGTGC CCCACACTTT CCTGCCTCTG TTGTTCATTT CACACCCTTC 1980
CACTGGCATC CCCTTCCCAC AGGTCGAAAT TCTACTTAGC CTTTTGGCTC AGCTCAAATG 2040
CCACCTCTTA CATCAAGCCT CTAAGATTCT CTTGATCAGA AGGAATCTTT CCCTCCTTTG 2100
ATACCTACAG TATTATGCCT TCTCCCTATT TCTTGACTTT AAACTCTTTA AAGTTAAAAA 2160
ACATCATATT CATTTTTGTG TACCATCAGT ACCTCGCACA ATACTCAGTA AATATTTTAA 2220
TGAATAAATA AACTGAGAGT ACTAAGTATT TTTCTTGATT GGTCTTACAG 2270

(2) INFORMATION FOR SEQ ID NO:17:

(i) SEQUENCE CHARACTERISTICS:
(A) LENGTH: 97 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:17:

CTGGGGAGAA AACTGGGGAA ACAAAGGATA TATCCTCATG GCTCGAAATA AGAACAACGC 60
CTGTGGCATT GCCAACCTGG CCAGCTTCCC CAAGATG 97

(2) INFORMATION FOR SEQ ID NO:18:

(i) SEQUENCE CHARACTERISTICS:
(A) LENGTH: 598 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:18:

TGACTCCAGC CAGCCCAAAT CCATCCTGCT CTTCCATTTC CTTCCACGAT GGTGCAGTGT 60
AACGATGCAC TTTGGAAGGG AGTTGGTGTG CTATTTTTGA AGCAGATGTG GTGATACTGA 120
GATTGTCTGT TCAGTTTCCC CATTTGTTTG TGCTTCAAAT GATCCTTCCT ACTTTGCTTC 180
TCTCCACCCA TGACCTTTTT CCACTGTGGC CATCAGGACT TTCCCCTGAC AGCTGTGTAC 240
TCTTAGGCTA AGAGATGTGA CTACAGCCTG CCCCTGACTG TGTTGTCCCA GGGCTGATGC 300
TGTACAGGTA CAGGCTGGAG ATTTTCACAT AGGTTAGATT CTCATTCACG GGACTAGTTA 360
GCTTTAAGCA CCCTAGAGGA CTAGGGTAAT CTGACTTCTC ACTTCCTAAG TTCCCTTCTA 420
TATCCTCAAG GTAGAAATGT CTATGTTTTC TACTCCAATT CATAAATCTA TTCATAAGTC 480
TTTGGTACAA GTTTACATGA TAAAAAGAAA TGTGATTTGT CTTCCCTTCT TTGCACTTTT 540
GAAATAAAGT ATTTATCTCC TGTCTACAGT TTAATAAATA GCATCTAGTA CACATTCA 598

(2) INFORMATION FOR SEQ ID NO:19:

(i) SEQUENCE CHARACTERISTICS:
(A) LENGTH: 459 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:19:

TTTTGTGTTG GATACTGTGT TAGGTGCTGG AGGAAAAAAG ATGAATAGAA CATCTTCTAT 60
GTACTTCATG CGCTCACAGT CTGGTTGTAG AGACTGTCAC ATAAACATTT CATCCCAATT 120
CATTTATTTG TTCATTCCTT CAGCCAATAT ATATTGAGTT CTTACTCTGT GCCAAGAACT 180
GTACTACATT TCTGGGATTA AGTGGATATA AGGAGATCTC AGTGTTTAAT CTGCCTGAGG 240
GGAGACTAAA TTAAGTGACA TGGAAACTTG GGTCTTGAAA AACATTTTAA GGTTATTTTT 300
TCTTTTCTCT CTCTCTCGCT CTGTCTTTCT CTCTCTTTCG TCAGGGTCTC CCTCTGTTGC 360
CCAGGCTGGA GTCAGTGGCA CTCATAGCTC ACTGCAGCCT TGATCTCCTG GGCTCAAGAG 420
TTCTTCCCAC CTCAGTCTCC TAAGTAGCTT GGACTACGG 459

(2) INFORMATION FOR SEQ ID NO:20:

(i) SEQUENCE CHARACTERISTICS:
(A) LENGTH: 329 amino acids
(B) TYPE: amino acid
(C) STRANDEDNESS: single
(D) TOPOLOGY: linear

(ii) MOLECULE TYPE: protein
(iii) HYPOTHETICAL: NO
(iv) ANTISENSE: NO
(v) FRAGMENT TYPE: N-terminal
(vi) ORIGINAL SOURCE:

(xi) SEQUENCE DESCRIPTION: SEQ ID NO:20:

(2) INFORMATION FOR SEQ ID NO:21:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:21:

CCGAAACGAA GCCAGACAAC                                                20

(2) INFORMATION FOR SEQ ID NO:22:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:22:

GCTCACCACA GGTAGCAGCA G 21

(2) INFORMATION FOR SEQ ID NO:23:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:23:

CTGCTGCTAC CTGTGGTGAG C 21

(2) INFORMATION FOR SEQ ID NO:24:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:
(xi) SEQUENCE DESCRIPTION: SEQ ID NO:24:

CCCAAATTAA ACGCCGAGAG 20

(2) INFORMATION FOR SEQ ID NO:25:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:25:

CTCTCGGCGT TTAATTTGGG 20

(2) INFORMATION FOR SEQ ID NO:26:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:26:

GGTACTTTGA GTCCAGTCAT C 21

(2) INFORMATION FOR SEQ ID NO:27:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:27:

CCAGACTCTG TCGACTATCG 20

(2) INFORMATION FOR SEQ ID NO:28:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:28:

CACATATGGG TAGGCATCTT C 21

(2) INFORMATION FOR SEQ ID NO:29:

(i) SEQUENCE CHARACTERISTICS:
(A) LENGTH: 21 base pairs
(B) TYPE: nucleic acid
(C) STRANDEDNESS: single

(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:29:

GAAGATGCCT ACCCATATGT G 21

(2) INFORMATION FOR SEQ ID NO:30:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:30:

GTTACATTAT CGCTATTGCA C 21

(2) INFORMATION FOR SEQ ID NO:31:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:31:

GCAAAGGTGT GTATTATGAT G 21

(2) INFORMATION FOR SEQ ID NO:32:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Genomic DNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:32:

GCCGTTGTTC TTATTTCGAG C 21

## Description

### RELATED APPLICATIONS

This application claims the benefit of U.S. Application No. 60/019,942 filed June 14, 1996, U.S. Provisional Application No. 60/020,273 filed June 17, 1996 and U.S. Provisional Application No. 60/026,083 filed August 26, 1996.

This invention relates, in part, to newly identified polynucleotides and polypeptides; variants and derivatives of the polynucleotides and polypeptides; processes for making the polynucleotides and the polypeptides, and their variants and derivatives; agonists and antagonists of the polypeptides; and uses of the polynucleotides, polypeptides, variants, derivatives, agonists and antagonists. In particular, in these and in other regards, the invention relates to polynucleotides and polypeptides of human cathepsin K, especially genomic sequences of cathepsin K, and most especially promoter and intronic sequences.

### BACKGROUND OF THE INVENTION

Bone resorption involves the simultaneous removal of both the mineral and the organic constituents of the extracellular matrix. This occurs mainly in an acidic phagolysosome-like extracellular compartment covered by the ruffled border of osteoclasts. Barron, et al., J. Cell Biol., 101:2210-22, (1985). Osteoclasts are multinucleate giant cells that play key roles in bone resorption. Attached to the bone surface, osteoclasts produce an acidic microenvironment between osteoclasts and bone matrix. In this acidic microenvironment, bone minerals and organic components ate solubilized. Organic components, mainly type-I collagen, are thought to be solubilized by protease digestion. There is evidence that cysteine proteinases may play an important role in the degradation of organic components of bone. Among cysteine proteinases, cathepsins B, L, H, and S can degrade type-I collagen in the acidic condition. Etherington, D.J. Biochem. J., 127, 685-692 (1972). Cathepsin L is the most active of the lysosomal cysteine proteases with regard to its ability to hydrolyze azocasein, elastin, and collagen.

Cathepsins are proteases that function in the normal physiological as well as pathological degradation of connective tissue. Cathepsins play a major role in intracellular protein degradation and turnover, bone remodeling, and prohormone activation. Marx, J.L., Science. 235:285-286 (1987). Cathepsin B, H, L and S are ubiquitously expressed lysosomal cysteine proteinases that belong to the papain superfamily. They are found at constitutive levels in many tissues in the human including kidney, liver, lung and spleen. Some pathological roles of cathepsins include an involvement in glomerulonephritis, arthritis, and cancer metastasis. Sloan, B.F., and Honn, K.V., Cancer Metastasis Rev., 3:249-263 (1984). Greatly elevated levels of cathepsin Land B mRNA and protein ate seen in tumor cells. Cathepsin L mRNA is also induced in fibroblasts treated with tumor promoting agents and growth factors. Kane, S.E. and Gottesman, M.M. Cancer Biology, 1:127-136 (1990).

The gene expression and cellular content of a non-cysteine protease, cathepsin D, in Alzheimer's disease brain showed evidence for early up-regulation of the endosomallysosomal system. Cataldo AM, et al., Neuron, 1995, 14 (3), 671-680).

*In vitro* studies on bone resorption have shown that cathepsins L and B may be involved in the remodelling of this tissue. These lysosomal cysteine proteases digest extracellular matrix proteins such as elastin, laminin, and type I collagen under acidic conditions. Osteoclast cells require this activity to degrade the organic matrix prior to bone regeneration accomplished by osteoblasts. Several natural and synthetic inhibitors of cysteine proteinases have been effective in inhibiting the degradation of this matrix.

The isolation of cathepsins and their role in bone resorption has been the subject of an intensive study. OC-2 has recently been isolated from pure osteoclasts from rabbit bones. The OC-2 was found to encode a possible cysteine proteinase structurally related to cathepsins L and S. Tezuka, K., et al., J. Biol. Chem., 269:1106-1109, (1994).

An inhibitor of cysteine proteinases and collagenase, Z-Phe-Ala-CHN₂ has been studied for its effect on the resorptive activity of isolated osteoclasts and has been found to inhibit resorption pits in dentine. Delaisse, J.M. et al., Bone, 8:305-313 (1987). Also, the effect of human recombinant cystatin C, a cysteine proteinase inhibitor, on bone resorption in vitro has been evaluated, and has been shown to significantly inhibit bone resorption which has been stimulated by parathyroid hormone. Lerner, U.H. and Grubb Anders, Journal of Bone and Mineral Research, 7:433-439, (1989). Further, a cDNA clone encoding the human cysteine protease cathepsin L has been recombinantly manufactured and expressed at high levels in *E. coli* in a T7 expression system. Recombinant human procathepsin L was successfully expressed at high levels and purified as both procathepsin L and active processed cathepsin L forms. Information about the possible function of the propeptide in cathepsin L folding and/or processing and about the necessity for the light chain of the enzyme for protease activity was obtained by expressing and purifying mutant enzymes carrying structural alterations in these regions. Smith, S.M. and Gottesman, M.M., J. Bio Chem., 264:20487-20495, (1989). There has also been reported the expression of a functional human cathepsin S in *Sacchromyces* *cerevisiae* and the characterization of the recombinant enzyme. Bromme, D. et al., J. Biol. Chem., 268:4832-4838 (1993).

### SUMMARY OF THE INVENTION

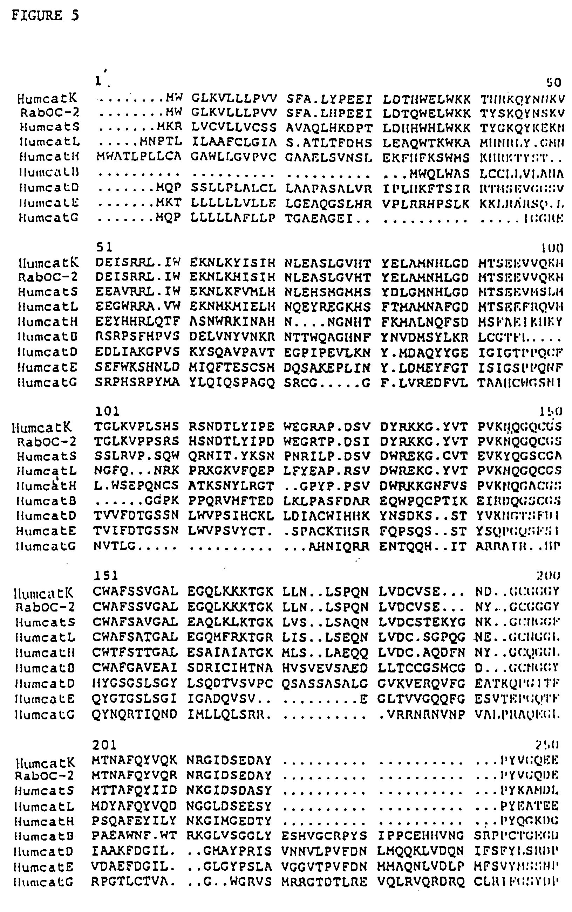

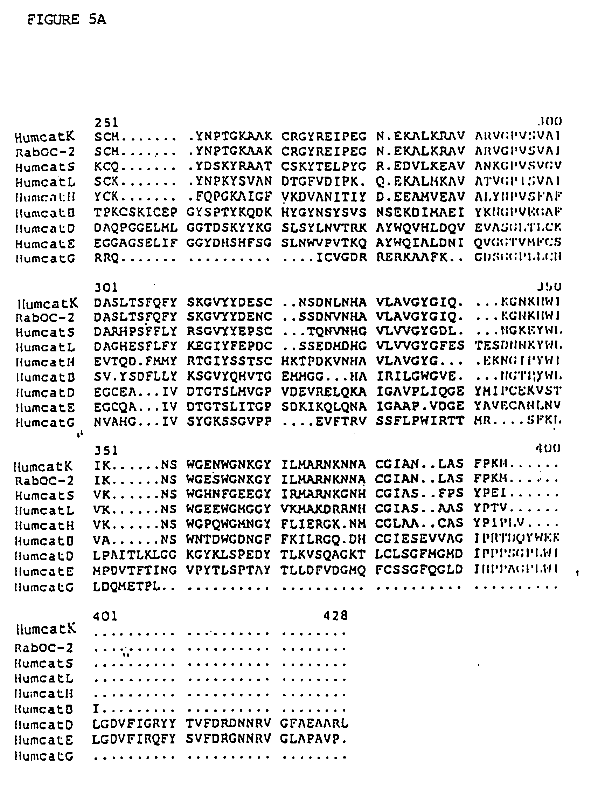

Toward these ends, and others, it is an object of the present invention to provide polypeptides, *inter alia*, that have been identified as novel cathepsin K by homology between the amino acid sequence set out in Figure 5 and known amino acid sequences of other proteins such as rabbit OC-2 and human cathepsin O cDNA. Tezuka, K., et al., J. Biol. Chem., 269:1106-1109, (1994).

It is a further object of the invention, moreover, to provide polynucleotides that encode cathepsin K, particularly polynucleotides that encode the polypeptide herein designated cathepsin K.

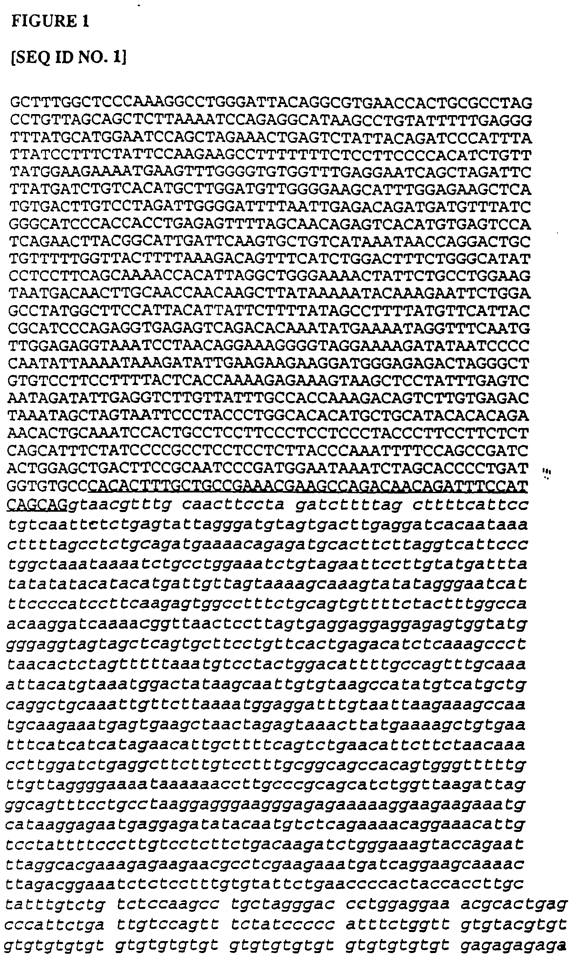

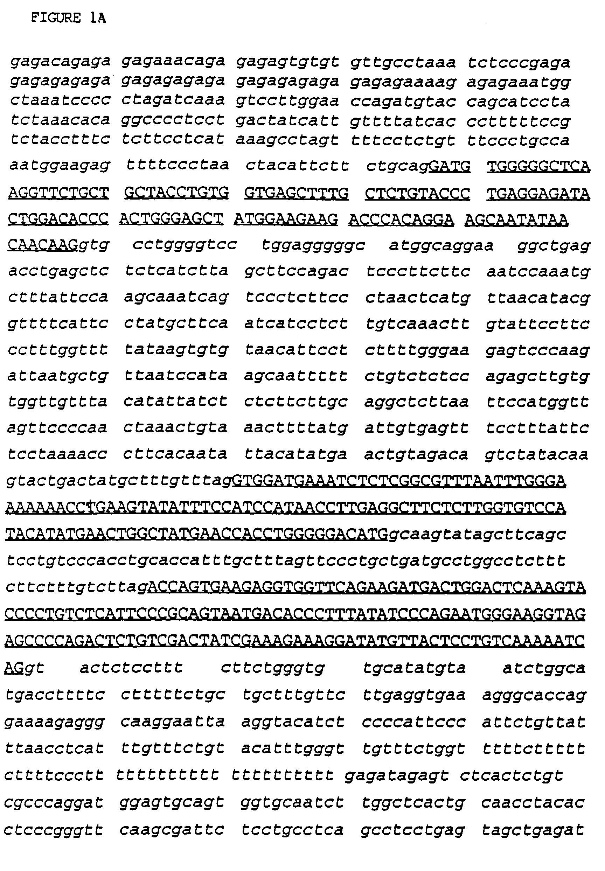

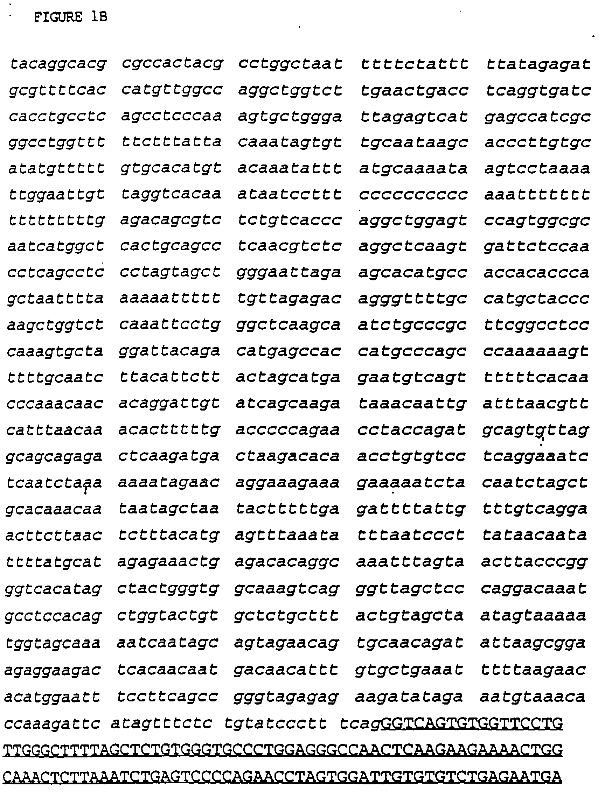

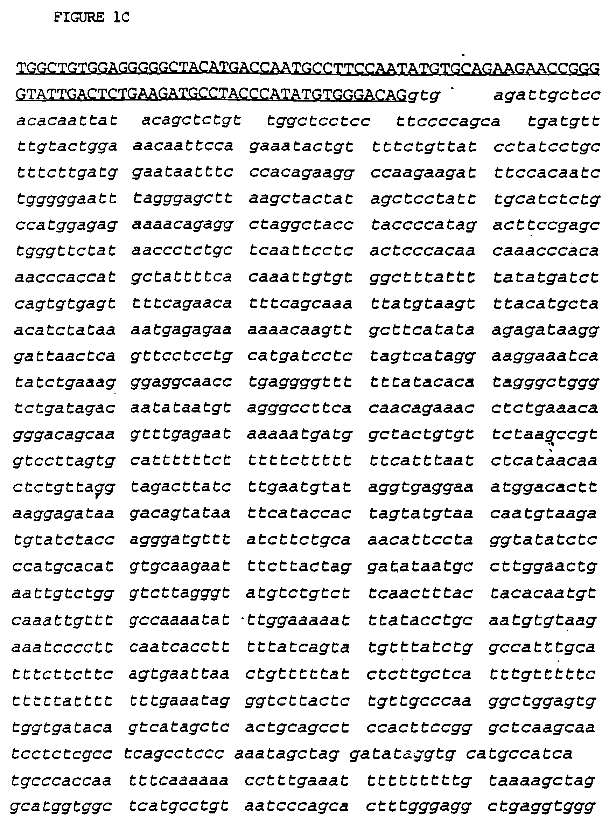

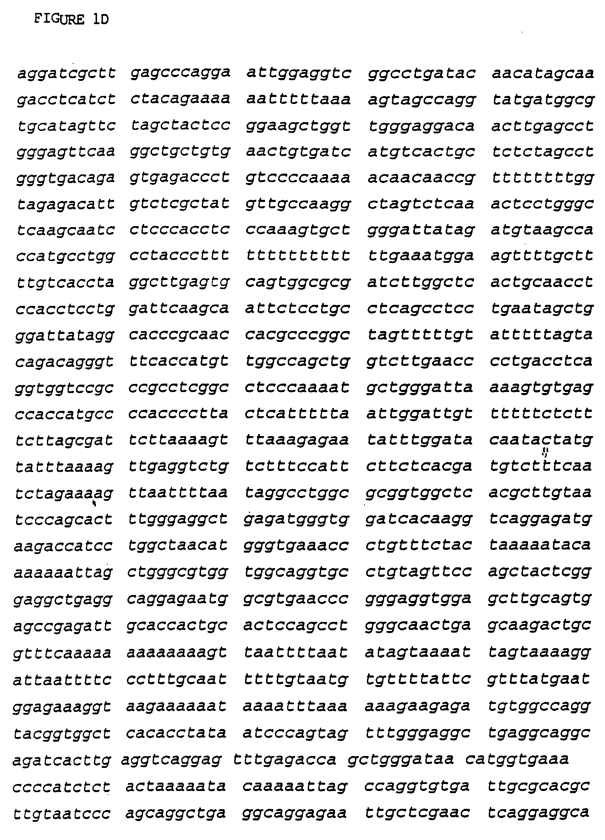

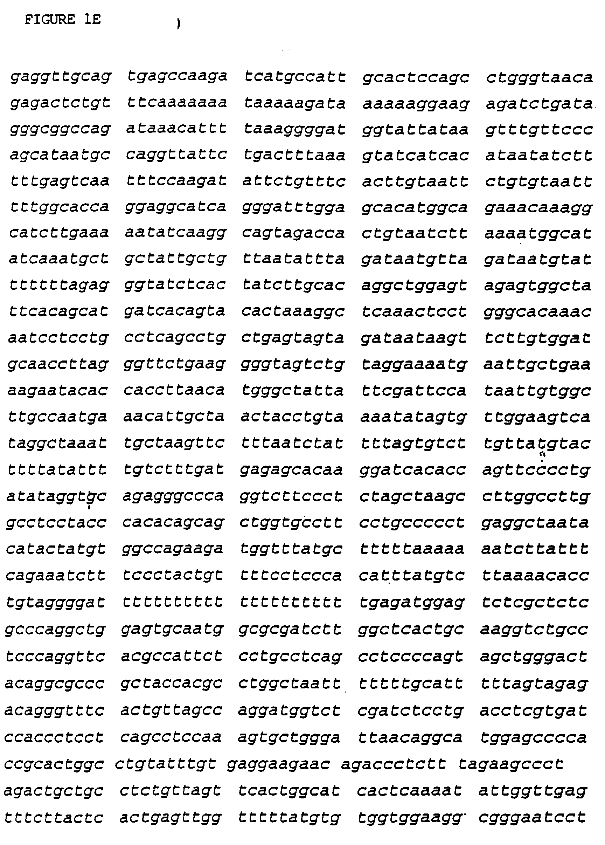

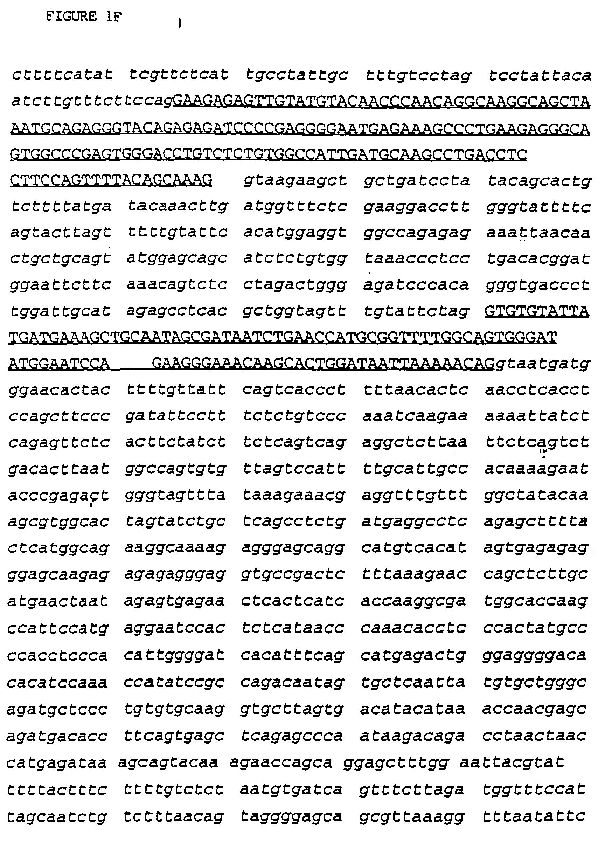

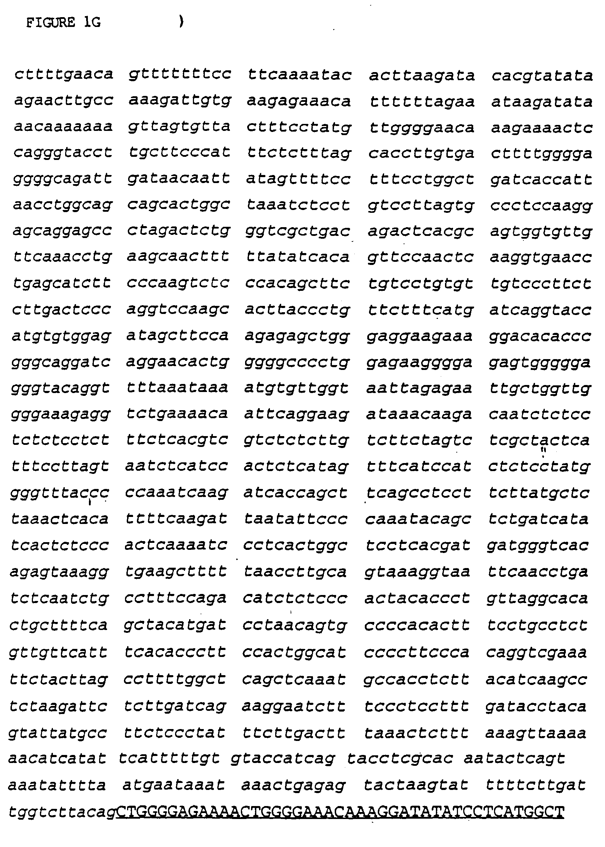

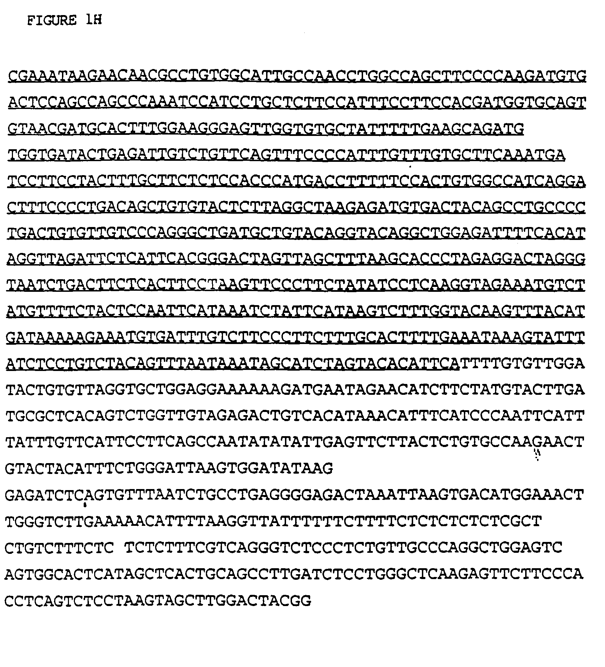

In a particularly preferred embodiment of this aspect of the invention the polynucleotide comprises the region encoding human cathepsin K in the sequence set out in Figure 1 [SEQ ID NO: 1] or in the genomic DNA (herein "gDNA") in ATCC deposit No. 98035 (referred to herein as the deposited clone).

In accordance with this aspect of the invention there are provided isolated nucleic acid molecules encoding human cathepsin K, including mRNAs, cDNAs, genomic DNAs and, in further embodiments of this aspect of the invention, biologically, diagnostically, clinically or therapeutically useful variants, analogs or derivatives thereof, or fragments thereof, including fragments of the variants, analogs and derivatives.

Among the particularly preferred embodiments of this aspect of the invention are naturally occurring allelic variants of human cathepsin K.

It also is an object of the invention to provide cathepsin K polypeptides, particularly human cathepsin K polypeptides, that cause or are associated with disease, for example, osteoporosis, Paget's disease, Gaucher's disease, CNS inflammation, Alzheimer's disease, hyporparathyroidism, bone degradation, metastatic tumors, rhemuatoid arthritis, osteoarthritis, peridontal disease and degradation of bone implants and bone protheses, particularly dental implants.

In accordance with this aspect of the invention there are provided novel polypeptides of human origin referred to herein as cathepsin K as well as biologically, diagnostically or therapeutically useful fragments, variants and derivatives thereof, variants and derivatives of the fragments, and analogs of the foregoing.

Among the particularly preferred embodiments of this aspect of the invention are variants of human cathepsin K encoded by naturally occurring alleles of the human cathepsin K gene.

It is another object of the invention to provide a process for producing the aforementioned polypeptides, polypeptide fragments, variants and derivatives, fragments of the variants and derivatives, and analogs of the foregoing.

In a preferred embodiment of this aspect of the invention there are provided methods for producing the aforementioned cathepsin K polypeptides comprising culturing host cells having expressibly incorporated therein an exogenously-derived human cathepsin K-encoding polynucleotide under conditions for expression of human cathepsin K in the host and then recovering the expressed polypeptide.

In accordance with yet another object of the invention there are methods to determine drug responsiveness of individuals having or suspected of having a defect in the cathepsin K gene.

In accordance with yet another object the invention there are provided products, compositions, processes and methods that utilize the aforementioned polypeptides and polynucleotides for research, biological, clinical and therapeutic purposes, *inter alia*.

In accordance with certain preferred embodiments of this aspect of the invention, there are provided products, compositions and methods, *inter alia*, for, among other things: assessing cathepsin K expression in cells by determining cathepsin K polypeptides of cathepsin K-encoding mRNA or hnRNA *in vitro*, *ex vivo* or *in vivo* by exposing cells to cathepsin K polypeptides, polynucleotides or antibodies as disclosed herein; assaying genetic variation and aberrations, such as defects, in cathepsin K polynucleotides, genes and gene control sequences; and administering a cathepsin K polypeptide or polynucleotide to an organism to augment cathepsin K function or remediate cathepsin K dysfunction.

In accordance with certain preferred embodiments of this and other aspects of the invention there are provided probes that hybridize specifically to human cathepsin K sequences.

In certain additional preferred embodiments of this aspect of the invention there are provided antibodies against cathepsin K polypeptides. In certain particularly preferred embodiments in this regard, the antibodies are highly selective for human cathepsin K.

In accordance with another aspect of the present invention, there are provided cathepsin K agonists. Among preferred agonists are molecules that mimic cathepsin K, that bind to cathepsin K-binding molecules or receptor molecules, and that elicit or augment cathepsin K-induced responses. Also among preferred agonists are molecules that interact with cathepsin K or cathepsin K polypeptides, or with other modulators of cathepsin K activities, and thereby potentiate or augment an effect of cathepsin K or more than one effect of cathepsin K.

In accordance with yet another aspect of the present invention, there are provided cathepsin K antagonists. Among preferred antagonists are those which mimic cathepsin K so as to bind to cathepsin K receptor or binding molecules but not elicit a cathepsin K-induced response or more than one cathepsin K-induced response. Also among preferred antagonists are molecules that bind to or interact with cathepsin K so as to inhibit an effect of cathepsin K or more than one effect of cathepsin K.

The agonists and antagonists may be used to mimic, augment or inhibit the action of cathepsin K polypeptides. They may be used, for instance, to treat osteoporosis, Paget's disease, Gaucher's disease, CNS inflammation, Alzheimer's disease, hyperparathyroidism, bone degradation, metastatic tumors, and degradation of bone implants and bone protheses, particularly dental implants. Such antagonists may be particularly useful to treat osteoporosis, Paget's disease, Gaucher's disease, Alzheimer's disease, hyperparathyroidism, bone degradation, metastatic tumors, CNS inflammation, rhemuatoid arthritis, osteoarthritis, periodontal disease and degradation of bone implants and bone protheses, particularly dental implants.

In a further aspect of the invention there are provided compositions comprising a cathepsin K polynucleotide or a cathepsin K polypeptide for administration to cells in vitro, to cells *ex vivo* and to cells *in vivo*, or to a multicellular organism. In certain particularly preferred embodiments of this aspect of the invention, the compositions comprise a cathepsin K polynucleotide for expression of a cathepsin K polypeptide in a host organism for treatment of disease. Particularly preferred in this regard is expression in a human patient for treatment of a dysfunction associated with aberrant endogenous activity of cathepsin K or to provide therapeutic.

Other objects, features, advantages and aspects of the present invention will become apparent to those of skill from the following description. It should be understood, however, that the following description and the specific examples, while indicating preferred embodiments of the invention, are given by way of illustration only. Various changes and modifications within the spirit and scope of the disclosed invention will become readily apparent to those skilled in the art from reading the following description and from reading the other parts of the present disclosure.

### BRIEF DESCRIPTION OF THE DRAWINGS

The following drawings depict certain embodiments of the invention. They are illustrative only and do not limit the invention otherwise disclosed herein.
Figure 1 shows the genomic nucleotide sequence of human cathepsin K. [SEQ ID NO: 1]. Exons are capitalized and underlined. Intron sequence is in lower case letters.
Figure 2 shows the nucleotide, exon-intron boundaries and deduced amino acid sequence of human cathepsin K.
Figure 3 (A - S) shows structural features of cathepsin K [SEQ ID NO; 2-19].
Figure 4 shows the intron-exon junctions.
Figure 5 shows the regions of similarity between amino acid sequences of cathepsin K, human cathepsins S, L, H, B, D, E, G and rabbit OC2 polypeptides.
Figure 6 shows the deduced amino acid sequence of human cathepsin K.

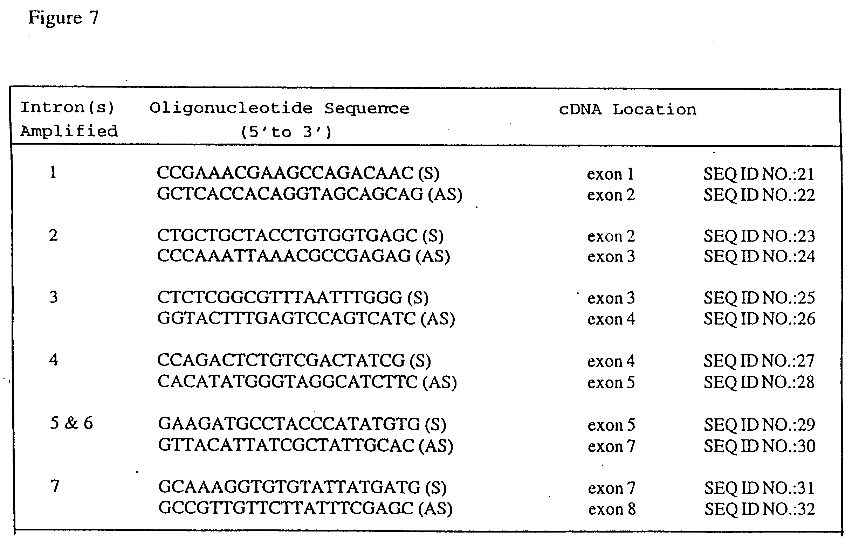

Figure 7 shows the PCR primers to amplify genomic DNA.

### GLOSSARY

The following illustrative explanations are provided to facilitate understanding of certain terms used frequently herein, particularly in the examples. The explanations are provided as a convenience and are not limitative of the invention.

DIGESTION of DNA refers to catalytic cleavage of the DNA with a restriction enzyme that acts only at certain sequences in the DNA. The various restriction enzymes referred to herein are commercially available and their reaction conditions, cofactors and other requirements for use are known and routine to the skilled artisan.

For analytical purposes, typically, 1 µg of plasmid or DNA fragment is digested with about 2 units of enzyme in about 20 ml of reaction buffer. For the purpose of isolating DNA fragments for plasmid construction, typically 5 to 50 µg of DNA are digested with 20 to 250 units of enzyme in proportionately larger volumes.

Appropriate buffers and substrate amounts for particular restriction enzymes are described in standard laboratory manuals, such as those referenced below, and they are specified by commercial suppliers.

Incubation times of about 1 hour at 37°C are ordinarily used, but conditions may vary in accordance with standard procedures, the supplier's instructions and the particulars of the reaction. After digestion, reactions may be analyzed, and fragments may be purified by electrophoresis through an agarose or polyacrylamide gel, using well known methods that are routine for those skilled in the art.

GENETIC ELEMENT generally means a polynucleotide comprising a region that encodes a polypeptide or a region that regulates transcription or translation or other processes important to expression of the polypeptide in a host cell, or a polynucleotide comprising both a region that encodes a polypeptide and a region operably linked thereto that regulates expression.

Genetic elements may be comprised within a vector that replicates as an episomal element; that is, as a molecule physically independent of the host cell genome. They may be comprised within mini-chromosomes, such as those that arise during amplification of transfected DNA by methotrexate selection in eukaryotic cells. Genetic elements also may be comprised within a host cell genome; not in their natural state but, rather, following manipulation such as isolation, cloning and introduction into a host cell in the form of purified DNA or in a vector, among others.

IDENTITY means the degree of sequence relatedness between two polypeptide or two polynucleotides sequences as determined by the identity of the match between two strings of such sequences. Identity can be readily calculated. While there exist a number of methods to measure identity between two polynucleotide or polypeptide sequences, the term "identity" is well known to skilled artisans (*Computational Molecular Biology*, Lesk, A.M., ed., Oxford University Press, New York, 1988; *Biocomputing: Informatics and Genome Projects*, Smith, D.W., ed., Academic Press, New York, 1993; *Computer Analysis of Sequence Data*, Part I, Griffin, A.M., and Griffin, H.G., eds., Humana Press, New Jersey, 1994; *Sequence Analysis in Molecular Biology*, von Heinje, G., Academic Press, 1987; and *Sequence Analysis Primer*, Gribskov, M. and Devereux, J., eds., M Stockton Press, New York, 1991). Methods commonly employed to determine identity between two sequences include, but are not limited to disclosed in Guide to Huge Computers, Martin J. Bishop, ed., Academic Press, San Diego, 1994, and Carillo, H., and Lipman, D., SIAM J. Applied Math., 48: 1073 (1988). Preferred methods to determine identity are designed to give the largest match between the two sequences tested. Such methods are codified in computer programs. Preferred computer program methods to determine identity between two sequences include, but are not limited to, GCG program package (Devereux, J., et al., *Nucleic Acids Research 12(1):* 387(1984)), BLASTP, BLASTN, FASTA (Atschul, S.F. et al., *J. Molec. Biol. 215:* 403 (1990)).

ISOLATED means altered "by the hand of man" from its natural state; i.e., that, if it occurs in nature, it has been changed or removed from its original environment, or both.

For example, a naturally occurring polynucleotide or a polypeptide naturally present in a living animal in its natural state is not "isolated," but the same polynucleotide or polypeptide separated from some or all of the coexisting materials of its natural is "isolated", as the term is employed herein.

As part of or following isolation, such polynucleotides can be joined to other polynucleotides, such as DNAs, for mutagenesis, to form fusion proteins, and for propagation or expression in a host, for instance. The isolated polynucleotides, alone or joined to other polynucleotides such as vectors, can be introduced into host cells, in culture or in whole organisms. Introduced into host cells in culture or in whole organisms, such DNAs still would be isolated, as the term is used herein, because they would not be in their naturally occurring form or environment. Similarly, the polynucleotides and polypeptides may occur in a composition, such as a media formulations, solutions for introduction of polynucleotides or polypeptides, for example, into cells, compositions or solutions for chemical or enzymatic reactions, for instance, which are not naturally occurring compositions, and, therein remain isolated polynucleotides or polypeptides within the meaning of that term as it is employed herein.

LIGATION refers to the process of forming phosphodiester bonds between two or more polynucleotides, which most often are double stranded DNAs. Techniques for ligation are well known to the art and protocols for ligation are described in standard laboratory manuals and references, such as, for instance, Sambrook et al., MOLECULAR CLONING, A LABORATORY MANUAL, 2nd Ed.; Cold Spring Harbor Laboratory Press, Cold Spring Harbor, New York (1989) and Maniatis et al., pg. 146, as cited below.

OLIGONUCLEOTIDE(S) refers to relatively short polynucleotides. Often the term refers to single-stranded deoxyribonucleotides, but it can refer as well to single-, double-, or triple-stranded ribonucleotides, antisense polynucleotides, RNA:DNA hybrids and double-stranded DNAs, among others.

Oligonucleotides, such as single-stranded DNA probe oligonucleotides, often are synthesized by chemical methods, such as those implemented on automated oligonucleotide synthesizers. However, oligonucleotides can be made by a variety of other methods, including in vitro recombinant DNA-mediated techniques and by expression of DNAs in cells and organisms.

Initially, chemically synthesized DNAs typically are obtained without a 5' phosphate. The 5' ends of such oligonucleotides are not substrates for phosphodiester bond formation by ligation reactions that employ DNA ligases typically used to form recombinant DNA molecules. Where ligation of such oligonucleotides is desired, a phosphate can be added by standard techniques, such as those that employ a kinase and ATP.

The 3' end of a chemically synthesized oligonucleotide generally has a free hydroxyl group and, in the presence of a ligase, such as T4 DNA ligase, readily will form a phosphodiester bond with a 5' phosphate of another polynucleotide, such as another oligonucleotide. As is well known, this reaction can be prevented selectively, where desired, by removing the 5' phosphates of the other polynucleotide(s) prior to ligation.

PLASMIDS generally are designated herein by a lower case letter *p* preceded and/or followed by capital letters and/or numbers, in accordance with standard naming conventions that are familiar to those of skill in the art. Starting plasmids disclosed herein are either commercially available, publicly available on an unrestricted basis, or can be constructed from available plasmids by routine application of well known, published procedures. Many plasmids and other cloning and expression vectors that can be used in accordance with the present invention are well known and readily available to those of skill in the art. Moreover, those of skill readily may construct any number of other plasmids suitable for use in the invention. The properties, construction and use of such plasmids, as well as other vectors, in the present invention will be readily apparent to those of skill from the present disclosure.

POLYNUCLEOTIDE(S) generally refers to any polyribonucleotide or polydeoxribonucleotide, which may be unmodified RNA or DNA or modified RNA or DNA. Thus, for instance, polynucleotides as used herein refers to, among others, single-and double-stranded DNA, DNA that is a mixture of single-and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions.

In addition, polynucleotide as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide.

As used herein, the term polynucleotide includes DNAs or RNAs as described above that contain one or more modified bases. Thus, DNAs or RNAs with backbones modified for stability or for other reasons are "polynucleotides" as that term is intended herein. Moreover, DNAs or RNAs comprising unusual bases, such as inosine, or modified bases, such as tritylated bases, to name just two examples, are polynucleotides as the term is used herein.

It will be appreciated that a great variety of modifications have been made to DNA and RNA that serve many useful purposes known to those of skill in the art. The term polynucleotide as it is employed herein embraces such chemically, enzymatically or metabolically modified forms of polynucleotides, as well as the chemical forms of DNA and RNA characteristic of viruses and cells, including simple and complex cells, *inter alia*.

POLYPEPTIDES, as used herein, includes all polypeptides as described below. The basic structure of polypeptides is well known and has been described in innumerable textbooks and other publications in the art. In this context, the term is used herein to refer to any peptide or protein comprising two or more amino acids joined to each other in a linear chain by peptide bonds. As used herein, the term refers to both short chains, which also commonly are referred to in the art as peptides, oligopeptides and oligomers, for example, and to longer chains, which generally are referred to in the art as proteins, of which there are many types.

It will be appreciated that polypeptides often contain amino acids other than the 20 amino acids commonly referred to as the 20 naturally occurring amino acids, and that many amino acids, including the terminal amino acids, may be modified in a given polypeptide, either by natural processes, such as processing and other post-translational modifications, but also by chemical modification techniques which are well known to the art. Even the common modifications that occur naturally in polypeptides are too numerous to list exhaustively here, but they are well described in basic texts and in more detailed monographs, as well as in a voluminous research literature, and they are well known to those of skill in the art. Among the known modifications which may be present in polypeptides of the present are, to name an illustrative few, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination.

Such modifications are well known to those of skill and have been described in great detail in the scientific literature. Several particularly common modifications, glycosylation, lipid attachment, sulfation, gamma-carboxylation of glutamic acid residues, hydroxylation and ADP-ribosylation, for instance, are described in most basic texts, such as, for instance PROTEINS - STRUCTURE AND MOLECULAR PROPERTIES, 2nd Ed., T. E. Creighton, W. H. Freeman and Company, New York (1993). Many detailed reviews are available on this subject, such as, for example, those provided by Wold, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York (1983); Seifter et al., Analysis for protein modifications and nonprotein cofactors, Meth. Enzymol. 182: 626-646 (1990) and Rattan et al., Protein Synthesis: Posttranslational Modifications and Aging, Ann. N.Y. Acad. Sci. 663: 48-62 (1992).

It will be appreciated, as is well known and as noted above, that polypeptides are not always entirely linear. For instance, polypeptides may be branched as a result of ubiquitination, and they may be circular, with or without branching, generally as a result of posttranslation events, including natural processing event and events brought about by human manipulation which do not occur naturally. Circular, branched and branched circular polypeptides may be synthesized by non-translation natural process and by entirely synthetic methods, as well.

Modifications can occur anywhere in a polypeptide, including the peptide backbone, the amino acid side-chains and the amino or carboxyl termini. In fact, blockage of the amino or carboxyl group in a polypeptide, or both, by a covalent modification, is common in naturally occurring and synthetic polypeptides and such modifications may be present in polypeptides of the present invention, as well. For instance, the amino terminal residue of polypeptides made in *E. coli*, prior to proteolytic processing, almost invariably will be N-formylmethionine.

The modifications that occur in a polypeptide often will be a function of how it is made. For polypeptides made by expressing a cloned gene in a host, for instance, the nature and extent of the modifications in large part will be determined by the host cell postranslational modification capacity and the modification signals present in the polypeptide amino acid sequence. For instance, as is well known, glycosylation often does not occur in bacterial hosts such as *E. coli*. Accordingly, when glycosylation is desired, a polypeptide should be expressed in a glycosylating host, generally a eukaryotic cell. Insect cells often carry out the same posttranslational glycosylations as mammalian cells and, for this reason, insect cell expression systems have been developed to express efficiently mammalian proteins having native patterns of glycosylation, *inter alia*. Similar considerations apply to other modifications.

It will be appreciated that the same type of modification may be present in the same or varying degree at several sites in a given polypeptide. Also, a given polypeptide may contain many types of modifications.

In general, as used herein, the term polypeptide encompasses all such modifications, particularly those that are present in polypeptides synthesized by expressing a polynucleotide in a host cell.

VARIANT(S) of polynucleotides or polypeptides, as the term is used herein, are polynucleotides or polypeptides that differ from a reference polynucleotide or polypeptide, respectively. Variants in this sense are described below and elsewhere in the present disclosure in greater detail.
(1) A polynucleotide that differs in nucleotide sequence from another, reference polynucleotide. Generally, differences are limited so that the nucleotide sequences of the reference and the variant are closely similar overall and, in many regions, identical.
   As noted below, changes in the nucleotide sequence of the variant may be silent. That is, they may not alter the amino acids encoded by the polynucleotide. Where alterations are limited to silent changes of this type a variant will encode a polypeptide with the same amino acid sequence as the reference. Also as noted below, changes in the nucleotide sequence of the variant may alter the amino acid sequence of a polypeptide encoded by the reference polynucleotide. Such nucleotide changes may result in amino acid substitutions, additions, deletions, fusions and truncations in the polypeptide encoded by the reference sequence, as discussed below.
(2) A polypeptide that differs in amino acid sequence from another, reference polypeptide. Generally, differences are limited so that the sequences of the reference and the variant are closely similar overall and, in many region, identical.

A variant and reference polypeptide may differ in amino acid sequence by one or more substitutions, additions, deletions, fusions and truncations, which may be present in any combination.

RECEPTOR MOLECULE, as used herein, refers to molecules which bind or interact specifically with cathepsin K polypeptides of the present invention, including not only classic receptors and enzymatic substrates, both of which are preferred, but also other molecules that specifically bind to or interact with polypeptides of the invention (which also may be referred to as "binding molecules" and "interaction molecules," respectively and as "cathepsin K binding molecules" and "cathepsin K interaction molecules." These cathepsin K binding molecules also include, for example, cathepsin K substrate analogs. Binding between polypeptides of the invention and such molecules, including receptor or binding or interaction molecules may be exclusive to polypeptides of the invention, which is very highly preferred, or it may be highly specific for polypeptides of the invention, which is highly preferred, or it may be highly specific to a group of proteins that includes polypeptides of the invention, which is preferred, or it may be specific to several groups of proteins at least one of which includes polypeptides of the invention.

Receptors also may be non-naturally occurring, such as antibodies and antibody-derived reagents that bind specifically to polypeptides of the invention.

### DESCRIPTION OF THE INVENTION

The present invention relates to novel cathepsin K polypeptides and polynucleotides, among other things, as described in greater detail below. In particular, the invention relates to polypeptides and polynucleotides of a novel human cathepsin K, which is related by amino acid sequence homology to rabbit OC-2 and human cathepsin O cDNA. Tezuka, K., et al., J. Biol. Chem., 269:1106-1109, (1994). The invention relates especially to cathepsin K having the nucleotide sequences set out in Figure 1 [SEQ ID NO: 1], and to the cathepsin K nucleotide sequences of the gDNA in ATCC Deposit No. 98035, which is herein referred to as "the deposited clone" or as the "gDNA of the deposited clone." It will be appreciated that the nucleotide sequences set out in Figure 1 [SEQ ID NO: 1] were obtained by sequencing the gDNA of the deposited clone, as more specifically set forth elsewhere herein. Hence, the sequence of the deposited clone is controlling as to any discrepancies between the two.

### Polynucleotides

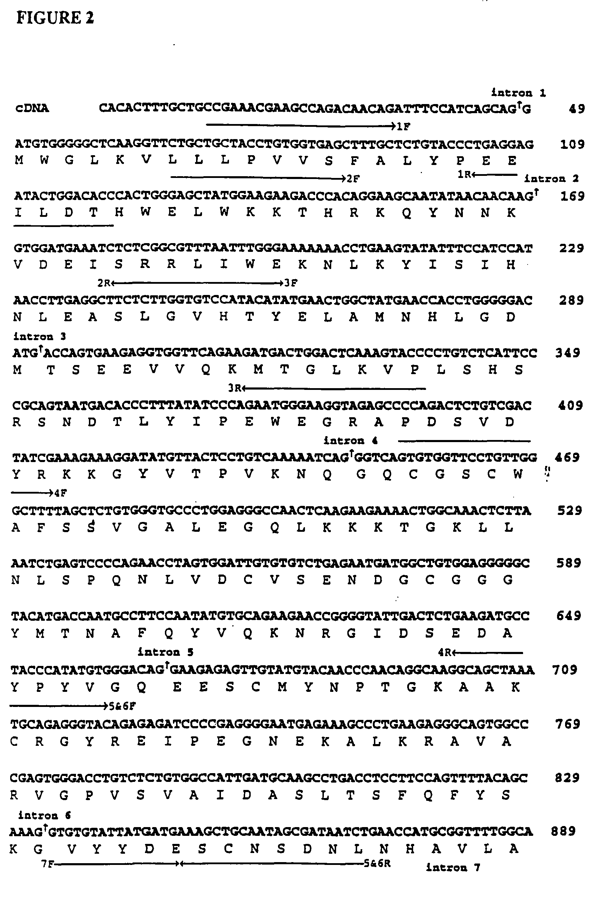

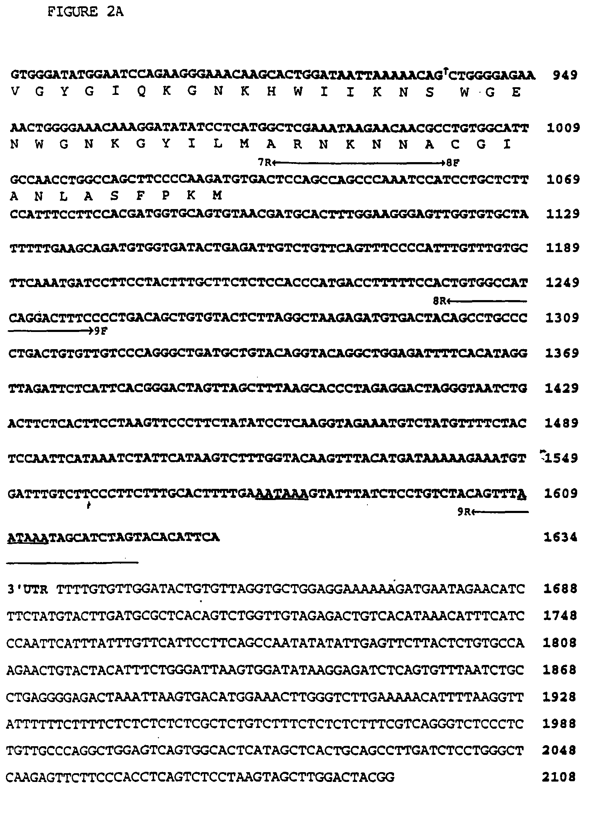

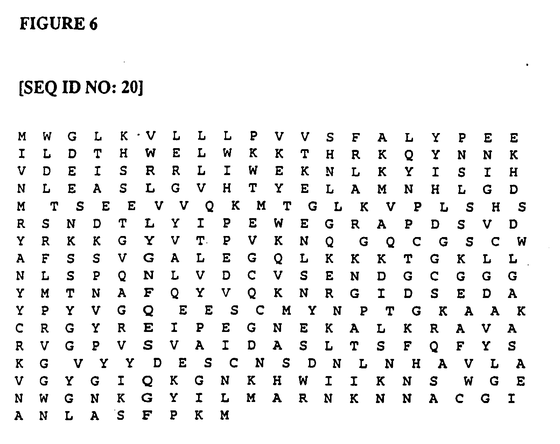

In accordance with one aspect of the present invention, there are provided isolated polynucleotides which encode the cathepsin K polypeptide having the deduced amino acid sequence of Figure 2 [SEQ ID NO:20] (see also Figure 6 for the deduced amino acid sequence) or the cathepsin K polypeptide encoded by the gDNA in the deposited clone.

Using the information provided herein, such as the polynucleotide sequence set out in Figure 1 [SEQ ID NO: 1], a polynucleotide of the present invention encoding human cathepsin K polypeptide may be obtained using standard cloning and screening procedures, such as those for cloning gDNAs using DNA from cells of a human as starting material. Illustrative of the invention, the polynucleotide set out in Figure 1 [SEQ ID NO: 1] was discovered in a human gDNA library as described in Example 1.

Human cathepsin K of the invention is structurally related to other proteins of the cathepsin family, as shown by the results of sequencing the gDNA encoding human cathepsin K in the deposited clone. The gDNA sequence thus obtained is set out in Figure 1 [SEQ ID NO: 1]. It contains a non-contiguous open reading frame encoding, after intron removal, but including all exons, a protein of about 329 amino acid residues.

Polynucleotides of the present invention may be in the form of RNA, such as mRNA or hnRNA, or in the form of DNA, including, for instance, cDNA and gDNA obtained by cloning or produced by chemical synthetic techniques or by a combination thereof. The DNA may be triple-stranded, double-stranded or single-stranded. Single-stranded DNA may be the coding strand, also known as the sense strand, or it may be the non-coding strand, also referred to as the anti-sense strand.

The coding sequence which encodes the polypeptide may be identical to the exon sequence of the polynucleotide shown in Figure 1 [SEQ ID NO: 1] or that of the deposited clone. It also may be a polynucleotide with a different sequence, which, as a result of the redundancy (degeneracy) of the genetic code, encodes the polypeptide of the DNA of Figure 2 [SEQ ID NO:20] or of the deposited gDNA, including, but not limited to, splice variants transcribed from such gDNA.

Polynucleotides of the present invention which encode the polypeptide of Figure 1 [SEQ ID NO: 1] or the polypeptide encoded by the deposited gDNA may include, but are not limited to the coding sequence for the mature polypeptide, by itself; the coding sequence for the mature polypeptide and additional coding sequences, such as those encoding a leader or secretory sequence, such as a pre-, or pro- or prepro- protein sequence; the coding sequence of the mature polypeptide, with or without the aforementioned additional coding sequences, together with additional, non-coding sequences, including for example, but not limited to introns and non-coding 5' and 3' sequences, such as the transcribed, non-translated sequences that play a role in transcription, mRNA processing - including splicing and polyadenylation signals, for example - ribosome binding and stability of mRNA; additional coding sequence which codes for additional amino acids, such as those which provide additional functionalities. Thus, for instance, the polypeptide may be fused to a marker sequence, such as a peptide, which facilitates purification of the fused polypeptide. In certain preferred embodiments of this aspect of the invention, the marker sequence is a hexa-histidine peptide, such as the tag provided in the vector pQE-9, among others, many of which are commercially available. As described in Gentz et al., Proc. Natl. Acad. Sci., USA 86: 821-824 (1989), for instance, hexa-histidine provides for convenient purification of the fusion protein. The HA tag corresponds to an epitope derived of influenza hemagglutinin protein, which has been described by Wilson et al., Cell 37: 767 (1984), for instance.

In accordance with the foregoing, the term "polynucleotide encoding a polypeptide" as used herein encompasses polynucleotides which include a sequence encoding a polypeptide of the present invention, particularly the human cathepsin K having the amino acid sequence set out in Figure 2 [SEQ ID NO:20] or the amino acid sequence of the human cathepsin K encoded by the gDNA of the deposited clone. The term encompasses polynucleotides that include a single continuous region or discontinuous regions encoding the polypeptide (for example, interrupted by introns) together with additional regions, that also may contain coding and/or non-coding sequences.

The present invention further relates to variants of the herein above described polynucleotides which encode for fragments, analogs and derivatives of the polypeptide having the deduced amino acid sequence of Figure 2 [SEQ ID NO:20] or the polypeptide encoded by the exons of the gDNA of the deposited clone, including, but not limited to, splice variants transcribed from such gDNA. A variant of the polynucleotide may be a naturally occurring variant such as a naturally occurring allelic variant or splice variant, or it may be a variant that is not known to occur naturally. Such non-naturally occurring variants of the polynucleotide may be made by mutagenesis techniques, including those applied to polynucleotides, cells or organisms. Such non-naturally occurring variants of the polynucleotide may be made by modifying splice acceptor, donor and/or branch sites, or by expressing the gDNA in cells where it is not naturally expressed, or cell extracts made from such cells.

Among variants in this regard are variants that differ from the aforementioned polynucleotides by nucleotide substitutions, deletions or additions. The substitutions, deletions or additions may involve one or more nucleotides. The variants may be altered in coding or non-coding regions or both. Alterations in the coding regions may produce conservative or non-conservative amino acid substitutions, deletions or additions.

Among the particularly preferred embodiments of the invention in this regard are polynucleotide sequence of cathepsin K set out in Figure 1 [SEQ ID NO: 1] or the polynucleotide sequence of cathepsin K of the gDNA of the deposited clone; variants, analogs, derivatives and fragments thereof, and fragments of the variants, analogs and derivatives.

Further particularly preferred in this regard are polynucleotides encoding cathepsin K variants, analogs, derivatives and fragments, and variants, analogs and derivatives of the fragments, which have the amino acid sequence of the cathepsin K polypeptide of Figure 2 [SEQ ID NO:20] or of the deposit in which several, a few, 5 to 10, 1 to 5, 1 to 3, 2, 1 or no amino acid residues are substituted, deleted or added, in any combination. Especially preferred among these are silent substitutions, additions and deletions, which do not alter the properties and activities of the cathepsin K. Also especially preferred in this regard are conservative substitutions. Most highly preferred are polypeptides having the amino acid sequence of Figure 2 [SEQ ID NO:20] or of the deposit, without substitutions.

Further preferred embodiments of the invention are polynucleotides that are at least 70% identical to a polynucleotide encoding the cathepsin K polypeptide having the amino acid sequence set out in Figure 2 [SEQ ID NO:20], or variants, close homologs, derivatives and analogs thereof, as described above, and polynucleotides which are complementary to such polynucleotides. Alternatively, most highly preferred are polynucleotides that comprise a region that is at least 80% identical to a polynucleotide encoding the cathepsin K polypeptide of the gDNA of the deposited clone and polynucleotides complementary thereto. In this regard, polynucleotides at least 90% identical to the same are particularly preferred, and among these particularly preferred polynucleotides, those with at least 95% are especially preferred. Furthermore, those with at least 97% are highly preferred among those with at least 95%, and among these those with at least 98% and at least 99% are particularly highly preferred, with at least 99% being the more preferred.

Still further preferred embodiments of the invention are polynucleotides comprising cathepsin K intron polynucleotide sequences, particularly polynucleotides comprising intron 1[SEQ ID NO: 4], 2 [SEQ ID NO: 6], 3 [SEQ ID NO: 8], 4 [SEQ ID NO: 10], 5 [SEQ ID NO: 12], 6 [SEQ ID NO: 14] or 7[SEQ ID NO: 16], having the intron polynucleotide sequence set out in Figures 1 [SEQ ID NO: 1] and 3 [SEQ ID NO: 2-19], or variants, close homologs, derivatives and analogs thereof, as described above, and polynucleotides which are complementary to such polynucleotides. Other preferred embodiments of the invention are polynucleotides comprising cathepsin K intron 1[SEQ ID NO: 4], 2 [SEQ ID NO: 6], 3 [SEQ ID NO: 8], 4 [SEQ ID NO: 10], 5 [SEQ ID NO: 12], 6 [SEQ ID NO: 14] or 7[SEQ ID NO: 16], operatively linked to the exon of a gene other than cathepsin K, or joining a cathepsin K exon and an exon of another gene.

Still other preferred embodiments of the invention are polynucleotides comprising cathepsin K exon polynucleotide sequences, particularly polynucleotides comprising exon 1 [SEQ ID NO: 3], 2 [SEQ ID NO: 5], 3 [SEQ ID NO: 7], 4 [SEQ ID NO: 9], 5 [SEQ ID NO: 11], 6 [SEQ ID NO: 13], 7 [SEQ ID NO: 15] or 8 [SEQ ID NO: 17], having the exon polynucleotide sequence set out in Figures 1 [SEQ ID NO: 1] and 3 [SEQ ID NO: 2-19], or variants, close homologs, derivatives and analogs thereof, as described above, and polynucleotides which are complementary to such polynucleotides. Other preferred embodiments of the invention are polynucleotides comprising cathepsin K exon 1 [SEQ ID NO: 3], 2 [SEQ ID NO: 5], 3 [SEQ ID NO: 7], 4 [SEQ ID NO: 9], 5 [SEQ in NO: 11], 6 [SEQ ID NO: 13], 7 [SEQ ID NO: 15] or 8 [SEQ ID NO: 17], operatively linked to the intron of a gene other than cathepsin K.

More preferred embodiments of the invention are differentially spliced polynucleotides, particularly those comprising any one or more of the following exon-exon pairs: 1-3, 1-4, 1-5, 1-6, 1-7, 1-8, 2-4, 2-5, 2-6, 2-7, 2-8, 3-4, 3-5, 3-6, 3-7, 3-8, 4-5, 4-6, 4-7, 4-8, 5-7, 5-8, or 6-8. Particularly preferred embodiments of the invention are differentially spliced polynucleotides which encode polypeptides which function in cells, especially those which have a biological activity of cathepsin K, most especially those expressed in human cells.

Polynucleotides comprising exon-exon pairs may be a naturally occurring variant such as a naturally occurring splice variant, or it may be a variant that is not known to occur naturally. Such non-naturally occurring variants of the polynucleotide may be made by mutagenesis techniques, including those applied to polynucleotides, cells or organisms. Such non-naturally occurring variants of the polynucleotide may be made by modifying splice acceptor, donor and/or branch sites, or by expressing the gDNA in cells where it is not naturally expressed, or cell extracts made from such cells. Exon-exon pairs can be full, fused exons or can be fused fragments of exons with a splice junction present. Preferred exon-exon pairs comprising exon fragments may be made from at least two exons, one of which comprises an operable splice donor site and the other of which comprises an operable splice acceptor site and which both are operatively linked by an intron.

Particularly preferred embodiments in this respect, moreover, are polynucleotides which encode polypeptides which retain substantially the same biological function or activity as the mature polypeptide encoded by the cDNA of Figure 2 [SEQ ID NO:20] or the gDNA of the deposited clone.

The present invention further relates to polynucleotides that hybridize to the herein above-described sequences. In this regard, the present invention especially relates to polynucleotides which hybridize under stringent conditions to the herein above-described polynucleotides. As herein used, the term "stringent conditions" means hybridization will occur only if there is at least 95% and preferably at least 97% identity between the sequences.

As discussed additionally herein regarding polynucleotide assays of the invention, for instance, polynucleotides of the invention as discussed above, may be used as a hybridization probe for cDNA and genomic DNA to isolate full-length cDNAs and genomic clones encoding cathepsin K and to isolate cDNA and genomic clones of other genes that have a high sequence similarity to the human cathepsin K gene. Such probes generally will comprise at least 15 bases. Preferably, such probes will have at least 30 bases and may have at least 50 bases. Particularly preferred probes will have at least 30 bases and will have 50 bases or less.

For example, the coding region of the cathepsin K gene may be isolated by screening using the known DNA sequence to synthesize an oligonucleotide probe. A labeled oligonucleotide having a sequence complementary to that of a gene of the present invention is then used to screen a library of human cDNA, genomic DNA or mRNA to determine which members of the library the probe hybridizes to.

The polynucleotides and polypeptides of the present invention may be employed as research reagents and materials for discovery of treatments and diagnostics to human disease, as further discussed herein relating to polynucleotide assays, *inter alia*.

The polynucleotides may encode a polypeptide which is the mature protein plus additional amino or carboxyl-terminal amino acids, or amino acids interior to the mature polypeptide (when the mature form has more than one polypeptide chain, for instance). Such sequences may play a role in processing of a protein from precursor to a mature form, may facilitate protein trafficking, may prolong or shorten protein half-life or may facilitate manipulation of a protein for assay or production, among other things. As generally is the case *in situ*, the additional amino acids may be processed away from the mature protein by cellular enzymes.

A precursor protein, having the mature form of the polypeptide fused to one or more prosequences may be an inactive form of the polypeptide. When prosequences are removed such inactive precursors generally are activated. Some or all of the prosequences may be removed before activation. Generally, such precursors are called proproteins.

In sum, a polynucleotide of the present invention may encode a mature protein, a mature protein plus a leader sequence (which may be referred to as a preprotein), a precursor of a mature protein having one or more prosequences which are not the leader sequences of a preprotein, or a preproprotein, which is a precursor to a proprotein, having a leader sequence and one or more prosequences, which generally are removed during processing steps that produce active and mature forms of the polypeptide.

### Deposited materials

A deposit containing a human cathepsin K gDNA has been deposited with the American Type Culture Collection, as noted above. Also as noted above, the gDNA deposit is referred to herein as "the deposited clone" or as "the gDNA of the deposited clone."

The deposited clone was deposited with the American Type Culture Collection, 12301 Park Lawn Drive, Rockville, Maryland 20852, USA, on April 26, 1996, and assigned ATCC Deposit No. 98035.

The deposited material is a P1 cosmid that contains the full length cathepsin K gDNA, referred to as "P1SacB2CatK/P129" upon deposit.

The deposit has been made under the terms of the Budapest Treaty on the international recognition of the deposit of micro-organisms for purposes of patent procedure. The strain will be irrevocably and without restriction or condition released to the public upon the issuance of a patent. The deposit is provided merely as convenience to those of skill in the art and is not an admission that a deposit is required for enablement, such as that required under 35 U.S.C. section 112.

The sequence of the polynucleotides contained in the deposited material, as well as the amino acid sequence of the polypeptide encoded thereby, are controlling in the event of any conflict with any description of sequences herein.

A license may be required to make, use or sell the deposited materials, and no such license is hereby granted.

### Polypeptides

The present invention further relates to a human cathepsin K polypeptide which has the deduced amino acid sequence of Figure 2 [SEQ ID NO:20], which is encoded by an unspliced or differentially spliced hnRNA or mRNA transcribed from the sequence of Figure 1 [SEQ ID NO: 1], or which has the amino acid sequence encoded by the deposited clone. Also provided are polypetides encoded by the cathepsin K gDNA comprising missense or nonsense mutations, or those polypeptides encoded by unspliced or partially spliced hnRNAs which still comprise at least one intron, particularly those polypeptides which are naturally found in cells, especially human cells. Frameshift mutations have been shown to be associated with disease (Hol, FA, et al. Journal of Medical Genetics, 1995, 32 (1), 52-56).

Preferred polypeptides provided by the invention are encoded by differentially spliced polynucleotides, particularly those polypeptides encoded by polynucleotides comprising any one or more of the following exon-exon pairs: 1-3, 1-4, 1-5, 1-6, 1-7, 1-8, 2-4, 2-5, 2-6, 2-7, 2-8, 3-4, 3-5, 3-6, 3-7, 3-8, 4-5, 4-6, 4-7, 4-8, 5-7, 5-8, or 6-8. Particularly preferred embodiments of the invention are polypeptides encoded by differentially spliced polynucleotides, which polypeptides function in cells, especially those which have a biological activity of cathepsin It, most especially those expressed in human cells.

Still further preferred embodiments of the invention are polypeptides encoded by polynucleotides comprising exon polynucleotide sequences, particularly polynucleotides comprising cathepsin K exon 1 [SEQ ID NO: 3], 2 [SEQ ID NO: 5], 3 [SEQ ID NO: 7], 4 [SEQ ID NO: 9], 5 [SEQ ID NO: 11], 6 [SEQ ID NO: 13], 7 [SEQ ID NO: 15] or 8 [SEQ ID NO: 17], having the exon polynucleotide sequence set out in Figures 1 [SEQ ID NO: 1] and 3 [SEQ ID NO: 2-19], or variants, close homologs, derivatives and analogs thereof, as described above, and polypeptides encoded by polynucleotides which are complementary to such polynucleotides. Other preferred embodiments of the invention are polypeptides encoded by polynucleotides comprising comprising cathepsin K exon exon 1 [SEQ ID NO: 3], 2 [SEQ ID NO: 5], 3 [SEQ ID NO: 7], 4 [SEQ ID NO: 9], 5 [SEQ ID NO: 11], 6 [SEQ ID NO: 13], 7 [SEQ ID NO: 15] or 8 [SEQ ID NO: 17], operatively liked to the intron of a gene other then cathepsin K, or joined to an exon of another gene.

The invention also relates to fragments, analogs and derivatives of these polypeptides. The terms "fragment," "derivative" and "analog" when referring to the polypeptide of Figure 2 [SEQ ID NO:20], a polypeptide encoded by an unspliced or differentially spliced hnRNA or mRNA transcribed from the sequence of Figure 1 [SEQ ID NO: 1], or that encoded by the deposited gDNA, means a polypeptide which retains essentially the same biological function or activity as such polypeptide. Thus, an analog includes a proprotein which can be activated by cleavage of the proprotein portion to produce an active mature polypeptide.

The polypeptide of the present invention may he a recombinant polypeptide, a natural polypeptide or a synthetic polypeptide. In certain preferred embodiments it is a recombinant polypeptide.

The fragment, derivative or analog of the polypeptide of Figure 2 [SEQ ID NO:20], or that encoded by an unspliced or differentially spliced hnRNA or mRNA transcribed from the sequence of Figure 1 [SEQ ID NO: 1], or that encoded by the gDNA in the deposited clone may be (i) one in which one or more of the amino acid residues are substituted with a conserved or non-conserved amino acid residue (preferably a conserved amino acid residue) and such substituted amino acid residue may or may not be one encoded by the genetic code, or (ii) one in which one or more of the amino acid residues includes a substituent group, or (iii) one in which the mature polypeptide is fused with another compound, such as a compound to increase the half-life of the polypeptide (for example, polyethylene glycol), or (iv) one in which the additional amino acids are fused to the mature polypeptide, such as a leader or secretory sequence or a sequence which is employed for purification of the mature polypeptide or a proprotein sequence. Such fragments, derivatives and analogs are deemed to be within the scope of those skilled in the art from the teachings herein.

Among the particularly preferred embodiments of the invention in this regard are polypeptides having the amino acid sequence of cathepsin K set out in Figure 2 [SEQ ID NO:20], variants, analogs, derivatives and fragments thereof, and variants, analogs and derivatives of the fragments. Alternatively, particularly preferred embodiments of the invention in this regard are polypeptides having the amino acid sequence of the cathepsin K of the gDNA in the deposited clone, variants, analogs, derivatives and fragments thereof, and variants, analogs and derivatives of the fragments.

Among preferred variants are those that vary from a reference by conservative amino acid substitutions. Such substitutions are those that substitute a given amino acid in a polypeptide by another amino acid of like characteristics. Typically seen as conservative substitutions are the replacements, one for another, among the aliphatic amino acids Ala, Val, Leu and Ile; interchange of the hydroxyl residues Ser and Thr, exchange of the acidic residues Asp and Glu, substitution between the amide residues Asn and Gln, exchange of the basic residues Lys and Arg and replacements among the aromatic residues Phe, Tyr.

Further particularly preferred in this regard are variants, analogs, derivatives and fragments, and variants, analogs and derivatives of the fragments, having the amino acid sequence of the cathepsin K polypeptide of Figure 2 [SEQ ID NO:20] or of the gDNA in the deposited clone, in which several, a few, 5 to 10, 1 to 5, 1 to 3, 2, 1 or no amino acid residues are substituted, deleted or added, in any combination. Especially preferred among these are silent substitutions, additions and deletions, which do not alter the properties and activities of the cathepsin K. Also especially preferred in this regard are conservative substitutions. Most highly preferred are polypeptides having the amino acid sequence of Figure 2 [SEQ ID NO:20] or the deposited clone without substitutions.

The polypeptides and polynucleotides of the present invention are preferably provided in an isolated form, and preferably are purified to homogeneity.

The polypeptides of the present invention include the polypeptide encoded by at least one of the exons of SEQ ID NO: 3, SEQ ID NO: 5, SEQ ID NO: 7, SEQ ID NO: 9, SEQ ID NO: 11, SEQ ID NO: 13, SEQ ID NO: 15 or SEQ ID NO: 17, (in particular the mature polypeptide) as well as polypeptides which have at least 70% similarity (preferably at least 70% identity) to the polypeptide encoded by at least one of the exons of SEQ ID NO: SEQ ID NO: 3, SEQ ID NO: 5, SEQ ID NO: 7, SEQ ID NO: 9, SEQ ID NO: 11, SEQ ID NO: 13, SEQ ID NO: 15 or SEQ ID NO: 17 and more preferably at least 90% similarity (more preferably at least 90% identity) to the polypeptide encoded by at least one of the exons of SEQ ID NO: SEQ ID NO: 3, SEQ ID NO: 5, SEQ ID NO: 7, SEQ ID NO: 9, SEQ ID NO: 11, SEQ ID NO: 13, SEQ ID NO: 15 or SEQ ID NO: 17 and still more preferably at least 95% similarity (still more preferably at least 95% identity) to the polypeptide encoded by at least one of the exons of SEQ ID NO: SEQ ID NO: 3, SEQ ID NO: 5, SEQ ID NO: 7, SEQ ID NO: 9, SEQ ID NO: 11, SEQ ID NO: 13, SEQ ID NO: 15 or SEQ ID NO: 17 and also include portions of such polypeptides with such portion of the polypeptide generally containing at least 30 amino acids and more preferably at least 50 amino acids.

As known in the art "similarity" between two polypeptides is determined by comparing the amino acid sequence and its conserved amino acid substitutes of one polypeptide to the sequence of a second polypeptide.

Fragments or portions of the polypeptides of the present invention may be employed for producing the corresponding full-length polypeptide by peptide synthesis; therefore, the fragments may be employed as intermediates for producing the full-length polypeptides. Fragments or portions of the polynucleotides of the present invention may be used to synthesize full-length polynucleotides of the present invention.

### Fragments

Also among preferred embodiments of this aspect of the present invention are polypeptides comprising fragments of cathepsin K, most particularly fragments of the cathepsin K having the amino acids encoded by the exons set out in Figure 1 [SEQ ID NO: 1], or having the amino acid encoded by the exon sequence of the cathepsin K of the deposited clone, and exon fragments or variants and derivatives of the cathepsin K of Figure 1 [SEQ ID NO: 1] or of the deposited clone.

In this regard a fragment is a polypeptide having an amino acid sequence that entirely is the same as part but not all of the amino acid sequence of the aforementioned cathepsin K polypeptides and variants or derivatives thereof.

Such fragments may be "free-standing," i.e., not part of or fused to other amino acids or polypeptides, such as, for example, an exon, or they may be comprised within a larger polypeptide of which they form a part or region. When comprised within a larger polypeptide, the presently discussed fragments most preferably form a single continuous region. However, several fragments may he comprised within a single larger polypeptide. For instance, certain preferred embodiments relate to a fragment of a cathepsin K polypeptide of the present comprised within a precursor polypeptide designed for expression in a host and having heterologous pre and pro-polypeptide regions fused to the amino terminus of the cathepsin K fragment and an additional region fused to the carboxyl terminus of the fragment. Therefore, fragments in one aspect of the meaning intended herein, refers to the portion or portions of a fusion polypeptide or fusion protein derived from cathepsin K.

As representative examples of polypeptide fragments of the invention, there may be mentioned those which are encoded by the polynucleotide sequence comprising cathepsin K exon 1, 2, 3, 4, 5, 6, 7 or 8, having the exon or intron 1,2,3,4,5,6 or 7 polynucleotide sequences respectively as set out in Figures 1 [SEQ ID NO: 1] and 3 [SEQ ID NO: 2-19], or variants, close homologs, derivatives and analogs thereof, as described above, and polypeptides encoded by polynucleotides which are complementary to such polynucleotides.

In this context about includes the particularly recited range and ranges larger or smaller by several, a few, 5, 4, 3, 2 or 1 amino acid at either extreme or at both extremes. For instance, about 65-90 amino acids in this context means a polypeptide fragment of 65 plus or minus several, a few, 5, 4, 3, 2 or 1 amino acids to 90 plus or minus several a few, 5, 4, 3, 2 or 1 amino acid residues, i.e., ranges as broad as 65 minus several amino acids to 90 plus several amino acids to as narrow as 65 plus several amino acids to 90 minus several amino acids.

Highly preferred in this regard are the recited ranges plus or minus as many as 5 amino acids at either or at both extremes. Particularly highly preferred are the recited ranges plus or minus as many as 3 amino acids at either or at both the recited extremes. Especially particularly highly preferred are ranges plus or minus 1 amino acid at either or at both extremes or the recited ranges with no additions or deletions. Most highly preferred of all in this regard are fragments encoded by each of the exons of cathepsin K.

Among especially preferred fragments of the invention are truncation mutants of cathepsin K. Truncation mutants include cathepsin K polypeptides having the amino acid sequence encoded by the exons of Figure 1 [SEQ ID NO: 1], or of the deposited clone, or of variants or derivatives thereof, except for deletion of a continuous series of residues (that is, a continuous region, part or portion) that includes the amino terminus, or a continuous series of residues that includes the carboxyl terminus or, as in double truncation mutants, deletion of two continuous series of residues, one including the amino terminus and one including the carboxyl terminus. Fragments having the size ranges set out about also are preferred embodiments of truncation fragments, which are especially preferred among fragments generally.

Also preferred in this aspect of the invention are fragments characterized by structural or functional attributes of cathepsin K. Preferred embodiments of the invention in this regard include fragments that comprise alpha-helix and alpha-helix forming regions ("alpha-regions"), beta-sheet and beta-sheet-forming regions ("beta-regions"), turn and turn-forming regions ("turn-regions"), coil and coil-forming regions ("coil-regions"), hydrophilic regions, hydrophobic regions, alpha amphipathic regions, beta amphipathic regions, flexible regions, surface-forming regions and high antigenic index regions of cathepsin K.

Certain preferred regions include Garnier-Robson alpha-regions, beta-regions, turn-regions and coil-regions, Chou-Fasman alpha-regions, beta-regions and turn-regions, Kyte-Doolittle hydrophilic regions and hydrophilic regions, Eisenberg alpha and beta amphipathic regions, Karplus-Schulz flexible regions, Emini surface-forming regions and Jameson-Wolf high antigenic index regions.

Among highly preferred fragments in this regard are those that comprise regions of cathepsin K that combine several structural features, such as several of the features set out above. In this regard, the exon sequences of Figure 1 [SEQ ID NO: 1], which all are characterized by encoding amino acid compositions highly characteristic of turn-regions, hydrophilic regions, flexible-regions, surface-forming regions, and high antigenic index-regions, are especially highly preferred regions. Such regions may be comprised within a larger polypeptide or may be by themselves a preferred fragment of the present invention, as discussed above. It will be appreciated that the term "about" as used in this paragraph has the meaning set out above regarding fragments in general.

Further preferred regions are those that mediate activities of cathepsin K. Most highly preferred in this regard are fragments that have a chemical, biological, antigenic or other activity of cathepsin K, including those with a similar activity or an improved activity, or with a decreased undesirable activity.

It will be appreciated that the invention also relates to, among others, polynucleotides encoding the aforementioned fragments, polynucleotides that hybridize to polynucleotides encoding the fragments, particularly those that hybridize under stringent conditions, and polynucleotides, such as PCR primers, for amplifying polynucleotides that encode the fragments. In these regards, preferred polynucleotides are those that correspondent to the preferred fragments, as discussed above.

Other preferred polynucleotides are genetic elements of cathepsin K, including, but not being limited to, a polyadenylation region, enhancers, a promoter, a cap site introns, exons, and splice sites (references describing these elements include, Darnel, J. et al. *Molecular Cell Biology*, second edition, W.H. Freeman, New York (1990); Watson, J.D., et al. *Molecular Biology of the Gene*, Benjamin/Cummings Pub., Menlo Park, CA, (1987)).

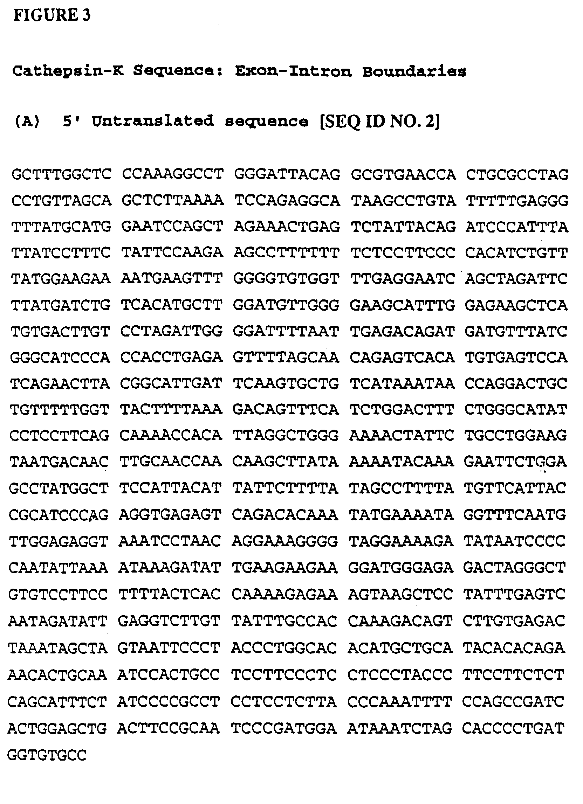

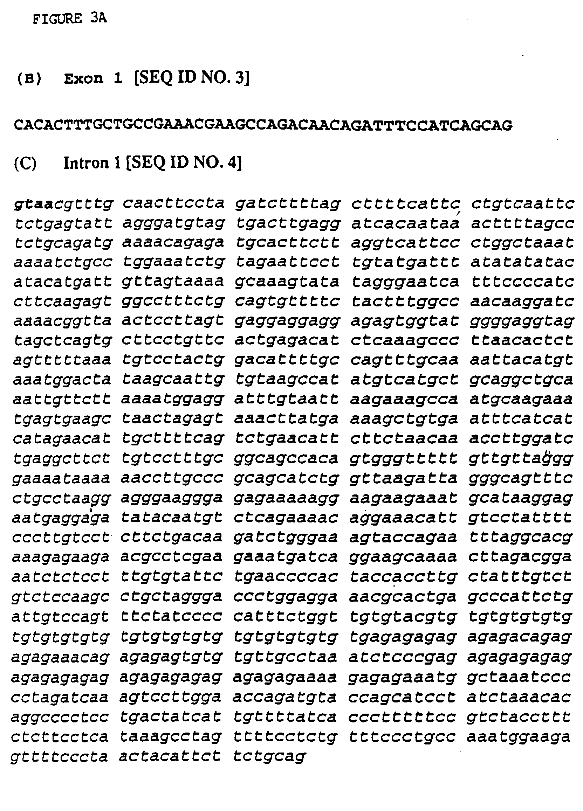

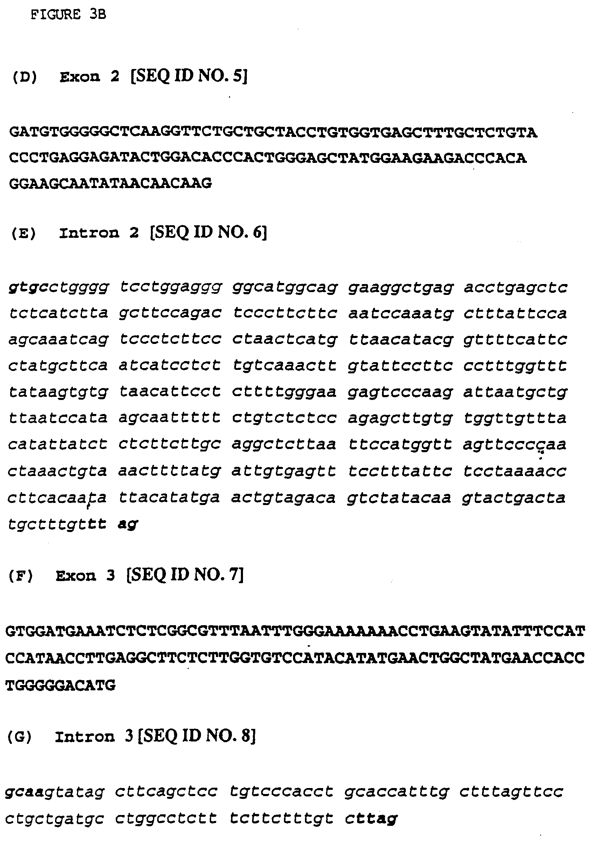

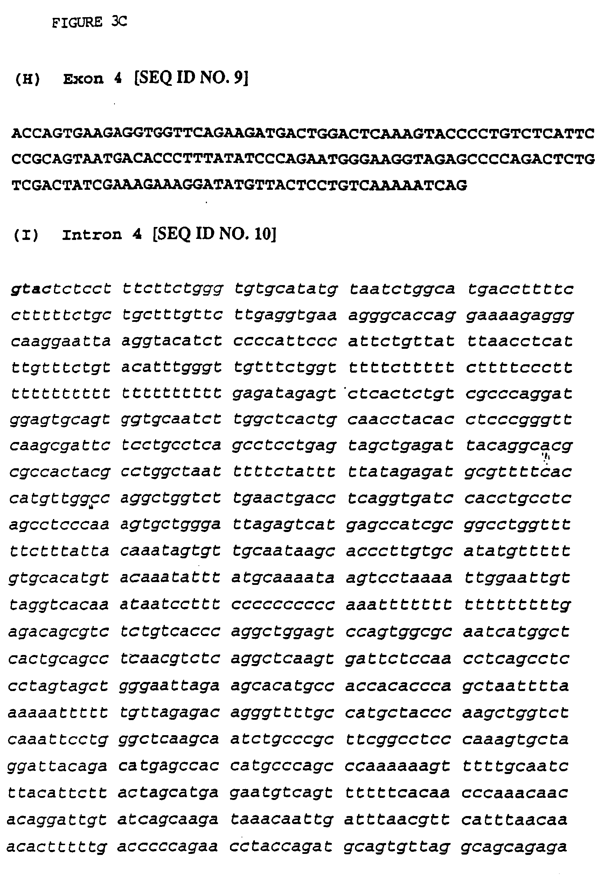

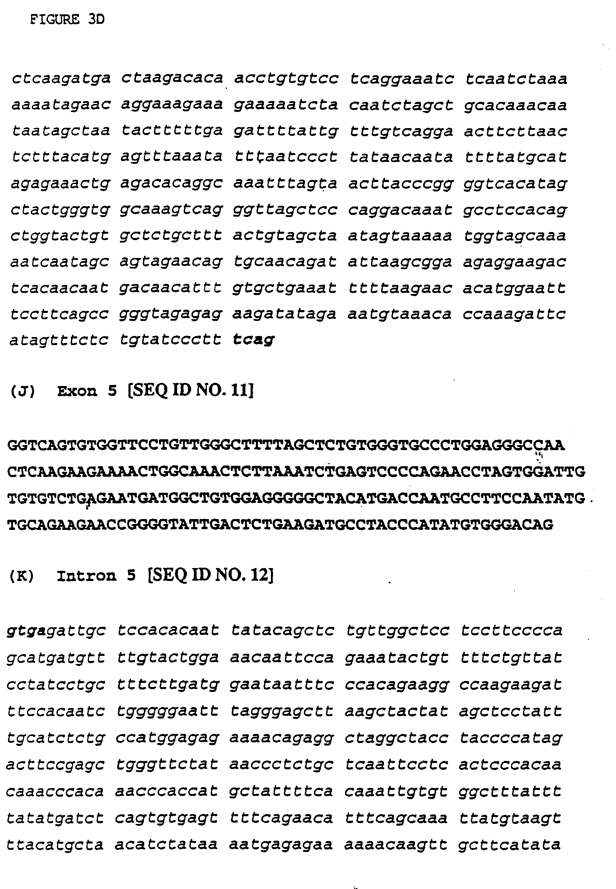

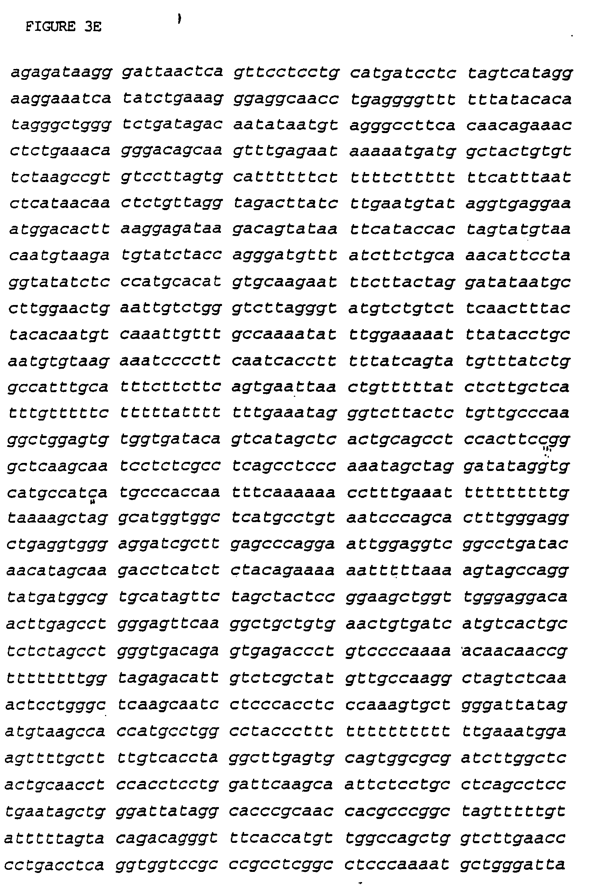

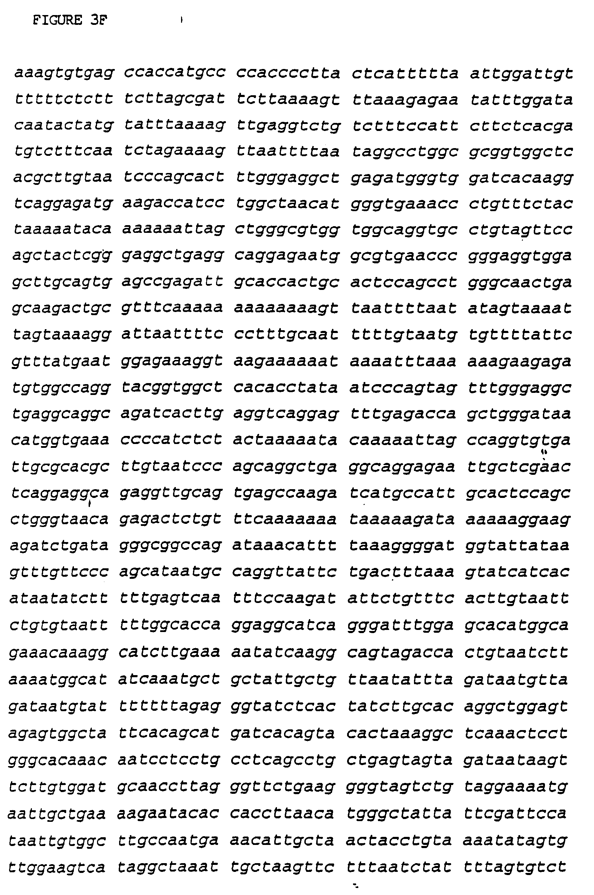

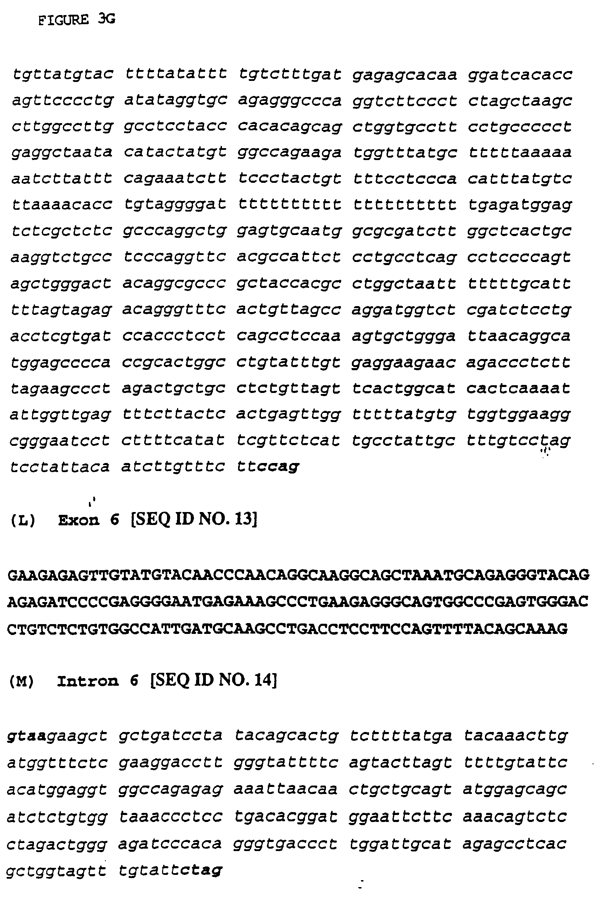

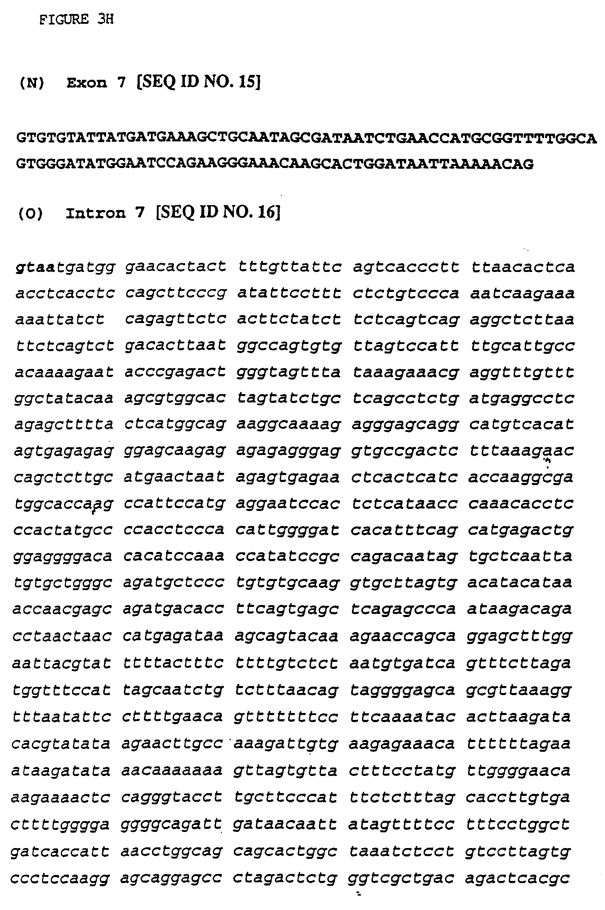

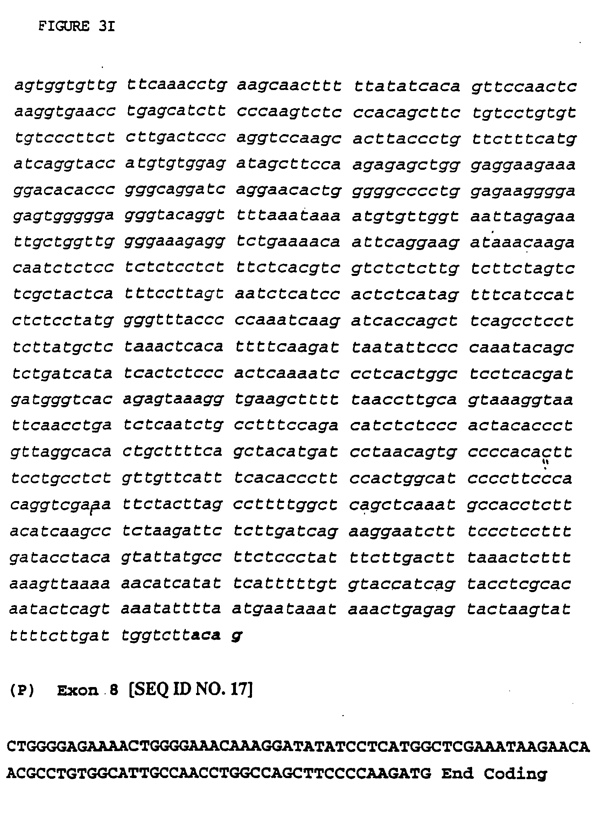

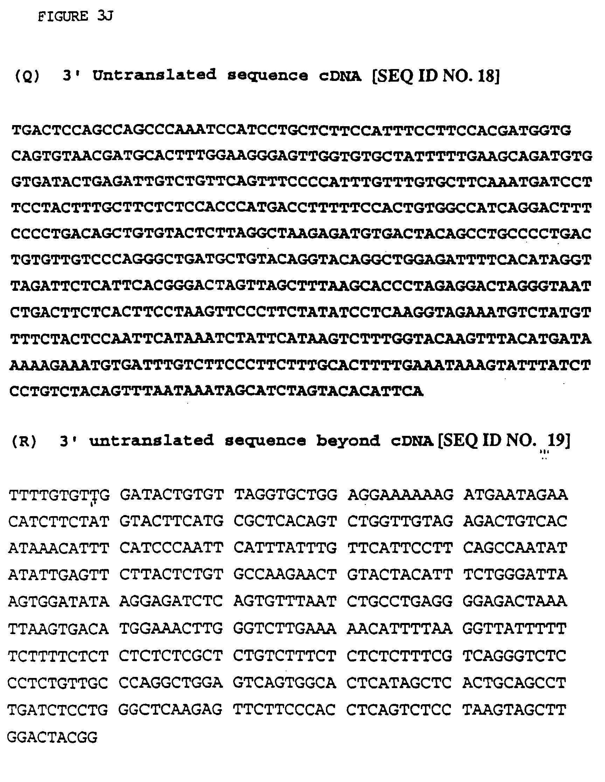

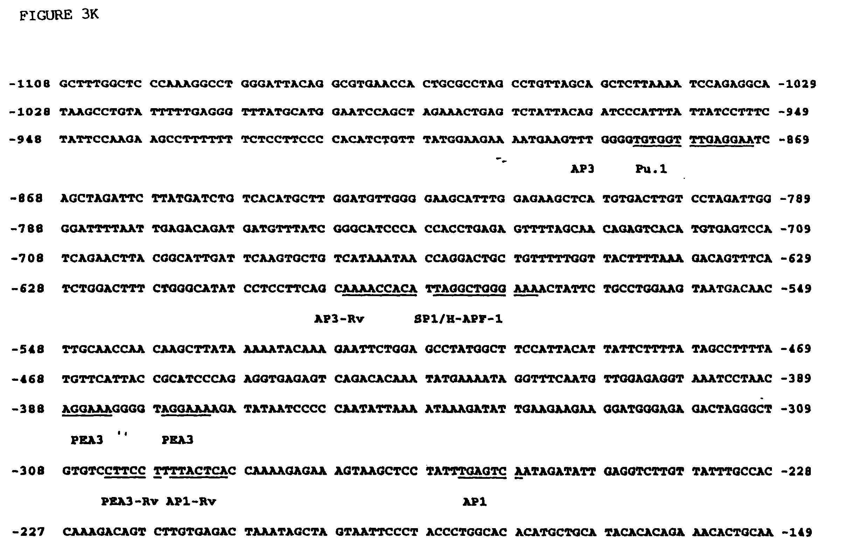

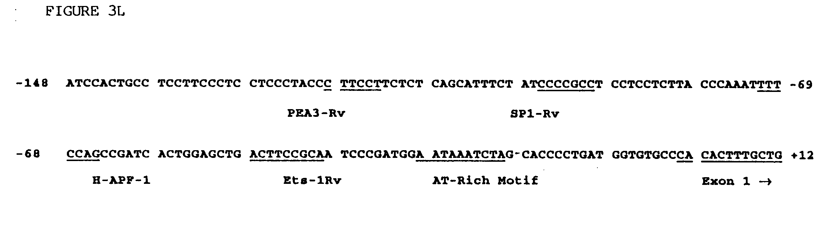

Untranslated regions contain many elements important in regulating gene expression. Mutations and markers in these regions have also been associated with disease (Ozawa T, et al., European Journal of Immunogenetics, APR 1995, 22 (2), 163-169). A preferred embodiment of the invention is the 5'UTR, particularly the sequence set forth in Figure 3(A) [SEQUENCE ID NO: 2]. Mutations and markers in the 5' UTR have been associated with disease (Carlock L, et al., Human Genetics, APR 1994, 93 (4), 457-459). A particularly preferred polynucleotide is an enhancer and promoter in the 5' UTR region of the cathepsin K gDNA. Enhancers are often found in the 5' UTR and upregulate gene expression (see Miller et al., Biotechniques 7:980-990 (1989) for a general reference on promoters). The enhancer of the present invention can be operatively fused to heterologous genes to upregulate gene expression. It is believed that the enhancer promoter will regulate tissue-specific gene expression, being particularly useful to express genes is osteoclast and leukocytes, particularly macrophages cells. A particularly preferred polynucleotide is the enhancer promoter having the sequence set forth in Figure 3(A) [SEQUENCE ID NO: 2]. Transcription factors are often associated with the enhancer and promoter and act to modulate the function of these regions and binding sites for these factors have been described (Faisst, Steffen and Meyer, Silke, Nucleic Acids Research, Vol. 20, No. 1, pp. 3-26, 1991; Smale, Stephen T., Transcription: Mechanisms and Regulation, Raven Press, Ltd. pp. 63-81 (1994)). These sites bind such factors as, for example, Sp1, Ap1, and Ap3 which are involved in transcription initiation (Faisst, Steffen and Meyer, Silke, Nucleic Acids Research, Vol. 20, No. 1, pp. 3-26, 1991). Preferred canonical binding sites for transcription factors are underlined in Figure 3(S) [SEQUENCE ID NO: 2]. The Pu Box in Figure 3(S) [SEQUENCE ID NO: 2] has been described to be present in a macrophage gene, a cell in which cathepsin K is also found (Zhang, Dong-Er, Mol. and Cell. Biol., Vol. 14, No. 1, pp. 373-381 (1994)). The present invention provides a promoter region that is useful, among other things, for the mediation of tissue-specific expression in osteoclasts and leukocytes, particularly macrophages. A Pu box (AGGAA), present in the enhancer and promoter region has also been observed in a macrophage cell line (THP1). Pu boxes in the sequences in the invention are provided. These Pu boxes are believed to be active in the cathepsin K gene in macrophages. RT-PCR performed in THP1 cells, using cathepsin K sequence as a probe, showed expression. The promoter is particularly useful for the study of the control of cathepsin K gene expression, particularly as a region to be probed to diagnose disease. Vitamin D response elements have been found in the 5'UTR of known genes (Kahlen, Jean-Pierre and Carlberg, Carsten, Biochemical & Biophysical Research Communications, Vol. 202, No. 3, pp. 1366-1372 (1994); Darwish, Hisham and DeLuca, Hector, Critical Reviews in Eukaryotic Gene Expression, 3(2):89-116 (1993); Carlberg, Carsten, Eur. J. Biochem. 231, pp. 517-527 (1995); Ohyama, Yoshihiko, J. Biol. Chem., Vol. 269, No. 14, pp. 10545-10550 (1994)). Portions of vitamin D ("vD half sites") responsive elements and calcium ion responsive elements ("Ca half pairs") are present in the 3' UTR sequence as set forth in Figure 3(S) [SEQUENCE ID NO: 2]. Such sites have been described (Katz, Ronald, W., Subauste, Jose, S., and Koenig, Ronald J., J. Biol. Chem., Vol. 270, No. 10, pp. 5238-5242 (1995)). Other half sites present in the sequence of the 5'UTR set forth in Figure 3(A and S) [SEQUENCE ID NO: 2] include osteopontin/parathyroid hormone responsive element, calcitrol response element and osteocalcin half site (see, for example, Juge-Aubry, Cristiana, et al., J. Biol. Chem., Vol. 270, No. 30, pp. 18117-18122 (1995)). Promoter factor binding sites found in the promoter and enhancer region and provided in the invention are also found in cathepsin K introns. Estrogen response elements are also expected to be present in cathepsin K 5' UTR. Skilled artisans can readily find such elements using the methods provided herein.

A further preferred polynucleotide is a cap site located 49 base pairs upstream of the ATG start codon of the sequence set forth in (Figure 2).

A further preferred embodiment of the invention is the promoter region of cathepsin K (Figure 3(A) and (S) [SEQUENCE ID NO: 2]). Functional promoter region sequences have been described (Corden, J., et al., Science, 209, pp. 1406-1414 (1990)). A non-canonical promoter region in the sequence of cathepsin K set forth in Figures 3(A) [SEQUENCE ID NO: 2] and (S) [SEQUENCE ID NO: 2] comprises an A-T rich stretch at 19-27 base pairs upstream of the start codon ATG. Mutations in the TATA box region of promoters have been shown to be associated with disease (Peltoketo H, et al., Genomics, 1994, 23 (1), 250-252).

The 3' untranslated region of cathepsin K is a preferred polynucleotide of the invention, especially that polynucleotide set forth in Figure 3(Q) [SEQUENCE ID NO: 18], especially that region set forth in Figure 3(R) [SEQUENCE ID NO: 19]. Mutations in the 3' UTR have been associated with disease (Saito A, et al., Journal of the American Society of Nephrology, 1994, 4 (9), 1649-1653; Payne SJ, et al., Human Molecular Genetics, 1994,3 (2), 390). The polyadenylation region set forth in Figure 3(Q) [SEQUENCE ID NO: 18] is also a preferred polynucleotide of the 3' UTR. The polyadenylation region comprises two copies of the canonical polyadenylation hexanucleotide, AATAAA. The polyadenylation region can be used, for example, in expression vectors to mediate mRNA 3' end formation (see, for example Gil, A. *et al*., *Nature 312*:473-474 (London) (1984)).

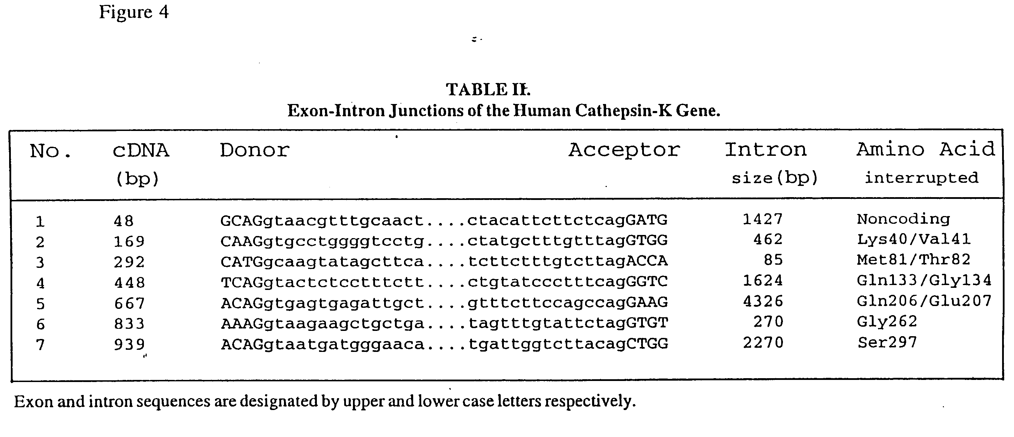

Other particularly preferred polynucleotides of the invention are the splice sites, including, but not limited to the splice donors, splice acceptors and the splice branchpoint. Splice junctions formation is essential for the proper creation of an open reading frame (Mount, Stephen, M., Department of Molecular Biophysics and Biochemistry, Yale University, Sterling Hall of Medicine, New Haven, CT, USA, IRL Press Limited, London, pp. 459-472 (1981)). Diseases associated with the improper formation of the splice junction are known. Particularly preferred splice junction polynucleotides are set forth in Figure 4.

Introns comprise elements important in gene expression and in the formation of mature mRNA. Mutations and markers in introns have been shown to be associated with diseases (Peral, G. et al., Human Molecular Genetics, APR 1995, 4 (4), 569-574; Chrysogelos, S.A., Nucleic Acids Research, 1993, 21 (24), 5736-5741; Ameis, D., Journal of Lipid Research, 1995, 36 (2), 241-250). The splice junctions have also been shown to be associated with disease (Ameis D, et al., Journal of Lipid Research, FEB 1995, 36 (2), 241-250; Petrini JHJ, et al., Journal of Immunology, 1994, 152 (1), 176-183; Kleiman FE, et al., Human Genetics, 1994, 94 (3), 279-282). Alternative splicing and cryptic splice sites selection also have been shown to he associated with disease (Arakawa H, et al., Human Molecular Genetics, 1994, 3 (4), 565-568; Tieu PT, et al., Human Mutation, 1994, 3 (3), 333-336; Reale MA, et al., Cancer Research, 1994, 54 (16), 4493-4501). Introns may also comprise enhancer elements as part of their sequence.

Preferred embodiments of the invention are the cathepsin K introns, particularly those introns having the sequences set forth in Figure 3 (C, E, G, I, K, M, and O) [SEQUENCE ID NO: 4, 6, 8, 10, 14, and 16]. Polymorphisms in the introns can serve as markers for disease following linkage analysis. Moreover, genetic analyses described herein can be used to locate mutations in the introns associated with and/or causing disease.

Another preferred embodiment is a cathepsin K intronic enhancer.

Intron 3 does not follow consensus splice junction GT/AG rule. This intron/exon boundaries was verified by sequencing of the P1 clone and the genomic DNA. GC/AG splice junctions though not common, have been described(Mount, Stephen, M., Department of Molecular Biophysics and Biochemistry, Yale University, Sterling Hail of Medicine, New Haven, CT, USA, IRL Press Limited, London, pp. 459-472 (1981)).

Further preferred embodiments of the invention are the cathepsin K exons, particularly those exons having the sequences set forth in Figure 3 (B, D, F, H, J, L, N, and P) [SEQUENCE ID NO: 3, 5, 7, 9, 11, 13, 15 and 17 respectively]. Polymorphisms in the exons can serve as markers for disease following linkage analysis. Moreover, genetic analyses described herein can be used to locate mutations in the exons associated with and/or causing disease.

Polynucleotide fragments of the invention can be used to create ribozymes that inhibit the expression of the cathepsin K gene. General methods for the construction of ribozyme constructs are known in the art (Stram Y, and Molad T, Virus Genes, 1995, 9 (2), 155-159). Skilled artisans can readily adapt these methods using the novel fragments of the invention to create novel ribozyme constructs. Preferred ribozyme constructs comprise sequences which are complementary to the transcribed control elements of the cathepsin K gene, particularly polynucleotides that are complementary to the 5' untranslated region, splice junctions, and 3'untranslated region, especially the polyadenylation region.

The fragments of the invention, particularly regions in the untranslated region, the promoter and introns are useful as diagnostic probes for disease, particularly bone disease, such as osteoporosis, and including, for example, Paget's disease, Gaucher's disease, CNS inflammation, Alzheimer's disease, hyperparathyroidism, bone degradation, metastatic tumors, rhemuatoid arthritis, osteoarthritis, periodontal disease and degradation of bone implants and bone protheses, particularly dental implants. Moreover, markers for disease can be located in regions of the cathepsin gene, particularly untranslated regions, which are useful with the diagnostic methods of the invention.

### Vectors, host cells, expression

The present invention also relates to vectors which include polynucleotides of the present invention, host cells which are genetically engineered with vectors of the invention and the production of polypeptides of the invention by recombinant techniques.

Host cells can be genetically engineered to incorporate polynucleotides and express polypeptides of the present invention. For instance, polynucleotides may be introduced into host cells using well known techniques of infection, transduction, transfection, transvection and transformation. The polynucleotides may be introduced alone or with other polynucleotides. Such other polynucleotides may be introduced independently, co-introduced or introduced joined to the polynucleotides of the invention.

Thus, for instance, polynucleotides of the invention may be transfected into host cells with another, separate, polynucleotide encoding a selectable marker, using standard techniques for co-transfection and selection in, for instance, mammalian cells. In this case the polynucleotides generally will be stably incorporated into the host cell genome.

Alternatively, the polynucleotides may be joined to a vector containing a selectable marker for propagation in a host. The vector construct may be introduced into host cells by the aforementioned techniques. Generally, a plasmid vector is introduced as DNA in a precipitate, such as a calcium phosphate precipitate, or in a complex with a charged lipid. Electroporation also may be used to introduce polynucleotides into a host. If the vector is a virus, it may be packaged in vitro or introduced into a packaging cell and the packaged virus may be transduced into cells. A wide variety of techniques suitable for making polynucleotides and for introducing polynucleotides into cells in accordance with this aspect of the invention are well known and routine to those of skill in the art. Such techniques are reviewed at length in Sambrook et al. cited above, which is illustrative of the many laboratory manuals that detail these techniques.

In accordance with this aspect of the invention the vector may he, for example, a plasmid vector, a single or double-stranded phage vector, a single or double-stranded RNA or DNA viral vector. Such vectors may be introduced into cells as polynucleotides, preferably DNA, by well known techniques for introducing DNA and RNA into cells. The vectors, in the case of phage and viral vectors also may be and preferably are introduced into cells as packaged or encapsidated virus by well known techniques for infection and transduction. Viral vectors may be replication competent or replication defective. In the latter case viral propagation generally will occur only in complementing host cells.

Preferred among vectors, in certain respects, are those for expression of polynucleotides and polypeptides of the present invention. Generally, such vectors comprise cis-acting control regions effective for expression in a host operatively linked to the polynucleotide to be expressed. Appropriate trans-acting factors either are supplied by the host, supplied by a complementing vector or supplied by the vector itself upon introduction into the host.

In certain preferred embodiments in this regard, the vectors provide for specific expression. Such specific expression may be inducible expression or expression only in certain types of cells or both inducible and cell-specific. Particularly preferred among inducible vectors are vectors that can be induced for expression by environmental factors that are easy to manipulate, such as temperature and nutrient additives. A variety of vectors suitable to this aspect of the invention, including constitutive and inducible expression vectors for use in prokaryotic and eukaryotic hosts, are well known and employed routinely by those of skill in the art.

The engineered host cells can be cultured in conventional nutrient media, which may be modified as appropriate for, *inter alia*, activating promoters, selecting transformants or amplifying genes. Culture conditions, such as temperature, pH and the like, previously used with the host cell selected for expression generally will be suitable for expression of polypeptides of the present invention as will be apparent to those of skill in the art.

A great variety of expression vectors can be used to express a polypeptide of the invention. Such vectors include chromosomal, episomal and virus-derived vectors e.g., vectors derived from bacterial plasmids, from bacteriophage, from yeast episomes, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids, all may be used for expression in accordance with this aspect of the present invention. Generally, any vector suitable to maintain, propagate or express polynucleotides to express a polypeptide in a host may be used for expression in this regard.

The appropriate DNA sequence may be inserted into the vector by any of a variety of well-known and routine techniques. In general, a DNA sequence for expression is joined to an expression vector by cleaving the DNA sequence and the expression vector with one or more restriction endonucleases and then joining the restriction fragments together using T4 DNA ligase. Procedures for restriction and ligation that can be used to this end are well known and routine to those of skill. Suitable procedures in this regard, and for constructing expression vectors using alternative techniques, which also are well known and routine to those skill, are set forth in great detail in Sambrook et al. cited elsewhere herein.

The DNA sequence in the expression vector is operatively linked to appropriate expression control sequence(s), including, for instance, a promoter to direct mRNA transcription. Representatives of such promoters include the phage lambda PL promoter, the *E. coli* lac, trp and tac promoters, the SV40 early and late promoters and promoters of retroviral LTRs, to name just a few of the well-known promoters. It will be understood that numerous promoters not mentioned are suitable for use in this aspect of the invention are well known and readily may be employed by those of skill in the manner illustrated by the discussion and the examples herein.

In general, expression constructs will contain sites for transcription initiation and termination, and, in the transcribed region, a ribosome binding site for translation. The coding portion of the mature transcripts expressed by the constructs will include a translation initiating AUG at the beginning and a termination codon appropriately positioned at the end of the polypeptide to be translated.

In addition, the constructs may contain control regions that regulate as well as engender expression. Generally, in accordance with many commonly practiced procedures, such regions will operate by controlling transcription, such as repressor binding sites and enhancers, among others.

Vectors for propagation and expression generally will include selectable markers. Such markers also may be suitable for amplification or the vectors may contain additional markers for this purpose. In this regard, the expression vectors preferably contain one or more selectable marker genes to provide a phenotypic trait for selection of transformed host cells. Preferred markers include dihydrofolate reductase or neomycin resistance for eukaryotic cell culture, tetracycline, kanamycin, and ampicillin resistance genes for culturing *E. coli* and other bacteria.

The vector containing the appropriate DNA sequence as described elsewhere herein, as well as an appropriate promoter, and other appropriate control sequences, may be introduced into an appropriate host using a variety of well known techniques suitable to expression therein of a desired polypeptide. Representative examples of appropriate hosts include bacterial cells, such as *E. coli*, streptomyces and *Salmonella typhimurium* cells; fungal cells, such as yeast cells; insect cells such as Drosophila S2 and Spodoptera Sf9 cells; animal cells such as CHO, COS and Bowes melanoma cells; and plant cells. Hosts for a great variety of expression constructs are well known, and those of skill will be enabled by the present disclosure readily to select a host for expressing a polypeptides in accordance with this aspect of the present invention.

More particularly, the present invention also includes recombinant constructs, such as expression constructs, comprising one or more of the sequences described above. The constructs comprise a vector, such as a plasmid or viral vector, into which such a sequence of the invention has been inserted. The sequence may be inserted in a forward or reverse orientation. In certain preferred embodiments in this regard, the construct further comprises regulatory sequences, including, for example, a promoter, operably linked to the sequence. Large numbers of suitable vectors and promoters are known to those of skill in the art, and there are many commercially available vectors suitable for use in the present invention.

The following vectors, which are commercially available, are provided by way of example. Among vectors preferred for use in bacteria are pQE70, pQE60 and pQE-9, available from Qiagen; pBS vectors, Phagescript vectors, Bluescript vectors, pNH8A, pNH16a, pNH18A, pNH46A, available from Stratagene; and ptrc99a, pKK223-3, pKK233-3, pDR540, pRIT5 available from Pharmacia. Among preferred eukaryotic vectors are pWLNEO, pSV2CAT, pOG44, pXT1 and pSG available from Stratagene; and pSVK3, pBPV, pMSG and pSVL available from Pharmacia. These vectors are listed solely by way of illustration of the many commercially available and well known vectors that are available to those of skill in the art for use in accordance with this aspect of the present invention. It will be appreciated that any other plasmid or vector suitable for, for example, introduction, maintenance, propagation or expression of a polynucleotide or polypeptide of the invention in a host may be used in this aspect of the invention.

Promoter regions can be selected from any desired gene using vectors that contain a reporter transcription unit lacking a promoter region, such as a chloramphenicol acetyl transferase ("CAT") transcription unit, downstream of restriction site or sites for introducing a candidate promoter fragment; i.e., a fragment that may contain a promoter. As is well known, introduction into the vector of a promoter-containing fragment at the restriction site upstream of the cat gene engenders production of CAT activity, which can be detected by standard CAT assays. Vectors suitable to this end are well known and readily available. Two such vectors are pKK232-8 and pCM7. Thus, promoters for expression of polynucleotides of the present invention include not only well known and readily available promoters, but also promoters that readily may be obtained by the foregoing technique, using a reporter gene.

A preferred embodiment of the invention are expression vectors comprising cathepsin K promoter sequences that function as a promoter. Such vector constructs may be used for targeted gene expression in cells which utilize the cathepsin K promoter, for example, osteoclasts and macrophages. Any gene of interest can be expressibly linked to the cathepsin K promoter and expressed in such cells which utilize the cathepsin K promoter. In this manner genes which immortalize primary eukaryotic cells, such as, for example, SV40 T-Antigen, may be expressibly linked cathepsin K promoter to immortalize cells, such as, for example, bone cells, including osteoclasts, and macrophages. Certain preferred vectors comprise cathepsin K promoter expressibly linked to a toxin gene, such as for example, ricin, and are useful in methods for the targeted killing of cell populations that utilize the cathepsin K promoter for gene expression. Certain other preferred vectors comprise cathepsin K promoter expressibly linked to a anti-cathepsin K ribozyme or antisense polynucleotide, which are useful in methods for such targeted killing.

Among known bacterial promoters suitable for expression of polynucleotides and polypeptides in accordance with the present invention are the *E. coli* lacI and lacZ promoters, the T3 and T7 promoters, the gpt promoter, the lambda PR, PL promoters and the trp promoter. Among known eukaryotic promoters suitable in this regard are the CMV immediate early promoter, the HSV thymidine kinase promoter, the early and late SV40 promoters, the promoters of retroviral LTRs, such as those of the Rous sarcoma virus ("RSV"), and metallothionein promoters, such as the mouse metallothionein-I promoter.

Selection of appropriate vectors and promoters for expression in a host cell is a well known procedure and the requisite techniques for expression vector construction, introduction of the vector into the host and expression in the host are routine skills in the art.

The present invention also relates to host cells containing the above-described constructs discussed above. The host cell can be a higher eukaryotic cell, such as a mammalian cell or insect cell, or a lower eukaryotic cell, such as a yeast cell, or the host cell can be a prokaryotic cell, such as a bacterial cell.

Introduction of the construct into the host cell can be effected by calcium phosphate transfection, DEAE-dextran mediated transfection, cationic lipid-mediated transfection, electroporation, transduction, infection or other methods. Such methods are described in many standard laboratory manuals, such as Davis et al. BASIC METHODS IN MOLECULAR BIOLOGY, (1986).

Constructs in host cells can be used in a conventional manner to produce the gene product encoded by the recombinant sequence. Alternatively, the polypeptides of the invention can be synthetically produced by conventional peptide synthesizers.

Mature proteins can be expressed in mammalian cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook et al., MOLECULAR CLONING: A LABORATORY MANUAL, 2nd Ed., Cold Spring Harbor Laboratory Press, Cold Spring Harbor, N.Y. (1989).

Generally, recombinant expression vectors for yeast will include origins of replication, a promoter derived from a highly-expressed gene to direct transcription of a downstream structural sequence, and a selectable marker to permit isolation of vector containing cells after exposure to the vector. Among suitable promoters are those derived from the genes that encode glycolytic enzymes such as 3-phosphoglycerate kinase ("PGK"), a-factor, acid phosphatase, and heat shock proteins, among others. Selectable markers include the ampicillin resistance gene of *E. coli* and the trp1 gene of S. cerevisiae.

Transcription of the DNA encoding the polypeptides of the present invention by higher eukaryotes may he increased by inserting an enhancer sequence into the vector. Enhancers are *cis*-acting elements of DNA, usually about from 10 to 300 bp that act to increase transcriptional activity of a promoter in a given host cell-type. Examples of enhancers include the SV40 enhancer, which is located on the late side of the replication origin at bp 100 to 270, the cytomegalovirus early promoter enhancer, the polyoma enhancer on the late side of the replication origin, and adenovirus enhancers.

Polynucleotides of the invention, encoding the heterologous structural sequence of a polypeptide of the invention generally will be inserted into the vector using standard techniques so that it is operably linked to the promoter for expression. The polynucleotide will be positioned so that the transcription start site is located appropriately 5' to a ribosome binding site. The ribosome binding site will be 5' to the AUG that initiates translation of the polypeptide to be expressed. Generally, there will be no other open reading frames that begin with an initiation codon, usually AUG, and lie between the ribosome binding site and the initiating AUG. Also, generally, there will be a translation stop codon at the end of the polypeptide and there will be a polyadenylation signal and a transcription termination signal appropriately disposed at the 3' end of the transcribed region.

For secretion of the translated protein into the lumen of the endoplasmic reticulum, into the periplasmic space or into the extracellular environment, appropriate secretion signals may be incorporated into the expressed polypeptide. The signals may be endogenous to the polypeptide or they may be heterologous signals.

The polypeptide may be expressed in a modified form, such as a fusion protein, and may include not only secretion signals but also additional heterologous functional regions. Thus, for instance, a region of additional amino acids, particularly charged amino acids, may be added to the N-terminus of the polypeptide to improve stability and persistence in the host cell, during purification or during subsequent handling and storage. Also, a region may be added to the polypeptide to facilitate purification. Such regions may be removed prior to final preparation of the polypeptide. The addition of peptide moieties to polypeptides to engender secretion or excretion, to improve stability and to facilitate purification, among others, are familiar and routine techniques in the art.

Suitable prokaryotic hosts for propagation, maintenance or expression of polynucleotides and polypeptides in accordance with the invention include *Escherichia coli*, *Bacillus subtilis* and *Salmonella typhimurium*. Various species of Pseudomonas, Streptomyces, and Staphylococcus are suitable hosts in this regard. Moreover, many other hosts also known to those of skill may be employed in this regard.

As a representative but non-limiting example, useful expression vectors for bacterial use can comprise a selectable marker and bacterial origin of replication derived from commercially available plasmids comprising genetic elements of the well known cloning vector pBR322 (ATCC 37017). Such commercial vectors include, for example, pKK223-3 (Pharmacia Fine Chemicals, Uppsala, Sweden) and GEM1 (Promega Biotec, Madison, WI, USA). These pBR322 "backbone" sections are combined with an appropriate promoter and the structural sequence to be expressed.

Following transformation of a suitable host strain and growth of the host strain to an appropriate cell density, where the selected promoter is inducible it is induced by appropriate means (e.g., temperature shift or exposure to chemical inducer) and cells are cultured for an additional period.

Cells typically then are harvested by centrifugation, disrupted by physical or chemical means, and the resulting crude extract retained for further purification.

Microbial cells employed in expression of proteins can be disrupted by any convenient method, including freeze-thaw cycling, sonication, mechanical disruption, or use of cell lysing agents, such methods are well know to those skilled in the art.

Various mammalian cell culture systems can be employed for expression, as well. Examples of mammalian expression systems include the COS-7 lines of monkey kidney fibroblast, described in Gluzman et al., Cell 23: 175 (1981). Other cell lines capable of expressing a compatible vector include for example, the C127, 3T3, CHO, HeLa, human kidney 293 and BHK cell lines.

Mammalian expression vectors will comprise an origin of replication, a suitable promoter and enhancer, and also any necessary ribosome binding sites, polyadenylation sites, splice donor and acceptor sites, transcriptional termination sequences, and 5' flanking non-transcribed sequences that are necessary for expression. In certain preferred embodiments in this regard DNA sequences derived from the SV40 splice sites, and the SV40 polyadenylation sites are used for required non-transcribed genetic elements of these types.

The cathepsin K polypeptide can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification. Well known techniques for refolding protein may be employed to regenerate active conformation when the polypeptide is denatured during isolation and or purification.

Polypeptides of the present invention include naturally purified products, products of chemical synthetic procedures, and products produced by recombinant techniques from a prokaryotic or eukaryotic host, including, for example, bacterial, yeast, higher plant, insect and mammalian cells. Depending upon the host employed in a recombinant production procedure, the polypeptides of the present invention may be glycosylated or may be non-glycosylated. In addition, polypeptides of the invention may also include an initial modified methionine residue, in some cases as a result of host-mediated processes.

### Further illustrative aspects and preferred embodiments of the invention

Cathepsin K polynucleotides and polypeptides may be used in accordance with the present invention for a variety of applications, particularly those that make use of the chemical and biological properties cathepsin K. Among these are applications in the detection and treatment of disease, particularly bone disease, such as osteoporosis, and including, for example, Paget's disease, Gaucher's disease, CNS inflammation, Alzheimer's disease, hyperparathyroidism, bone degradation, metastatic tumors, rhemuatoid arthritis, osteoarthritis, periodontal disease and degradation of bone implants and bone protheses, particularly dental implants. Additional applications relate to diagnosis and to treatment of disorders of cells, tissues and organisms. These aspects of the invention are illustrated further by the following discussion.

### Polynucleotide assays

This invention is also related to the use of the cathepsin K exons, introns, promoters and polynucleotides to detect complementary polynucleotides such as, for example, as a diagnostic reagent. Detection of a mutated form of cathepsin K associated with a dysfunction will provide a diagnostic tool that can add or define a diagnosis of a disease or susceptibility to a disease which results from under-expression, over-expression or altered expression of cathepsin K, such as, for example, osteoporosis, periodontal disease, Paget's disease, Gaucher's disease, CNS inflammation, Alzheimer's disease, hyperparathyroidism, and bone degradation, metastatic tumors, and degradation of bone implants and bone protheses, particularly dental implants.

Individuals carrying mutations in the human cathepsin K gene may be detected at the DNA level by a variety of techniques. Nucleic acids for diagnosis may be obtained from a patient's cells, such as from blood, urine, saliva, tissue biopsy and autopsy material. The genomic DNA may he used directly for detection or may be amplified enzymatically by using PCR prior to analysis (Saiki et al., Nature, 324: 163-166 (1986)). Ligation-mediated amplification may also be used for amplification (Vollach, V., et al., *Nucl*. *Acids Res*. 22: 2507 (1994). RNA or cDNA may also be used in the same ways. As an example, PCR primers complementary to the nucleic acid encoding cathepsin K can be used to identify and analyze cathepsin K expression and mutations. For example, deletions and insertions can be detected by a change in size of the amplified product in comparison to the normal genotype. Point mutations can be identified by hybridizing amplified DNA to radiolabeled cathepsin K RNA or alternatively, radiolabeled cathepsin K antisense DNA sequences. Perfectly matched sequences can be distinguished from mismatched duplexes by RNase A digestion or by differences in melting temperatures.

Sequence differences between a reference gene and genes having mutations also may be revealed by direct DNA sequencing. In addition, cloned DNA segments may be employed as probes to detect specific DNA segments. The sensitivity of such methods can be greatly enhanced by appropriate use of PCR or another amplification method. For example, a sequencing primer is used with double-stranded PCR product or a single-stranded template molecule generated by a modified PCR. The sequence determination is performed by conventional procedures with radiolabeled nucleotide or by automatic sequencing procedures with fluorescent-tags.

Genetic testing based on DNA sequence differences may be achieved by detection of alteration in electrophoretic mobility of DNA fragments in gels, with or without denaturing agents. Small sequence deletions and insertions can be visualized by high resolution gel electrophoresis. DNA fragments of different sequences may be distinguished on denaturing formamide gradient gels in which the mobilities of different DNA fragments are retarded in the gel at different positions according to their specific melting or partial melting temperatures (see, e.g., Myers et al., Science, 230: 1242 (1985)).

Sequence changes at specific locations also may be revealed by nuclease protection assays, such as RNase and S1 protection or the chemical cleavage method (e.g., Cotton et al., Proc. Natl. Acad. Sci., USA, 85: 4397-4401 (1985)).

Thus, the detection of a specific DNA sequence may be achieved by methods such as hybridization, RNase protection, chemical cleavage, direct DNA sequencing or the use of restriction enzymes, (e.g., restriction fragment length polymorphisms ("RFLP"), SSCP and Southern blotting of genomic DNA.

In addition to more conventional gel-electrophoresis and DNA sequencing, mutations also can be detected by in situ analysis.

### Chromosome assays

The sequences of the present invention are also valuable for chromosome identification. The sequence is specifically targeted to and can hybridize with a particular location on an individual human chromosome. Moreover, there is a current need for identifying particular sites on the chromosome. Few chromosome marking reagents based on actual sequence data (repeat polymorphisms) are presently available for marking chromosomal location. The mapping of DNAs to chromosomes according to the present invention is an important first step in correlating those sequences with genes associated with disease.

In certain preferred embodiments in this regard, the gDNA herein disclosed is used to clone genomic DNA of a cathepsin K gene. This can be accomplished using a variety of well known techniques and libraries, which generally are available commercially. The genomic DNA then is used for in situ chromosome mapping using well known techniques for this purpose. Typically, in accordance with routine procedures for chromosome mapping, some trial and error may he necessary to identify a genomic probe that gives a good in situ hybridization signal.

In some cases, in addition, sequences can be mapped to chromosomes by preparing PCR primers (preferably 15-25 bp) from the gDNA. Computer analysis of the 3' untranslated region of the gene is used to rapidly select primers that do not span more than one exon in the genomic DNA complicate the amplification process. These primers are then used for PCR screening of somatic cell hybrids containing individual human chromosomes. Only those hybrids containing the human gene corresponding to the primer will yield an amplified fragment.

PCR mapping of somatic cell hybrids is a rapid procedure for assigning a particular DNA to a particular chromosome. Using the present invention with the same oligonucleotide primers, sublocalization can be achieved with panels of fragments from specific chromosomes (e.g., radiation hybrid panels) or pools of large genomic clones in an analogous manner. Other mapping strategies that can similarly be used to map to its chromosome include in situ hybridization, prescreening with labeled flow-sorted chromosomes and preselection by hybridization to construct chromosome specific-cDNA libraries.

Fluorescence in situ hybridization ("FISH") of a cDNA clone to a metaphase chromosomal spread can be used to provide a precise chromosomal location in one step. This technique can be used with gDNA as short as 50 to as long as 600. For a review of this technique, see Verma et al., HUMAN CHROMOSOMES: A MANUAL OF BASIC TECHNIQUES, Pergamon Press, New York (1988).

Once a sequence has been mapped to a precise chromosomal location, the physical position of the sequence on the chromosome can be correlated with genetic map data. Such data are found, for example, in V. McKusick, MENDELIAN INHERITANCE IN MAN, available on line through Johns Hopkins University, Welch Medical Library. The relationship between genes and diseases that have been mapped to the same chromosomal region are then identified through linkage analysis (coinheritance of physically adjacent genes).

Next, it is necessary to determine the differences in the cDNA or genomic sequence between affected and unaffected individuals. If a mutation is observed in some or all of the affected individuals but not in any normal individuals, then the mutation is likely to be the causative agent of the disease.

With current resolution of physical mapping and genetic mapping techniques, a gDNA precisely localized to a chromosomal region associated with the disease could be one of between 50 and 500 potential causative genes. (This assumes 1 megabase mapping resolution and one gene per 20 kb).

### Polypeptide assays

The present invention also relates to a diagnostic assays such as quantitative and diagnostic assays for detecting levels of cathepsin K protein in cells, tissues and bodily fluids, including determination of normal and abnormal levels of polypeptide. Bodily fluids useful in the diagnostic methods of the invention include, for example, synovial fluid, cerebrospinal fluid, urine, serum, gingival fluid and lymph. Thus, for instance, a diagnostic assay in accordance with the invention for detecting over-expression of cathepsin K protein compared to normal control tissue samples may be used to detect the presence of disease, for example, osteoporosis, Paget's disease, Gaucher's disease, CNS inflammation, Alzheimer's disease, hyperparathyroidism, bone degradation, metastatic tumors, rhemuatoid arthritis, osteoarthritis, periodontal disease and degradation of bone implants and bone protheses, particularly dental implants. Assay techniques that can be used to determine levels of a protein, such as an immunoassay for cathepsin K protein of the present invention, in a sample derived from a host are well-known to those of skill in the art. Such assay methods include radioimmunoassays, competitive-binding assays, Western Blot analysis and ELISA assays. Among these ELISAs frequently are preferred. An ELISA assay initially comprises preparing an antibody specific to cathepsin K, preferably a monoclonal antibody. In addition a reporter antibody generally is prepared which binds to the monoclonal antibody. The reporter antibody is attached a detectable reagent such as radioactive, fluorescent or enzymatic reagent, in this example horseradish peroxidase enzyme.

To carry out an ELISA a sample is removed from a host and incubated on a solid support, e.g. a polystyrene dish, that binds the proteins in the sample. Any free protein binding sites on the dish are then covered by incubating with a non-specific protein such as bovine serum albumin. Next, the monoclonal antibody is incubated in the dish during which time the monoclonal antibodies attach to any cathepsin K proteins attached to the polystyrene dish. Unbound monoclonal antibody is washed out with buffer. The reporter antibody linked to horseradish peroxidase is placed in the dish resulting in binding of the reporter antibody to any monoclonal antibody bound to cathepsin K. Unattached reporter antibody is then washed out. Reagents for peroxidase activity, including a colorimetric substrate are then added to the dish. Immobilized peroxidase, linked to cathepsin K through the primary and secondary antibodies, produces a colored reaction product. The amount of color developed in a given time period indicates the amount of cathepsin K protein present in the sample. Quantitative results typically are obtained by reference to a standard curve.

A competition assay may be employed wherein antibodies specific to cathepsin K attached to a solid support and labeled cathepsin K and a sample derived from the host are passed over the solid support and the amount of label detected attached to the solid support can be correlated to a quantity of cathepsin K in the sample.

### Antibodies

The polypeptides, their fragments or other derivatives, or analogs thereof, or cells expressing them can be used as an immunogen to produce antibodies thereto. These antibodies can be, for example, polyclonal or monoclonal antibodies. The present invention also includes chimeric, single chain, and humanized antibodies, as well as Fab fragments, or the product of a Fab expression library. Various procedures known in the art may be used for the production of such antibodies and fragments.

Antibodies generated against the polypeptides corresponding to a sequence of the present invention can be obtained by direct injection of the polypeptides into an animal or by administering the polypeptides to an animal, preferably a nonhuman. The antibody so obtained will then bind the polypeptides itself. In this manner, even a sequence encoding only a fragment of the polypeptides can be used to generate antibodies binding the whole native polypeptides. Such antibodies can then be used to isolate the polypeptide from tissue expressing that polypeptide.

For preparation of monoclonal antibodies, any technique which provides antibodies produced by continuous clonal cell line cultures can be used. Examples include the hybridoma technique (Kohler, G. and Milstein, C., Nature 256: 495-497 (1975), the trioma technique, the human B-cell hybridoma technique (Kozbor et al., Immunology Today 4:72 (1983) and the EBV-hybridoma technique to produce human monoclonal antibodies (Cole et al., pg. 77-96 in MONOCLONAL ANTIBODIES AND CANCER THERAPY, Alan R. Liss, Inc. (1985).

Techniques described for the production of single chain antibodies (U.S. Patent No. 4,946,778) can be adapted to produce single chain antibodies to immunogenic polypeptide products of this invention. Also, transgenic mice, or other organisms such as other mammals, may be used to express antibodies, including for example, humanized antibodies to immunogenic polypeptide products of this invention.

Thus, among others, such antibodies can be used to detect and treat diseases caused by or associated with mutant cathepsin K or abnormal cathepsin K levels, such as, osteoporosis, periodontal disease, Paget's disease, Gaucher's disease, CNS inflammation, Alzheimer's disease, hyperparathyroidism, bone degradation, metastatic tumors, and degradation of bone implants and bone protheses, particularly dental implants.

Immunization using polynucleotides of the inventions can be carried out using known methods to produce a cathepsin K-specific immune response.

### Clinical Genomics

This invention provides methods to determine drug responsiveness of individuals having or suspected of having a cathepsin K gene mutation or cathepsin K gene expression abnormality, and also provides reagents to carry out such methods. Individuals may be grouped by their responsiveness to a given compound, particularly drugs, used to treat diseases caused by or associated with a mutation of cathepsin K gene or cathepsin K gene expression. Such individuals may be further grouped by detecting different gene mutations or gene expression level variants. In this manner specific gene mutations and gene expression variants can be readily associated with a certain degree of responsiveness to a compound by an individual). Methods and reagents provided herein can be used to group compound responsiveness by detecting cathepsin K gene mutations and cathepsin K gene expression variants. Other methods for grouping individuals by compound responsivess are known to skilled artisans and can be adapted to use the polypetides and polynucleotides of the invention.

The invention also provides algorithms useful in conjunction with a device or embodied in a composition matter which are useful for the diagnosis of diseases caused by or associated with cathepsin K or mutants or variants thereof. Preferred algorithms are provided for disease stratification and staging.

### Cathepsin K binding molecules and assays

This invention also provides a method for identification of molecules, such as receptor molecules, that bind cathepsin K or fragments of cathepsin K of the invention. Genes encoding proteins that bind cathepsin K, such as receptor proteins, can be identified by numerous methods known to those of skill in the art, for example, ligand panning and FACS sorting. Such methods are described in many laboratory manuals such as, for instance, Coligan et al., Current Protocols in Immunology 1(2): Chapter 5 (1991).

For instance, expression cloning may be employed for this purpose. To this end polyadenylated RNA is prepared from a cell responsive to cathepsin K, a cDNA library is created from this RNA, the library is divided into pools and the pools are transfected individually into cells that are not responsive to cathepsin K. The transfected cells then are exposed to labeled cathepsin K. (Cathepsin K can be labeled by a variety of well-known techniques including standard methods of radio-iodination or inclusion of a recognition site for a site-specific protein kinase.) Following exposure, the cells are fixed and binding of cathepsin K, or a molecule which binds to cathepsin K, is determined. These procedures conveniently are carried out on glass slides.

Pools are identified of cDNA that produced cathepsin K-binding cells. Sub-pools are prepared from these positives, transfected into host cells and screened as described above. Using an iterative sub-pooling and re-screening process, one or more single clones that encode the putative binding molecule or substrate, such as cell matrix, bone matrix or a receptor molecule, and the any of the same can be isolated.

Alternatively a labeled ligand can be photoaffinity linked to a cell extract, such as a membrane or a membrane extract, prepared from cells that express a molecule that it binds, such as a receptor molecule. Cross-linked material is resolved by polyacrylamide gel electrophoresis ("PAGE") and exposed to X-ray film. The labeled complex containing the ligand-receptor can be excised, resolved into peptide fragments, and subjected to protein microsequencing. The amino acid sequence obtained from microsequencing can be used to design unique or degenerate oligonucleotide probes to screen cDNA libraries to identify genes encoding the putative receptor molecule.

Polypeptides of the invention also can be used to assess cathepsin K binding capacity of cathepsin K binding molecules, such as receptor molecules, in cells or in cell-free preparations.

### Agonists and antagonists - assays and molecules

The invention also provides a method of screening compounds to identify those which enhance or block the action of cathepsin K in or on cells, such as its interaction with cathepsin K-binding molecules such as receptor and enzymatic substrate molecules. An agonist is a compound which increases the natural biological functions of cathepsin K, while antagonists decrease or eliminate such functions.

For example, a cellular compartment, such as a membrane, vacuole, inclusion or a preparation of any thereof, such as a membrane-preparation, may be prepared from a cell that expresses a molecule that binds cathepsin K, such as a molecule of a signaling or regulatory pathway modulated by cathepsin K. The preparation is incubated with labeled cathepsin K in the absence or the presence of a candidate molecule which may be a cathepsin K agonist or antagonist. The ability of the candidate molecule to bind the binding molecule, such as a substrate, is reflected in decreased binding of the labeled ligand. Molecules which bind gratuitously, i.e., without inducing the effects of cathepsin K on binding the cathepsin K binding molecule, are most likely to be good antagonists. Molecules that bind well and elicit effects that are the same as or closely related to cathepsin K are agonists.

Cathepsin K-like effects of potential agonists and antagonists may by measured, for instance, by determining activity of a second messenger system following interaction of the candidate molecule with a cell or appropriate cell preparation, and comparing the effect with that of cathepsin K or molecules that elicit the same effects as cathepsin K. Second messenger systems that may be useful in this regard include but are not limited to AMP guanylate cyclase, ion channel, phosphoinositide hydrolysis second messenger systems, or compounds which signal the binding of a potential agonists and antagonists to cathepsin K or its substrate.

Another example of an assay for cathepsin K antagonists is a competitive assay that combines cathepsin K and a potential antagonist with enzymatic substrate or substrate analogs under appropriate conditions for a competitive inhibition assay. Cathepsin K can be labeled, such as by radioactivity, such that the number of cathepsin K molecules bound to a receptor molecule can be determined accurately to assess the effectiveness of the potential antagonist.

Potential antagonists include small organic molecules, peptides, polypeptides and antibodies that bind to a polypeptide of the invention and thereby inhibit or extinguish its activity. Potential antagonists also may be small organic molecules, a peptide, a polypeptide such as a closely related protein or antibody that binds the same sites on a binding molecule, such as a receptor molecule, without inducing cathepsin K-induced activities, thereby preventing the action of cathepsin K by excluding cathepsin K from binding.

Potential antagonists include a small molecule which binds to and occupies the binding site of the polypeptide thereby preventing binding to cellular binding molecules, such as receptor molecules, such that normal biological activity is prevented. Examples of small molecules include but are not limited to small organic molecules, peptides or peptide-like molecules.

Other potential antagonists include antisense molecules. Antisense technology can be used to control gene expression through antisense DNA or RNA or through triple-helix formation. Antisense techniques are discussed, for example, in - Okano, J. Neurochem. 56: 560 (1991); OLIGODEOXYNUCLEOTIDES AS ANTISENSE INHIBITORS OF GENE EXPRESSION, CRC Press, Boca Raton, FL (1988). Triple helix formation is discussed in, for instance Lee et al., Nucleic Acids Research 6: 3073 (1979); Cooney et al., Science 241: 456 (1988); and Dervan et al., Science 251: 1360 (1991). The methods are based on binding of a polynucleotide to a complementary DNA or RNA. For example, the 5' coding portion of a polynucleotide that encodes the mature polypeptide of the present invention may be used to design an antisense RNA oligonucleotide of from about 10 to 40 base pairs in length. A DNA oligonucleotide is designed to be complementary to a region of the gene involved in transcription thereby preventing transcription and the production of cathepsin K. The antisense RNA oligonucleotide hybridizes to the mRNA *in vivo* and blocks translation of the mRNA molecule into cathepsin K polypeptide. The oligonucleotides described above can also be delivered to cells such that the antisense RNA or DNA may be expressed *in vivo* to inhibit production of cathepsin K.

The antagonists may be employed in a composition with a pharmaceutically acceptable carrier, e.g., as hereinafter described.

The antagonists may be employed for instance to treat diseases caused by or associated with mutant cathepsin K or abnormal cathepsin K levels, such as, osteoporosis, Paget's disease, Gaucher's disease, CNS inflammation, Alzheimer's disease, hyperparathyroidism, bone degradation, metastatic tumors, rhemuatoid arthritis, osteoarthritis, periodontal disease and degradation of bone implants and bone protheses, particularly dental implants.

### Compositions

The invention also relates to compositions comprising the polynucleotides or the polypeptides discussed above or the agonists or antagonists. Thus, the polypeptides of the present invention may be employed in combination with a non-sterile or sterile carrier or carriers for use with cells, tissues or organisms, such as a pharmaceutical carrier suitable for administration to a subject. Such compositions comprise, for instance, a media additive or a therapeutically effective amount of a polypeptide of the invention and a pharmaceutically acceptable carrier or excipient. Such carriers may include, but are not limited to, saline, buffered saline, dextrose, water, glycerol, ethanol and combinations thereof. The formulation should suit the mode of administration.

### Kits

The invention further relates to pharmaceutical packs and kits comprising one or more containers filled with one or more of the ingredients of the aforementioned compositions of the invention. Associated with such container(s) can be a notice in the form prescribed by a governmental agency regulating the manufacture, use or sale of pharmaceuticals or biological products, reflecting approval by the agency of the manufacture, use or sale of the product for human administration.

### Administration

Polypeptides and other compounds of the present invention may be employed alone or in conjunction with other compounds, such as therapeutic compounds.

The pharmaceutical compositions may be administered in any effective, convenient manner including, for instance, administration by topical, oral, anal, vaginal, intravenous, intraperitoneal, intramuscular, subcutaneous, intranasal, intraarticular, or intradermal routes among others.

The pharmaceutical compositions generally are administered in an amount effective for treatment or prophylaxis of a specific indication or indications. In general, the compositions are administered in an amount of at least about 10 mg/kg body weight. Preferably, in most cases, dose is from about 10 mg/kg to about 1 mg/kg body weight, daily. It will be appreciated that optimum dosage will be determined by standard methods for each treatment modality and indication, taking into account the indication, its severity, route of administration, complicating conditions and the like.

### Gene therapy

The cathepsin K polynucleotides, polypeptides, agonists and antagonists that are polypeptides may be employed in accordance with the present invention by expression of such polypeptides *in vivo,* in treatment modalities often referred to as "gene therapy."

Thus, for example, cells from a patient may be engineered with a polynucleotide, such as a DNA or RNA, encoding a polypeptide *ex vivo*, and the engineered cells then can be provided to a patient to he treated with the polypeptide. For example, cells may be engineered *ex vivo* by the use of a retroviral plasmid vector containing RNA encoding a polypeptide of the present invention. Such methods are well-known in the art and their use in the present invention will be apparent from the teachings herein.

Cells from a patient may also be engineered with a polynucleotide, such as a ribozyme that has been constructed, using well known methods, to inhibit the gene expression of Cathepsin K. Other constructs may also be engineered into a patient's cells to contains antisense stretches of cathepsin K sequence, using well known methods. Such antisense constructs will inhibit Cathepsin K expression in the patient.

Similarly, cells may be engineered *in vivo* for expression of a polypeptide *in vivo* by procedures known in the art. For example, a polynucleotide of the invention may be engineered for expression in a replication defective retroviral vector, as discussed above. The retroviral expression construct then may be isolated and introduced into a packaging cell that is transduced with a retroviral plasmid vector containing RNA encoding a polypeptide of the present invention such that the packaging cell now produces infectious viral particles containing the gene of interest. These producer cells may be administered to a patient for engineering cells *in vivo* and expression of the polypeptide *in vivo*. These and other methods for administering a polypeptide of the present invention by such method should be apparent to those skilled in the art from the teachings of the present invention.

Retroviruses from which the retroviral plasmid vectors herein above mentioned may be derived include, but are not limited to, Moloney Murine Leukemia Virus, spleen necrosis virus, retroviruses such as Rous Sarcoma Virus, Harvey Sarcoma Virus, avian leukosis virus, gibbon ape leukemia virus, human immunodeficiency virus, adenovirus, Myeloproliferative Sarcoma Virus, and mammary tumor virus. In one embodiment, the retroviral plasmid vector is derived from Moloney Murine Leukemia Virus.

Such vectors well include one or more promoters for expressing the polypeptide. Suitable promoters which may be employed include, but are not limited to, cathepsin K promoter, a retroviral LTR, an SV40 promoter, and the human cytomegalovirus (CMV) promoter described in Miller et al., Biotechniques 7: 980-990 (1989), or any other promoter (e.g., cellular promoters such as eukaryotic cellular promoters including, but not limited to, the histone, RNA polymerase III, and alpha-actin promoters). Other viral promoters which may be employed include, but are not limited to, adenovirus promoters, thymidine kinase (TK) promoters, and B19 parvovirus promoters. The selection of a suitable promoter will be apparent to those skilled in the an from the teachings contained herein.

The nucleic acid sequence encoding the polypeptide of the present invention will be placed under the control of a suitable promoter. Suitable promoters which may be employed include, but are not limited to, adenoviral promoters, such as the adenoviral major late promoter; or heterologous promoters, such as the cytomegalovirus (CMV) promoter; the rous sarcoma virus (RSV) promoter; inducible promoters, such as the MMT promoter, the metallothionein promoter; heat shock promoters; the albumin promoter; the ApoAI promoter; human globin promoters; viral thymidine kinase promoters, such as the Herpes Simplex thymidine kinase promoter; retroviral LTRs (including the modified retroviral LTRs herein above described); the alpha-actin promoter; and human growth hormone promoters. The promoter also may be the native promoter which controls the gene encoding the polypeptide.

The retroviral plasmid vector is employed to transduce packaging cell lines to form producer cell lines. Examples of packaging cells which may be transfected include, but are not limited to, the PE501, PA317, Y-2, Y-AM, PA12, T19-14X, VT-19-17-H2, YCRE, YCRIP, GP⁺E-86, GP⁺envAm12, and DAN cell lines as described in Miller, A., Human Gene Therapy 1: 5-14 (1990). The vector may be transduced into the packaging cells through any means known in the art. Such means include, but are not limited to, electroporation, the use of liposomes, and CaPO₄ precipitation. In one alternative, the retroviral plasmid vector may be encapsulated into a liposome, or coupled to a lipid, and then administered to a host.

The producer cell line will generate infectious retroviral vector particles, which include the nucleic acid sequence(s) encoding the polypeptides. Such retroviral vector particles then may be employed to transduce eukaryotic cells, either in vitro or *in vivo*. The transduced eukaryotic cells will express the nucleic acid sequence(s) encoding the polypeptide. Eukaryotic cells which may be transduced include, but are not limited to, embryonic stem cells, embryonic carcinoma cells, as well as hematopoietic stem cells, hepatocytes, fibroblasts, myoblasts, keratinocytes, endothelial cells, and bronchial epithelial cells.

### EXAMPLES

The present invention is further described by the following examples. The examples are provided solely to illustrate the invention by reference to specific embodiments. These exemplifications, while illustrating certain specific aspects of the invention, do not portray the limitations or circumscribe the scope of the disclosed invention.

Certain terms used herein are explained in the foregoing glossary.

An N used herein in a nucleotide sequence refers to an unknown nucleotide or nucleotides.

All examples were or may be carried out using standard techniques, which are well known and routine to those of skill in the art, except where otherwise described in detail. Routine molecular biology techniques of the following examples can be carried out as described in standard laboratory manuals, such as Sambrook et al., MOLECULAR CLONING: A LABORATORY MANUAL, 2nd Ed.; Cold Spring Harbor Laboratory Press, Cold Spring Harbor, N.Y. (1989), herein referred to as "Sambrook."

All parts or amounts set out in the following examples are by weight, unless otherwise specified.

Unless otherwise stated size separation of fragments in the examples below was carried out using standard techniques of agarose and polyacrylamide gel electrophoresis ("PAGE") in Sambrook and numerous other references such as, for instance, by Goeddel et al., Nucleic Acids Res. 8: 4057 (1980).

Unless described otherwise, ligations were accomplished using standard buffers, incubation temperatures and times, approximately equimolar amounts of the DNA fragments to be ligated and approximately 10 units of T4 DNA ligase ("ligase") per 0.5 µg of DNA.

### Example 1 Isolation and sequencing of human cathepsin K genomic clone

cDNA as disclosed in U.S. Patent Number 5,501,969, was used to isolate the gDNA clone from a gDNA library (Clontech) according to the following method. Primers to adjacent exons (6 of the 7 exons) were prepared. The sequence of these primers is underlined in Figure 2. PCR was performed using standard methods well known in the art. Amplified fragments were cloned into a TA vector (Clontech) and the clones were sequenced by an automated sequencer (Applied BioSystems Model 373) by established methods well known in the art using forward and reverse sequencing primers. The sequence of all internal introns were obtained. 5' and 3' terminal intron sequences were obtained as follows. 5' end primers were designed to obtain sequence for the first intron (see underlined primer in Figure 2), using these primers 2 P1 clones were obtained (Genome Systems Inc.). Both clones were full length. PCR was used to confirm the sequence of internal intron-exon boundary junctions (see Example 2). Primers derived from sequence at the 5' end of the P1 clones was used to "walk" and sequence along the clone, in a stepwise fashion, using new primers at each sequence step, by routine methods known in the art. Purification of P1 clones was carried out as set forth in Example 1(d). "Walking" and sequencing was performed in both directions to confirm cathepsin K gDNA sequence. PCR was again performed using proofreading Taq polymerase (PCR Ultima, Perkin Elmer).

A transcription start site was obtained using a 5' RACE kit (Gibco BRL) and the protocol supplied therewith. This site was also confirmed using an RNASe protection assay kit (Hybspeed, RPA Ambion). Example 1 (a)-(d) provide further specifics concerning cloning and sequencing of cathepsin K

### (a) DNA sequencing of intron-exon boundaries

### Intron-Exon Boundaries

### Intron 1

Intron 1 was identified by utilization of 5' RACE (Gibco BRL) technique to determine 5' UTR sequence from which primer could be designed to PCR from exon 1 to exon 2. (intron 1 starts prior to ATG so PCR may not be readily employed based on cDNA sequence available.) Intron 1 was amplified by PCR on human genomic DNA (Clontech) and cloned into PCRII vector and sequenced as described in Example 1.

### Intron 2

Intron 2 was identified by PCR on human genomic DNA from primers designed in exon 2 to exon 3. PCR product was cloned and sequenced using standard methods.

### Intron 3

Intron 3 was identified by PCR on human genomic DNA from primers designed in exon 3 to exon 4. PCR product was cloned and sequenced using standard methods.

### Intron 4

Intron 4 was identified by PCR on human genomic DNA from primers designed in exon 4 to exon 5. PCR product was cloned and sequenced using standard methods.

### Introns 5 & 6

Introns 5 and 6 were identified by PCR on human genomic DNA from primers designed in exon 5 to exon 7. PCR product was cloned and sequenced using standard methods confirming presence of both introns.

### Intron 7

Intron 7 was identified by PCR on human genomic DNA from primers designed in exon 7 to exon 8. PCR product was cloned and sequenced using standard methods.

All introns that were identified by PCR on human genomic DNA were confirmed by PCR of the same regions on P1 clone A (see (b) below) clone (Genome Systems, Inc.)

### (b) DNA sequencing of 5' and 3' untranslated region (UTR)

5' and 3' untranslated regions were isolated from a single P1 clone (Genome Systems Inc.). This P1 clone has been identified herein as "P1 clone A." Sequence was obtained by direct sequence walking up and down the P1 clone with gene specific primers derived from confirmed cDNA sequence using standard methods. These regions were then cloned via PCR and confirmed by sequence analysis using standard methods. The 5' UTR was additionally amplified by PCR using proofreading Taq Polymerase Ultima in accordance with manufacturer instructions and cloned to eliminate sequence ambiguities. 5' and 3' UTR were further confirmed by PCR on human genomic DNA using standard methods.

### (c) DNA sequencing of mRNA Cap Site & size of exon 1

An mRNA Cap Site was determined to be about 48 bp upstream of the start codon based on 5' RACE sequencing. Ribonuclease Protection Assay confirmed a protected fragment of about 48 bp in size indicating that the start site from transcription resides about 48 bp upstream of ATG (start codon). Putative transcription factors have been identified by analysis of sequence with database transcription factor sequence information and these are set forth in Figure 3(S) [SEQUENCE ID NO: 2]. The 1.1 kb 5' UTR fragment was cloned into pCAT expression vectors to further analyze the promoter sequence region.

### (d) P1 DNA preparation

P1 clone A colonies were streaked out on Kanamycin LB plates. A single colony was picked and grown O/N in 20 mls with 25 µg/ml of kanamycin. 500 mls of media (25 µg/ml kanamycin) was inoculated with 16 mls of the O/N culture and grown for 10 hours. Cells were pelleted by centrifugation and resuspended in 10 mls of Qiagen P1 Solution. 10 mls of Qiagen P2 Solution was added and incubated at room temp. for 5 min. 10 mls of Qiagen P3 Solution was added and the mixture left on ice for 15 min. The sample was spun at 10,000g for 15 min. The supernatant was removed and extracted with phenol. The supernatant was then re-extracted with chloroform. The DNA was precipitated following addition of NaOAc pH 5.2 and 1.1 volumes of isopropanol. The DNA was pelleted by centrifugation for 15 min. at 10,000g and washed with 70% ethanol. To clean up the DNA for sequencing, 250 µl of DNA (about 50 µg) was added to 65 µl 30% PEG in 1.5M NaCl. 8.5 µl of 3M NaCl was added and the mixture incubated on ice for 30 min. The sample was spun at 12,000g for 10 min. The supernatant was discarded and the pellet dissolved in 200 µl distilled water. The DNA was then extracted with chloroform, vortexed and spun at 10,000g for 1 min. The aqueous layer was removed and the DNA precipitated with 40 µl of NaOAc pH 5.2 and 1 ml of ethanol. The sample was spun at 12,000g for 30 min. The DNA was washed with 1 ml of 70% ethanol and resuspended on distilled water. Prior to sequencing, the DNA was denatured with 0.1 volumes of 2M NaOH and 2mM EDTA and incubated at 37°C for 30 min. The mixture was neutralized with 0.1 volume of 3M NaOAc pH 5.2 and precipitated with 2.5 volumes of ethanol. The denatured DNA was resuspended in distilled water at a concentration of 1 µg/µl 6 µg/µl were used in each sequencing reaction (ABI) using TaqFS.

### Example 2 Chromosomal mapping of cathepsin K

Purified P1 DNA was used for FISH analysis (Genome Systems, Inc.) to map to specific chromosome. Prior results done showed by use of 2 PCR somatic cell hybrid panels that the gene mapped to Chromosome 1. FISH analysis confirmed mapping to chromosome 1 and also further mapped the gene to 1q21. This is the same locus as is known for cathepsin-S.

### Example 3 Expression of cathepsin K in COS cells

### CAT Assays

pCAT-CatK, which contains the 1100 bp putative CatK promoter, upstream of the CAT reporter gene was transfected into COS cells by the DEAE-dextran procedure. Transfections were done on COS cells in 100mm dishes and 5µg of DNA was used. As controls, pCAT basic, which contains no promoter or enhancer, and pCAT control, which contains the SV40 promoter and enhancer, were also transferred separately. 72 hours after transfection, extracts were made by freeze-thaw and equal amounts of extract protein were used in both 1-hour and overnight CAT assays. No activity was detected in untransfected COS cells. pCAT-CatK showed a 1.4-1.6 fold increase of CAT expression relative to pCAT basic after subtraction of background from untransfected cells. Since it is possible that higher levels of activation may be obtained in the presence of various inducers, activation of the CatK promoter by adding exogenous 1,25 di-hydroxy vitamin D3 is believed to occur. Vitamin D has been shown by others to activate transcription of osteocalcin, osteopontin, calcitonin and P450 promoters through interaction with the vitamin D receptor and the vitamin D response element(s) found in these various promoters. The ability of vitamin D to transactivate these promoters is believed thought to play a role in the control of bone formation and resorption. Similar experiments can be performed to assess estrogen responsiveness which is also believed thought to play a role in the control of bone formation and resorption.

### Example 4 Gene therapeutic expression of human cathepsin K

Fibroblasts are obtained from a subject by skin biopsy. The resulting tissue is placed in tissue-culture medium and separated into small pieces. Small chunks of the tissue are placed on a wet surface of a tissue culture flask, approximately ten pieces are placed in each flask. The flask is turned upside down, closed tight and left at room temperature overnight. After 24 hours at room temperature, the flask is inverted - the chunks of tissue remain fixed to the bottom of the flask - and fresh media is added (e.g., Ham's F12 media, with 10% FBS, penicillin and streptomycin). The tissue is then incubated at 37°C for approximately one week. At this time, fresh media is added and subsequently changed every several days. After an additional two weeks in culture, a monolayer of fibroblasts emerges. The monolayer is trypsinized and scaled into larger flasks.

A vector for gene therapy is digested with restriction enzymes for cloning a fragment to he expressed. The digested vector is treated with calf intestinal phosphatase to prevent self-ligation. The dephosphorylated, linear vector is fractionated on an agarose gel and purified.

Cathepsin K gDNA capable of expressing active cathepsin K, is isolated. Preferred constructs use the cathepsin K promoter for cell type-specific gene expression. The ends of the fragment are modified, if necessary, for cloning into the vector. For instance, 5' overhanging may be treated with DNA polymerase to create blunt ends. 3' overhanging ends may be removed using S1 nuclease. Linkers may be ligated to blunt ends with T4 DNA ligase.

Equal quantities of the Moloney murine leukemia virus linear backbone and the cathepsin K fragment are mixed together and joined using T4 DNA ligase. The ligation mixture is used to transform *E. Coli* and the bacteria are then plated on agar-containing kanamycin. Kanamycin phenotype and restriction analysis confirm that the vector has the properly inserted gene.

Packaging cells are grown in tissue culture to confluent density in Dulbecco's Modified Eagles Medium (DMEM) with 10% calf serum (CS), penicillin and streptomycin. The vector containing the cathepsin K gene is introduced into the packaging cells by standard techniques. Infectious viral particles containing the cathepsin K gene are collected from the packaging cells, which now are called producer cells.

Fresh media is added to the producer cells, and after an appropriate incubation period media is harvested from the plates of confluent producer cells. The media, containing the infectious viral particles, is filtered through a Millipore filter to remove detached producer cells. The filtered media then is used to infect fibroblast cells. Media is removed from a sub-confluent plate of fibroblasts and quickly replaced with the filtered media. Polybrene (Aldrich) may be included in the media to facilitate transduction. After appropriate incubation, the media is removed and replaced with fresh media. If the titer of virus is high, then virtually all fibroblasts will be infected and no selection is required. If the titer is low, then it is necessary to use a retroviral vector that has a selectable marker, such as neo or his, to select out transduced cells for expansion.

Engineered fibroblasts then may be injected into humans or animals, including for example, rats and mice, either alone or after having been grown to confluence on microcarrier beads, such as cytodex 3 beads. The injected fibroblasts produce cathepsin K product, and the biological actions of the protein are conveyed to the host.

It will be clear that the invention may be practiced otherwise than as particularly described in the foregoing description and examples.

Numerous modifications and variations of the present invention are possible in light of the above teachings and, therefore, are within the scope of the appended claims.

## Claims

1. An isolated polynucleotide comprising a region selected from the group consisting of:
a sequence at least 80% identical to the sequence in SEQ ID NO: 1,
a sequence at least 85% identical to the sequence in SEQ ID NO: 1,
a sequence at least 90% identical to the sequence in SEQ ID NO: 1,
a sequence at least 95% identical to the sequence in SEQ ID NO: 1, and
a sequence at least 97% identical to the sequence in SEQ ID NO: 1.

2. An isolated polynucleotide according to claim 1, wherein said region is a genomic DNA or a cDNA.

3. An isolated polynucleotide comprising cathepsin K enhancer or promoter.

4. The isolated polynucleotide of claim 3 having the sequence in SEQ ID NO: 1.

5. An isolated polynucleotide comprising cathepsin K polyadenylation region.

6. The isolated polynucleotide of claim 5 having the sequence in SEQ ID NO: 1.

7. An isolated polynucleotide comprising a cathepsin K intron.

8. The isolated polynucleotide of claim 7 having the sequence in SEQ ID NO: 1.

9. An isolated polynucleotide comprising a sequence selected from the group consisting of:
intron 1, 2, 3, 4, 5, 6 and 7.

10. An isolated polypeptide encoded by a polynucleotide comprising a sequence selected from the group consisting of:
intron 1, 2, 3, 4, 5, 6 and 7.

11. An isolated polynucleotide comprising a cathepsin K exon.

12. The isolated polynucleotide of claim 11 having the sequence in SEQ ID NO: 1.

13. An isolated polynucleotide comprising a sequence selected from the group consisting of:
exon 1, 2, 3, 4, 5, 6, 7 and 8.

14. An isolated polypeptide encoded by a polynucleotide comprising a sequence selected from the group consisting of:
exon 1, 2, 3, 4, 5, 6, 7, and 8.

15. An isolated polynucleotide comprising an exon-exon pairs selected from the group consisting of:
1-3, 1-4, 1-5, 1-6, 1-7, 1-8, 2-4, 2-5, 2-6, 2-7, 2-8, 3-4, 3-5, 3-6, 3-7, 3-8, 4-5, 4-6, 4-7, 4-8, 5-7, 5-8 and 6-8.

16. An isolated polypeptide encoded by a polynucleotide comprising an exon-exon pairs selected from the group consisting of:
1-3, 1-4, 1-5, 1-6, 1-7, 1-8, 2-4, 2-5, 2-6, 2-7, 2-8, 3-4, 3-5, 3-6, 3-7, 3-8, 4-5, 4-6, 4-7, 4-8, 5-7, 5-8 and 6-8.

17. An isolated polynucleotide comprising a region selected from the group consisting of:
a sequence at least 80% identical to the human cDNA in ATCC Deposit No.: 98035,
a sequence at least 85% identical to the human cDNA in ATCC Deposit No.: 98035,
a sequence at least 90% identical to the human cDNA in ATCC Deposit No.: 98035,
a sequence at least 95% identical to the human cDNA in ATCC Deposit No.: 98035, and
a sequence at least 97% identical to the human cDNA in ATCC Deposit No.: 98035.

18. An isolated polynucleotide comprising a member selected from the group consisting of:
(a) a polynucleotide having at least a 70% identity to a polynucleotide encoding a polypeptide comprising an amino acid sequence set forth in SEQ ID NO: 20;
(b) a polynucleotide having at least a 70% identity to a polynucleotide encoding a polypeptide comprising amino acid 1 to amino acid 329 forth in SEQ ID NO: 20;
(c) a polynucleotide which hybridizes to and is at least 70% complementary to the polynucleotide of (a) or (b); and
(d) a polynucleotide fragment of the polynucleotide of (a), (b) or (c) wherein said fragment comprises at least 30 consecutive bases of SEQ ID NO: 1.

19. An isolated polynucleotide according to claim 1, wherein said region is the region encoding cathepsin K in the human cDNA insert in ATCC Deposit No.: 98035.

20. An expression vector comprising cis-acting control elements effective for expression in a host cell of an operatively linked polynucleotide according to claim 1.

21. An expression vector according to claim 20, wherein said control elements are effective for inducible expression of said polynucleotide in said host cell.

22. An expression vector comprising cis-acting control elements effective for expression in a host cell of an operatively linked polynucleotide according to claim 18.

23. A host cell having expressibly incorporated therein an expression vector according to claim 22.

24. A host cell having expressibly incorporated therein an expression vector according to claim 18.

25. A process for making a polypeptide, comprising the step of expressing in a host cell a polynucleotide according to claim 1.

26. A process for making a polypeptide, comprising the step of expressing in a host cell a polynucleotide according to claim 18.

27. A polypeptide of 15 or more amino acids identical in sequence to a continuous region of the amino acid sequence of SEQ ID NO: 20.

28. A polypeptide of 15 or more amino acids identical in sequence to a continuous region of the amino acid sequence of the polypeptide encoded by the human cDNA in ATCC Deposit No. 98035.

29. A polypeptide of 50 or more amino acids identical in sequence to a continuous region of the amino acid sequence of SEQ ID NO: 20.

30. A polynucleotide of 25 or more nucleotides identical in sequence to a continuous region of a polynucleotide at least 90% identical in sequence to the polynucleotide of SEQ ID NO: 1.

31. A polynucleotide according to claim 23 of 50 or more nucleotides.

32. A polynucleotide according to claim 31 of 75 or more nucleotides.

33. A host cell genetically engineered with the vector of Claim 20.

34. A process for producing a polypeptide comprising: expressing from the host cell of Claim 33 the polypeptide encoded by said DNA.

35. A process for producing cells capable of expressing a polypeptide comprising genetically engineering cells with the vector of Claim 20.

36. A method for determining a cathepsin K-encoding polynucleotide in a sample, comprising the steps of:
hybridizing to a sample a probe specific for said polynucleotide under conditions effective for said probe to hybridize specifically to said polynucleotide and
determining the hybridization of said probe to polynucleotides in said sample, wherein said probe comprises its sequence a region of 20 or more base pairs at least 90% identical to the polynucleotide sequence of SEQ ID NO: 1.

37. A method for determining a cathepsin K-encoding polynucleotide in a sample, comprising the steps of:
hybridizing to a sample a probe specific for said polynucleotide under conditions effective for said probe to hybridize specifically to said polynucleotide and
determining the hybridization of said probe to polynucleotides in said sample, wherein said probe comprises its sequence a region of 20 or more base pairs at least 90% identical to the polynucleotide sequence of the human cDNA insert of ATCC deposit No. 98035.

38. A method for detecting in a sample a polypeptide comprising a region at least 90% identical in sequence to the amino acid sequence of residues 1 through 135 of SEQ ID NO: 1, said method comprising:
incubating with a sample a reagent that binds specifically to said polypeptide under conditions effective for specific binding and
determining the binding of said reagent to said polypeptide in said sample.

39. A method for diagnosing a disease characterized by aberrant expression of a cathepsin K polynucleotide, comprising:
hybridizing a probe specific for a polynucleotide comprising a region at least 90% identical in sequence to an RNA or DNA that encodes amino acids 1 - 329 in SEQ ID NO: 1 under condition effective for specific hybridization and
determining hybridization of said probe to said polynucleotide in said sample.

40. A method for diagnosing a disease characterized by aberrant expression of a cathepsin K polypeptide, comprising:
incubating with a sample a reagent that binds specifically to a polypeptide comprising a region at least 90% identical in sequence to the amino acid sequence of residues 1 through 329 of SEQ ID NO: 1 under conditions effective for specific binding, and
determining the binding of said reagent to said polypeptide in said sample.

41. A compound which inhibits activation of the polypeptide of claim 13.

42. A method for the treatment of a patient having need of cathepsin K comprising: administering to the patient a therapeutically effective amount of the polypeptide of claim 13.

43. The method of Claim 42 wherein said therapeutically effective amount of the polypeptide is administered by providing to the patient DNA encoding said polypeptide and expressing said polypeptide *in vivo*.

44. A method for the treatment of a patient having need to inhibit a cathepsin K polypeptide comprising: administering to the patient a therapeutically effective amount of the compound of Claim 41.

45. A process for diagnosing a disease or a susceptibility to a disease related to an under-expression of the polypeptide of claim 13 comprising:
determining a mutation in a nucleic acid sequence encoding said polypeptide.

46. A diagnostic process comprising:
analyzing for the presence of the polypeptide of claim 13 in a sample derived from a host.

47. A method for identifying compounds which bind to and inhibit activation of the polypeptide of claim 13 comprising:
contacting a cell expressing on the surface thereof a receptor for the polypeptide, said receptor being associated with a second component capable of providing a detectable signal in response to the binding of a compound to said receptor, with an analytically detectable cathepsin K polypeptide and a compound under conditions to permit binding to the receptor; and
determining whether the compound binds to and inhibits the receptor by detecting the absence of a signal generated from the interaction of the cathepsin K with the receptor.
